# EUROPEAN PATENT SPECIFICATION

(11) **EP 2 099 748 B1**
(45) Date of publication and mention of the grant of the patent: **30.09.2015**
(21) Application number: 07725015.7
(22) Date of filing: 09.05.2007
(51) Int. Cl.: C07C 317/42, C07C 323/36, C07C 323/44, C07C 323/63, C07D 295/195, A01N 41/10, A01N 37/52

(54) **INSECTICIDAL BENZAMIDINES**
INSEKTIZIDE BENZAMIDE
BENZAMIDINES INSECTICIDES

(30) Priority: 16.05.2006 JP 2006136726
(43) Date of publication of application: 16.09.2009
(73) Proprietor: Bayer Intellectual Property GmbH, 40789 Monheim am Rhein (DE)
(72) Inventor: YAMAZAKI, Daiei, Oyama-shi, Tochigi 323-0829 (JP); ITO, Masahito, Ushiku-shi, Ibaraki 300-1232 (JP); GOMIBUCHI, Takuya, Ibaraki 305-0032 (JP); SHIBUYA, Katsuhiko, Tochigi 329-0433 (JP); SHIMOJO, Eiichi, Oyama-shi, Tochigi 323-0820 (JP); EMOTO, Akira, Oyama-shi, Tochigi 323-0022 (JP)
(74) Representative: BIP Patents
(86) International application number: PCT/EP2007/004092
(87) International publication number: WO 2007/131680

(56) References cited:
- EP-A- 0 698 604
- EP-A- 1 803 712
- EP-A1- 0 270 138
- WO-A-2007/015533
- GB-A- 2 014 575
- JP-A- 2 017 180
- JP-A- 57 106 674
- US-A- 4 374 143
- US-A- 4 670 593
- US-A- 4 913 722
- US-B1- 6 509 354
- J.L. COLLINS, ET AL.: "N-Phenylamidines as selective inhibitors of human neuronal nitric oxide synthase: structure-activity studies and demonstration of in vivo activity" JOURNAL OF MEDICINAL CHEMISTRY, vol. 41, no. 15, 23 June 1998 (1998-06-23), pages 2858-2871, XP002937233 AMERICAN CHEMICAL SOCIETY, WASHINGTON, DC, US ISSN: 0022-2623
- S.J. ARCHIBALD, ET AL.: "Synthesis and characterisation of functionalised N,N'-diphenylformamidinate silver(I) dimers: solid-state structures and solution properties" INORGANIC CHEMISTRY, vol. 38, no. 24, 29 November 1999 (1999-11-29), pages 5571-5578, XP002446640 AMERICAN CHEMICAL SOCIETY, WASHINGTON, DC, US

## Description

The present invention relates to novel benzamidines and a use thereof as insecticides.

It has been known that benzamide derivatives are useful as insecticides. (See WO 99/55668 and WO 2006/043635)

There have now been found novel benzamidines of the formula (I) 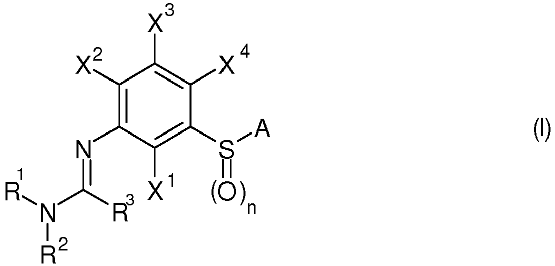
wherein R¹ represents hydrogen, straight or branched C₁₋₁₂ alkyl, C₂₋₅ alkenyl, C₂₋₃ alkynyl, alkoxy, C₃₋₈ cycloalkyl, haloalkyl, alkoxyalkyl, phenyl, α- or β-naphthyl, aralkyl, alkylaminocarbonyl, arylaminocarbonyl, or dialkylaminoalkyl,
R² represents hydrogen or straight or branched C₁₋₁₂ alkyl,
R¹ and R² may form, together with an N atom to which they are attached, pyrrolidine, piperidine or morpholine,
R³ represents hydrogen, straight or branched C₁₋₁₂ alkyl, haloalkyl, cyano or phenyl, α- or β-naphthyl,
R¹ and R³ may form, together with an N atom and C atom to which they are attached, pyrrolidine or piperidine optionally substituted with oxo,
X¹, X², X³ and X⁴ may be the same or different and represent hydrogen, halogen, straight or branched C₁₋₁₂ alkyl, haloalkyl, haloalkoxy, alkoxycarbonyl, nitro or cyano,
A represents trifluoroethyl, and
n represents 0 or 1.

The compounds of the formula (I), according to the present invention, can be obtained, for example, by the following production methods (a) to f).

### Production method (a): {in the formula (I), at least one of X² and X⁴ represents an electron attractive group, and n represents 0}

Compounds of the formula (II) 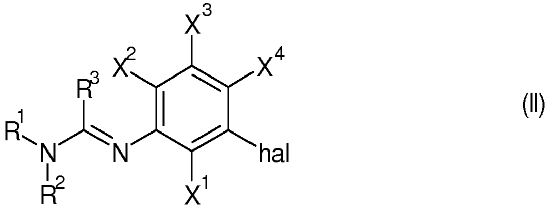 wherein, R¹, R², R³, X¹, X², X³ and X⁴ are as defined above and at least one of X² and X⁴ represents an electron attractive group, and hal represents a halogen,
are reacted with compounds of the formula (III)

HS-A (III)

wherein, A is as defined above,
in the presence of inert solvent and if appropriate, in the presence of a base,

### Production method (b) :{in the formula (I), n represents 1}

Compounds of the formula (Ib) 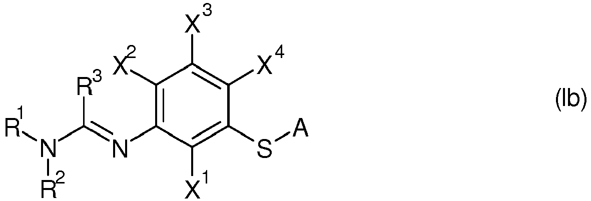 wherein, R¹, R², R³, X¹, X², X³, X⁴ and A are as defined above, are oxidized in the presence of inert solvents,

### Production method (c): {in the formula (I), R³ represents hydrogen, alkyl or aryl}

Compounds of the formula (IV) 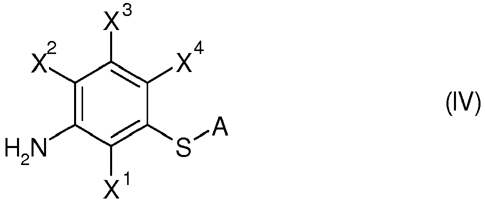 wherein, X¹, X², X³, X⁴ and A are as defined above, are reacted with compounds of the formula (V) 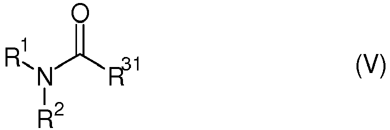 wherein, R¹ and R² are as defined above, R³¹ represents hydrogen, alkyl or aryl,
in the presence of inert solvents,

### Production method (d): {in the formula (I), R³ represents perfluoroalkyl}

Compounds of the formula (VI) 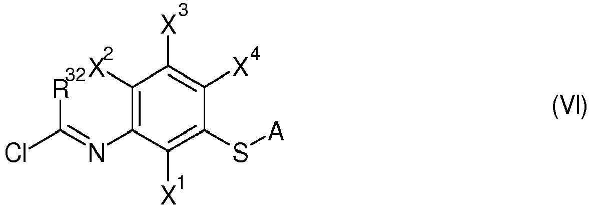 wherein, X¹, X², X³, X⁴ and A are as defined above, and R³² represents perfluoroalkyl,
are reacted with compounds of the formula (VII) 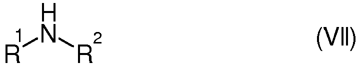 wherein, R¹ and R² are as defined above,
in the presence of inert solvents and if appropriate, in the presence of a base,

### Production method (e): {in the formula (I), R³ represents cyano}

Compounds of the formula (VIII) 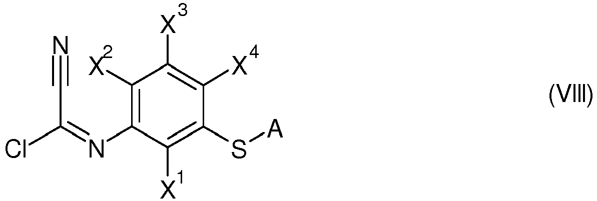 wherein, X¹, X², X³, X⁴ and A are as defined above,
are reacted with compounds of the above-mentioned compounds of the formula (VII)in the presence of inert solvents,

### Production method (f): {in the formula (I), R¹ represents alkylaminocarbonyl}

Compounds of the formula (If) 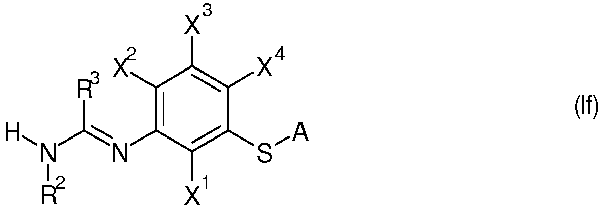 wherein, R², R³, X¹, X², X³, X⁴ and A are as defined above, are reacted with compounds of the formula (X)

R¹¹-N=C=O (X)

wherein, R¹¹ represents alkylaminocarbonyl,
in the presence of inert solvents and if appropriate, in the presence of a base.

According to the present invention, the benzamidines of the formula (I) show a strong insecticidal action.

In this specification,
"alkyl" shows a straight or branched C₁₋₁₂ alkyl such as, for example, methyl, ethyl, n- or iso-propyl, n-, iso-, sec-or tert-butyl, n-pentyl, n-hexyl, n-heptyl, n-octyl, n-nonyl, n-decyl, n-undecyl, n-dodecyl and the like, preferably, a C₁₋₆ alkyl.
"alkenyl" shows a C₂₋₅ alkenyl such as, for example, vinyl, allyl, 1-propenyl, isopropenyl, 1-butenyl, 2-butenyl, 3-butenyl, 1,3-butanedienyl, 2-pentenyl and the like, preferably, a C₂₋₄ alkenyl.
"alkynyl" shows a C₂₋₃ alkynyl such as, for example, ethynyl, 1-propynyl, propargyl and the like, preferably, a C₂₋₃ alkynyl.

As each alkyl portion in "alkoxy", "haloalkyl", "alkoxyalkyl", "alkylaminocarbonyl", "dialkylaminoalkyl", "haloalkoxy", "alkoxycarbonyl", "alkoxycarbonyl" and "alkoxycarbonylalkyl", the same portions as described in the above-mentioned "alkyl" are exemplified.

"cycloalkyl" shows a C₃₋₈ cycloalkyl such as, for example, cyclopropyl, cyclobutyl, cyclopentyl, cyclohexyl, cycloheptyl, cyclooctyl and the like, preferably, a C₃₋₆ cycloalkyl.

"aryl" shows phenyl, α- or β-naphthyl, preferably, phenyl.

"aralkyl" shows, for example, benzyl, phenethyl or α-methylbenzyl, preferably, benzyl.

"halogen" shows fluorine, chlorine, bromine or iodine, preferably, fluorine, chlorine or bromine.

As each halogen portion in "haloalkyl" and "haloalkoxy", the same portions as described in the above-mentioned "halogen" are exemplified.

The 5 or 6-membered hetero ring containing one N atom and, in some cases, containing one O atom shows pyrrolidine, piperidine or morpholine.

An "electron attractive group" shows, for example, fluoro, chloro, methyl, fluoroalkyl, chloroalkyl, nitro, or cyano.

As the compounds of the formula (I) of the present invention, compounds in which
R¹ represents hydrogen, C₁₋₆ alkyl, C₂₋₄ alkenyl, C₂₋₃ alkynyl, C₁₋₄ alkoxy, C₃₋₆ cycloalkyl, C₁₋₄ haloalkyl, C₂₋₄ (total carbon number) alkoxyalkyl, phenyl, α- or β-naphthyl, C₇₋₈ aralkyl, C₁₋₂ alkylaminocarbonyl, C₆₋₁₀ arylaminocarbonyl, or C₂₋₄ (total carbon number) dialkylamino-C₁₋₂ alkyl,
R² represents hydrogen or C₁₋₄ alkyl,
R¹ and R² may form, together with an N atom to which they are attached, pyrrolidine, piperidine or morpholine,
R³ represents hydrogen, C₁₋₆ alkyl, C₁₋₄ haloalkyl, cyano or phenyl,
R¹ and R³ may form, together with an N atom and C atom to which they are attached, pyrrolidine or piperidine optionally substituted with oxo,
X¹, X², X³ and X⁴ may be the same or different and represent hydrogen, halogen, C₁₋₂ alkyl, C₁₋₂ haloalkyl, C₁₋₂ haloalkoxy, C₁₋₂ alkoxycarbonyl, nitro or cyano,
A represents trifluoroethyl, and
n represents 0 or 1,
are mentioned as preferable examples.

Of them, compounds of the formula (I) in which
R¹ represents hydrogen, C₁₋₃ alkyl, allyl, propargyl, C₁₋₂ alkoxy, cyclopropyl, cyclohexyl, trifluoroethyl, methoxyethyl, trifluoromethylphenyl, phenyl, benzyl, ethylaminocarbonyl, phenylaminocarbonyl, or dimethylaminoethyl,
R² represents hydrogen or C₁₋₂ alkyl,
R¹ and R² may form, together with an N atom to which they are attached, piperidine or morpholine,
R³ represents hydrogen, C₁ alkyl, fluoro-substituted-C₁₋₃ alkyl, cyano or phenyl,
R¹ and R³ may form, together with an N atom and C atom to which they are attached, pyrrolidine or piperidine optionally substituted with oxo,
X¹ represents hydrogen or chloro,
X² represents hydrogen, fluoro, chloro, bromo,iodo, methyl, difluoromethyl, trifluoromethyl, nitro or cyano,
X³ represents hydrogen,
X⁴ represents hydrogen, chloro, bromo, iodo, methyl, difluoromethyl, trifluoromethyl, nitro or cyano,
A represents trifluoroethyl, and
n represents 0 or 1,
are even more preferable.

Of them, compounds of the formula (I) in which
R¹ represents hydrogen, C₁₋₃ alkyl, allyl, propargyl, methoxy, cyclopropyl, trifluoroethyl, trifluoromethylphenyl, benzyl substituted with trifluoromethyl, or ethylaminocarbonyl,
R² represents hydrogen, or C₁₋₂ alkyl,
R³ represents C₁ alkyl, C₁ alkylene, fluoro-substituted-C₁₋₃ alkyl, cyano or phenyl,
R¹ and R³ may form, together with an N atom and C atom to which they are attached, pyrrolidine
X¹ represents hydrogen,
X² represents hydrogen, or fluoro
X³ represents hydrogen,
X⁴ represents difluoromethyl, or cyano,
A represents C₃ alkyl, or trifluoroethyl, and
n represents 0 or 1
are most preferred.

The above-mentioned production method (a) can be represented by the following reaction formula when, for example, N'-(2,5-dichloro-4-nitrophenyl)-N,N-diethyl-2,2,2-trifluoro ethaneimideamide and 2,2,2-trifluoroethanethiol are used as starting raw materials.

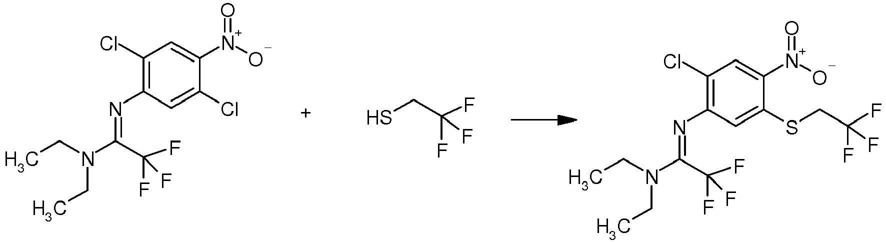

The above-mentioned production method (b) can be represented by the following reaction formula when, for example, N'-{4-cyano-2-fluoro-5-[(2,2,2-trifluoroethyl)thio)phenyl}-N,N-diethyl-2,2,2-trifluoroethaneimideamide and, m-chloroperbenzoic acid as an oxidizer, are used as starting raw materials.

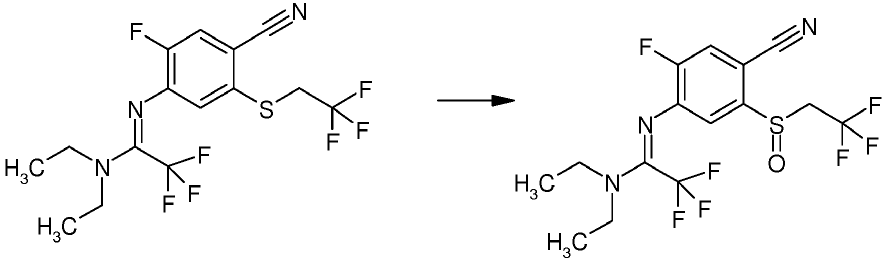

The above-mentioned production method (c) can be represented by the following reaction formula when, for example, 2-chloro-4-methyl-2-[(2,2,2-trifluoroethyl)thio]aniline and N,N-dimethylacetamide are used as starting raw materials.

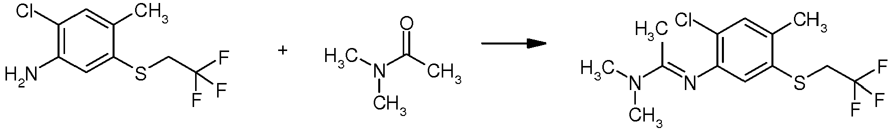

The above-mentioned production method (d) can be represented by the following reaction formula when, for example, N-{4-cyano-2-fluoro-5-[(2,2,2-trifluoroethyl)thio]phenyl}-2 ,2,2-trifluoroethaneimideyl chloride and diethylamine are used as starting raw materials.

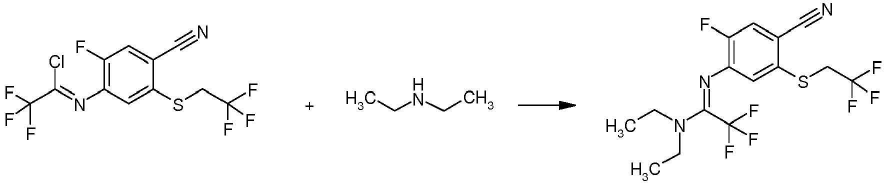

The above-mentioned production method (e) can be represented by the following reaction formula when, for example, 1-{[chloro(cyano)methylene]amino}-4-cyano-2-fluoro-5-[(2,2, 2-trifluoroethyl)thio]benzene and diethylamine are used as starting raw materials.

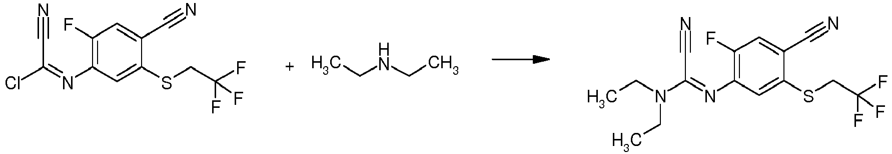

The above-mentioned production method (f) can be represented by the following reaction formula when, for example, N'-{4-cyano-2-fluoro-5-[(2,2,2-trifluoroethyl)thio]phenyl}-2,2,2-trifluoroethaneimideamide and ethyl isocyanate are used as starting raw materials.

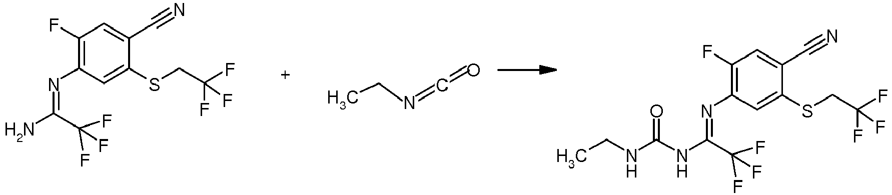

Regarding the compounds of the formula (II) used as a raw material in the above-mentioned production method (a), compounds of the following formula (XI) 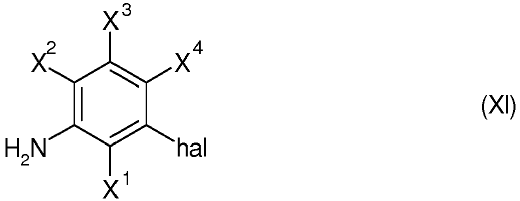 wherein, X¹, X², X³, X⁴ and hal are as defined above, can be reacted as a starting substance with compounds of the formula (V), (XXIX) or (XXX) according to the production methods (c), (d) or (e) described in detail later, to finally obtain the compounds corresponding to the formula (II).

The compounds of the above-mentioned formula (XI) includes, for example, 5-fluoro-2,4-dinitroaniline, 2,5-dichloro-4-nitroaniline, 5-chloro-2-nitro-4-trifluoromethylaniline and the like, and all of them are commercially available compounds.

The thiols of the above-mentioned formula (III) include, for example, methanethiol, ethanethiol, 1-propanethiol, 2-propanethiol, 1-butanethiol, 2-metyl-1-propanethiol, cyclopropylmethanethiol, 2,2,2-trifluoroethanethiol, methylmercapto acetate, phenylmethanethiol and the like, and all of them are commercially available compounds.

The reaction of the production method (a) can be carried out in a suitable diluting agent, and examples of the diluting agent used in this procedure include ethers, for example, dioxane, dimethoxyethane (DME), tetrahydrofuran (THF), diethylene glycol dimethyl ether (DGM) and the like; ketones, for example, acetone, methyl ethyl ketone (MEK), methyl isopropyl ketone, methyl isobutyl ketone (MIBK) and the like; nitriles, for example, acetonitrile, propionitrile, acrylonitriles and the like; acid amides, for example, dimethylformamide (DMF), dimethylacetamide (DMA), N-methylpyrrolidone, 1,3-dimethyl-2-imidazolidinone, hexamethylphosphoric triamide (HMPA) and the like.

The production method (a) can be carried out in the presence of a base, and inorganic bases as examples of the base include hydroxides, carbonates, bicarbonates and the like of alkali metals, for example, sodium hydrogencarbonate, potassium hydrogencarbonate, sodium carbonate, potassium carbonate, lithium hydroxide, sodium hydroxide, potassium hydroxide and the like.

The production method (a) can be carried out in a substantially wide temperature range. In general, it can be carried out between about -40°C to about 200°C, preferably about 0°C to about 100°C. It is desirable that the reaction is carried out under normal pressure, however, it can also be operated under increased pressure or reduced pressure.

In carrying out the production method (a), compounds of the formula (III) in an amount of 1 mol or slightly excess amount and potassium carbonate as a base in an amount of 1 mol or slightly excess amount are used per mol of compounds of the formula (II) and DMF is used as a diluting agent, and they are stirred at room temperature, thus, the aimed compounds can be obtained.

The compounds of the formula (Ib) in the production method (b) is a sulfide body included in the compounds (I) of the present invention which can be produced by the production method (a), (c), (d), (e) or (f).

As the oxidizer used in the oxidation reaction in the production method (b), for example, hydrogen peroxide, m-chloroperbenzoic acid, and the like are mentioned.

The reaction of the production method (b) can be carried out in a suitable diluting agent. Examples of the diluting agent used in this procedure include water; aliphatic, cyclic aliphatic and aromatic hydrocarbons (optionally chlorinated in some cases), for example, pentane, hexane, cyclohexane, petroleum ether, benzene, toluene, xylene, dichloromethane, dichloroethane and the like; nitriles, for example, acetonitrile, propionitrile, acrylonitrile and the like; organic acids, for example, formic acid, acetic acid, trifluoroacetic acid, propionic acid and the like.

The production method (b) can be carried out in a substantially wide temperature range. In general, it can be carried out between about -40°C to about 200°C, preferably about 0°C to about 100°C. It is desirable that the reaction is carried out under normal pressure, however, it can also be operated under increased pressure or reduced pressure.

In carrying out the production method (b), an oxidizer in an amount of 1 mol or slightly excess amount is used per mol of compounds of the formula (Ib) and dichloromethane is used as a diluting agent, and they are stirred at room temperature, thus, the aimed compounds can be obtained.

Among the compounds of the formula (IV) in the production method (c), aniline derivatives of the following formula (IV-A) are novel.

Formula (IV-A) 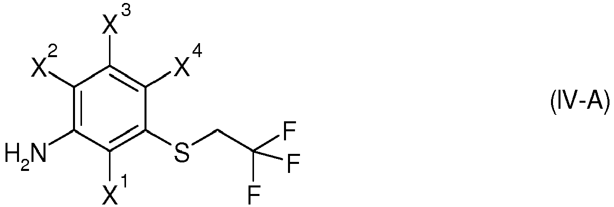 wherein, X¹, X², X³ and X⁴ are as defined above.

The compounds of the formula (IV) can be obtained, for example, by the following production methods (g) to (k). Production method (g):

A method of producing the compounds of the formula (IV) in which compounds of the formula (XII): 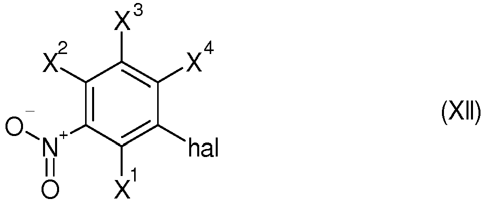 wherein, X¹, X², X³, X⁴ and hal are as defined above, are reacted with the compounds of the formula (III) in the presence of a base, to obtain compounds of the following formula (XIII) 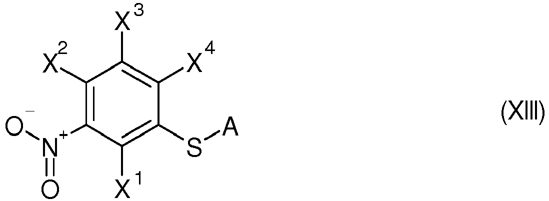 wherein, X¹, X², X³, X⁴ and A are as defined above, and then, the compounds of the formula (XIII) are reduced.

The compounds of the formula (XII) include, for example, 2-fluoro-1-methyl-4-nitrobenzene, 4-fluoro-1-methyl-2-nitrobenzene and the like, and these are easily available compounds.

The above-mentioned reduction reaction can provide the compounds of the formula (IV) according to methods widely known in the field of organic chemistry such as, for example, a method as described in Shin Jikken Kagaku Koza 14, III (publisher; Maruzen K.K.), p. 1333 to 1335, and the like.

### Production method (h):

A method of producing the compounds of the formula (IV) in which compounds of the formula (XIV): 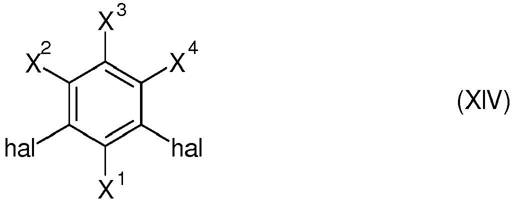 wherein, X¹, X², X³, X⁴ and hal are as defined above, are reacted with potassium phthalimide to obtain compounds of the formula (XV) : 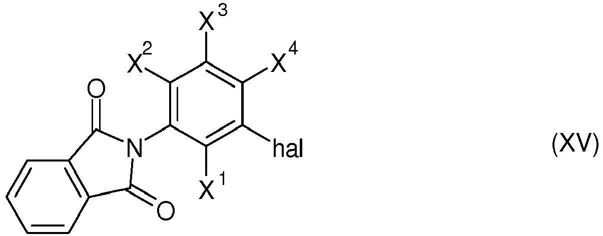 wherein, X¹, X², X³, X⁴ and hal are as defined above,
and then, the above-mentioned compounds of the formula (XV) are reacted with the compounds of the formula (III) in the presence of a base, to obtain compounds of the formula (XVI) 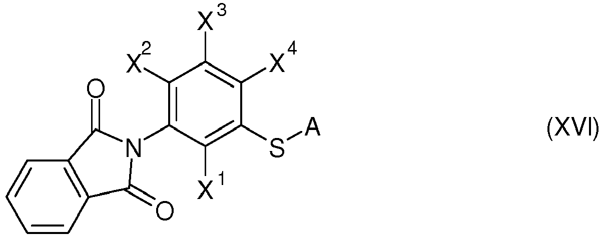 wherein, X¹, X², X³, X⁴ and A are as defined above,
and then, the compounds of the formula (XVI) are reacted with hydrazine.

The compounds of the formula (XIV) include, for example, 2,4-difluoro-benzonitrile, 2,4,5-trifluoro-benzonitrile, 1-difluoromethyl-2,4-difluorobenzene and the like, and all of them are commercially available compounds. These compounds can be synthesized from known 2,4-difluorobenzaldehyde according to methods widely known in the field of organic chemistry such as a method described in J. Org. Chem. , 40, 1975, 574-578, and the like.

The above-mentioned reaction of the compounds of the formula (XIV) with the compounds of the formula (XV) can be carried out in a suitable diluting agent, and as examples of the diluting agent used in this procedure, ethers, for example, dioxane, dimethoxyethane (DME), tetrahydrofuran (THF), diethylene glycol dimethyl ether (DGM) and the like; nitriles, for example, acetonitrile, propionitrile, acrylonitrile and the like: acid amides, for example, dimethylformamide (DMF), dimethylacetamide (DMA), N-methylpyrrolidone, 1,3-dimethyl-2-imidazolidinone, hexamethylphosphoric triamide (HMPA) and the like can be used.

This reaction can be carried out in a substantially wide temperature range. In general, it can be carried out between about 50°C to about 200°C, preferably about 80°C to about 120°C. It is desirable that the reaction is carried out under normal pressure, however, it can also be operated under increased pressure or reduced pressure.

In carrying out this reaction, potassium phthalimide in an amount of 1 mol or slightly excess amount per mol of the compounds of the formula (XIV) can be heated while stirring using DMF as a diluting agent, to obtain the compounds of the formula (XV).

The above-mentioned reaction of the compounds of the formula (XV) with the compounds of the formula (III) can be carried out under the same conditions as for the above-mentioned production method (a).

The above-mentioned reaction of the compounds of the formula (XVI) with hydrazine can be carried out in a suitable diluting agent, and as examples of the diluting agent used in this procedure, aliphatic, cyclic aliphatic and aromatic hydrocarbons (optionally chlorinated in some cases), for example, pentane, hexane, cyclohexane, petroleum ether, benzene, toluene, xylene, dichloromethane, dichloroethane and the like; nitriles, for example, acetonitrile, propionitrile, acrylonitrile and the like; alcohols, for example, methanol, ethanol, isopropanol, butanol, ethylene glycol and the like can be used.

This reaction can be carried out in a substantially wide temperature range. In general, it can be carried out between about -20°C to about 120°C, preferably about 0°C to about 80°C. It is desirable that the reaction is carried out under normal pressure, however, it can also be operated under increased pressure or reduced pressure.

In carrying out this reaction, a hydrazine hydrate in an amount of 1 mol or slightly excess amount per mol of the compounds of the formula (XVI) can be heated while stirring using ethanol as a diluting agent, to obtain the compounds of the formula (IV).

### Production method (i): {in the formula (IV), X¹ and X³ represent hydrogen, and X² and X⁴ represent a halogen other than fluorine}

A method of producing compounds of the formula (IV-i) 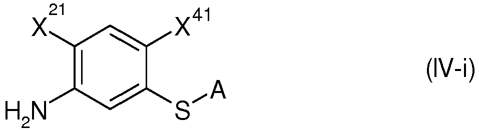 wherein, X²¹, X⁴¹ and A are as defined above, in which compounds of the formula (XVII) 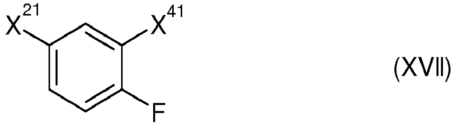 wherein, X²¹ and X⁴¹ represent a halogen other than fluorine, are nitrated to obtain compounds of the formula (XVIII) 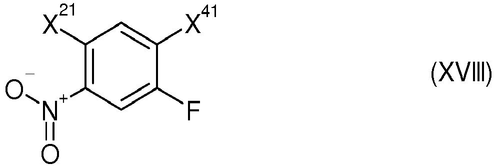 wherein, X²¹ and X⁴¹ are as defined above,
and then, the above compounds of the formula (XVIII) are reduced to obtain compounds of the formula (XIX) 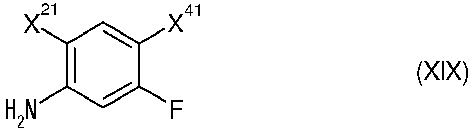 wherein, X²¹ and X⁴¹ are as defined above,
and then, the compounds of the formula (XIX) are reacted with phthalic anhydride under acidic condition to obtain compounds of the formula (XX) 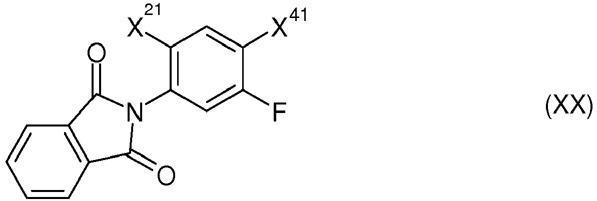 wherein, X²¹ and X⁴¹ are as defined above,
and then, the compounds of the formula (XX) are reacted with the compounds of the formula (III) to obtain compounds of the formula (XXI) 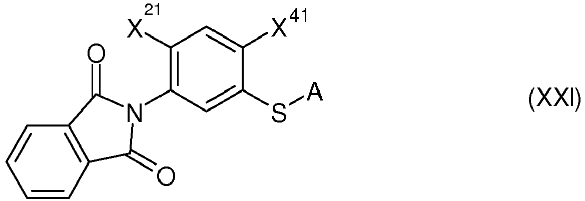 wherein, X²¹, X⁴¹ and A are as defined above,
and then, the compounds of the formula (XXI) are reacted with hydrazine.

The compounds of the formula (XVII) include, for example, 2,4-dichloro-1-fluorobenzene, 4-bromo-2-chloro-1-fluorobenzene, 2,4-dibromo-1-fluorobenzene, 2-chloro-1-fluoro-4-iodobenzene and the like, and all of them are commercially available compounds.

The above-mentioned nitration reaction can be carried out, for example, according to a method described in Shin Jikken Kagaku Koza 14, III (publisher; Maruzen K.K.), p. 1266 to 1277.

The above-mentioned reduction reaction can be carried out, for example, according to a method described in Shin Jikken Kagaku Koza 14, III (publisher; Maruzen K.K.), p. 1333 to 1335.

The above-mentioned reaction of the compounds of the formula (XIX) with phthalic anhydride can be carried out in a suitable diluting agent, and examples of the diluting agent used in this procedure include organic acids, for example, formic acid, acetic acid, trifluoroacetic acid, propionic acid, methanesulfonic acid, benzenesulfonic acid, p-toluenesulfonic acid and the like, or aromatic hydrocarbons (optionally chlorinated in some cases), for example, benzene, toluene, xylene and the like, to which an acid catalyst can be added and used.

This reaction can be carried out in a substantially wide temperature range. In general, it can be carried out between about 20°C to about 150°C, preferably about 60°C to about 120°C. It is desirable that the reaction is carried out under normal pressure, however, it can also be operated under increased pressure or reduced pressure.

In carrying out this reaction, phthalic anhydride in an amount of 1 mol or slightly excess amount per mol of the compounds of the formula (XIX) can be heated while stirring using acetic acid as a diluting agent, to obtain the compounds of the formula (XX).

The above-mentioned reaction of the compounds of the formula (XX) with the compounds of the formula (III) can be carried out under the same conditions as for the above-mentioned production method (a).

The above-mentioned reaction of the compounds of the formula (XXI) with hydrazine can be carried out under the same conditions as for the above-mentioned reaction of the compounds of the formula (XVI) with hydrazine in the above-mentioned production method (h).

### Production method (j)

A method of producing the compounds of the formula (IV) in which compounds of the formula (XXII): 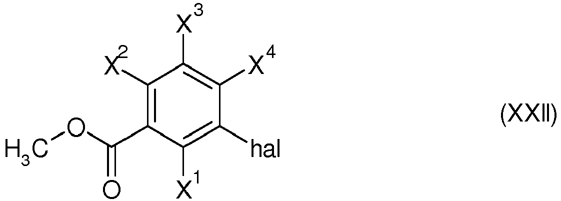 wherein, X¹, X², X³, X⁴ and hal are as defined above,
are reacted with the compounds of the formula (III) in the presence of a base to obtain compounds of the formula (XXIII): 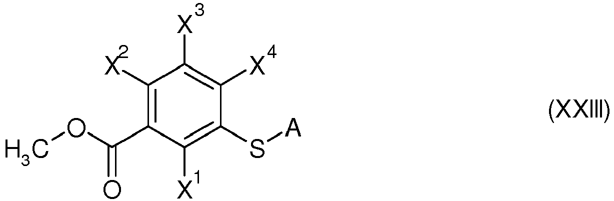 wherein, X¹, X², X³, X⁴ and A are as defined above,
and then, the compounds of the formula (XXIII) are hydrolyzed to obtain the compounds of the formula (XXIV) 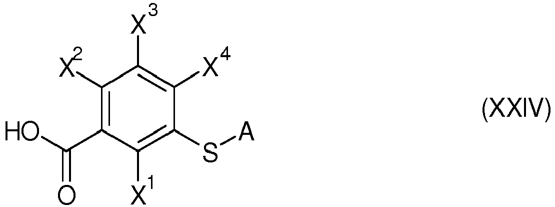 wherein, X¹, X², X³, X⁴ and A are as defined above,
and then, the compounds of the formula (XXIV) are reacted with a halogenating agent to obtain compounds of the formula (XXV) : 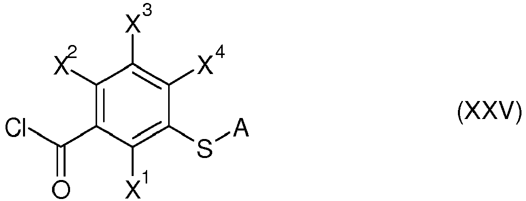 wherein, X¹, X², X³, X⁴ and A are as defined above,
and then, the compounds of the formula (XXV) are reacted with sodium azide to obtain acylazide compounds which are subjected to rearrangement reaction (Curtis rearrangement), then, subjected to a hydrolysis reaction.

The compounds of the formula (XXII) can be obtained easily from benzoic acid compounds such as 3-fluoro-4-trifluoromethylbenzoic acid, 5-fluoro-2-trifluoromethylbenzoic acid and the like according to an ordinary method. All of these benzoic acid compounds are well-known compounds.

The above-mentioned reaction of the compounds of the formula (XXII) with the compounds of the formula (III) can be carried out under the same conditions as for the above-mentioned method (a).

The above-mentioned hydrolysis reaction can be carried out, for example, according to a method described in Shin Jikken Kagaku Koza 14, II (publisher; Maruzen K.K.), p. 930 to 938.

The above-mentioned halogenation reaction can be carried out, for example, according to a method described in Shin Jikken Kagaku Koza 14, II (publisher; Maruzen K.K.), p. 1106 to 1111.

The above-mentioned rearrangement reaction can be carried out, for example, according to a method described in Shin Jikken Kagaku Koza 14, III (publisher; Maruzen K.K.), p. 1391 to 1393.

Production method (k) : {in the formula (IV), X¹ represents hydrogen and X² or X⁴ represents a halogen other than fluorine, or X¹ represents a halogen other than fluorine and X² or X⁴ represents hydrogen}

A method of producing compounds of the formula (IV-k1) 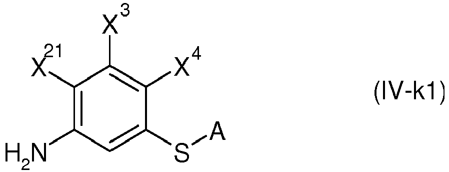 the formula (IV-k2) 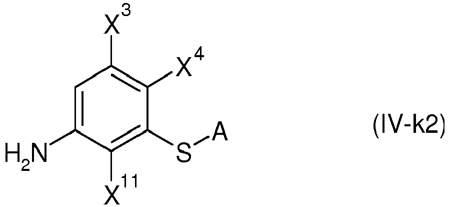 the formula (IV-k3) 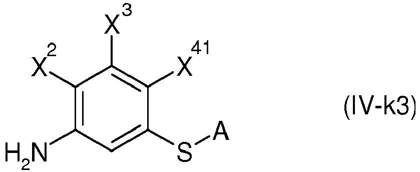 or the formula (IV-k4) 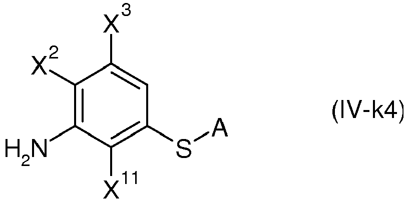 (wherein, X², X³, X⁴, X²¹, X⁴¹ and A are as defined above and X¹¹ represents a halogen other than fluorine.)
in which compounds of the formula (XXVI): 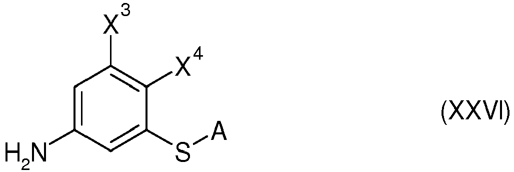 (wherein, X³, X⁴ and A are as defined above.)
or compounds of the formula (XXVII): 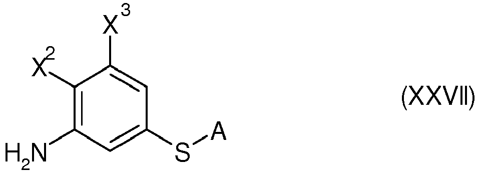 (wherein, X², X³ and A are as defined above.)
are reacted with a halogenating agent.

The compounds of the formulae (XXVI) or (XXVII) can be synthesized by the above-mentioned production methods (g), (h) or (j), and examples thereof include
4-amino-2-(2,2,2-trifluoroethylthio)benzonitrile
2-amino-4-(2,2,2-trifluoroethylthio)benzonitrile
3-(2,2,2-trifluoroethylthio)-4-trifluoromethylaniline
5-(2,2,2-trifluoroethylthio)-2-trifluoromethylaniline
4-difluoromethyl-3-(2,2,2-trifluoroethylthio)aniline
2-difluoromethyl-5-(2,2,2-trifluoroethylthio)aniline
4-methyl-3-(2,2,2-trifluoroethylthio)aniline
2-methyl-5-(2,2,2-trifluoroethylthio)aniline
and the like.

Examples of the halogenating agent used in the above-mentioned reaction include chlorine, bromine, iodine, N-chlorosuccinic imide, N-bromosuccinic imide, N-iodosuccinic imide and the like.

The above-mentioned reaction can be carried out in a suitable diluting agent, and as examples of the diluting agent used in this procedure, aliphatic halogenated hydrocarbons, for example, dichloromethane, dichloroethane, chloroform, carbon tetrachloride and the like; acid amides, for example, dimethylformamide (DMF), dimethylacetamide (DMA), N-methylpyrrolidone, 1,3-dimethyl-2-imidazolidinone, hexamethylphosphoric triamide (HMPA) and the like can be used.

This reaction can be carried out in a substantially wide temperature range. In general, it can be carried out between about -40°C to about 200°C, preferably about 0°C to about 100°C. It is desirable that the reaction is carried out under normal pressure, however, it can also be operated under increased pressure or reduced pressure.

In carrying out this reaction, N-halogenated succinic imide in an amount of 1 mol or slightly excess amount per mol of compounds of the formula (XXVI) can be stirred at room temperature or (XXVII) using DMF as a diluting agent, to obtain compounds of the formulae (IV-k1), (IV-k2), (IV-k3) or (IV-k4).

Of compounds of the formula (IV) in the production method (c), particularly compounds of the following formula are novel compounds.

Formula (IV-A) 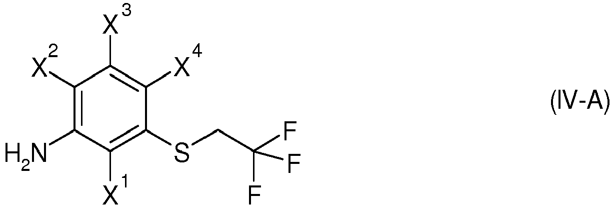 wherein, X¹, X², X³ and X⁴ are as defined above.

The compounds of the formula (V) which are another raw material in the production method (c) are known compounds, and in general, it can be obtained easily by a reaction of a carboxylic chloride with amines.

The reaction of the production method (c) can be carried out in a suitable diluting agent, and examples of the diluting agent used in this procedure include aromatic hydrocarbons (optionally chlorinated in some cases), for example, pentane, toluene, xylene and the like; nitriles, for example, acetonitrile, propionitrile, acrylonitrile and the like.

The production method (c) can be carried out in a substantially wide temperature range. In general, it can be carried out between about 50°C to about 150°C, preferably about 80°C to about 120°C. It is desirable that the reaction is carried out under normal pressure, however, it can also be operated under increased pressure or reduced pressure.

In carrying out the production method (c), compounds of the formula (V) in an amount of 1 mol or slightly excess amount can be heated while stirring in excess amount of phosphorus oxychloride per mol of compounds of the formula (IV) to obtain the aimed compounds.

In the production method (d), the compounds of the formula (VI) are obtained by reacting compounds of the formula (XXVIII) : 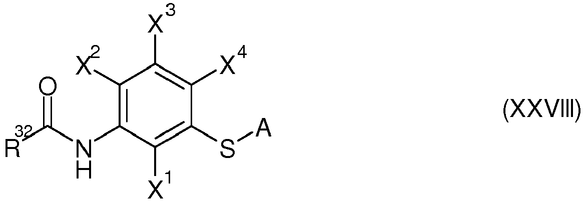 wherein, X¹, X², X³, X⁴, R³² and A are as defined above, with triphenylphosphine and carbon tetrachloride.

The compounds of the formula (XXVIII) are obtained by reacting the compounds of the formula (IV) with compounds of the formula (XXIX) 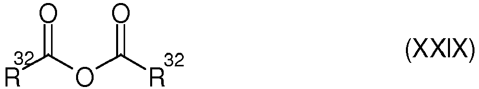 wherein, R³² is as defined above,
in the presence of a base

Specific examples of the compounds of the formula (XXIX) include, for example, trifluoroacetic anhydride, pentafluoropropionic anhydride, heptafluorobutanoic anhydride and the like.

The above-mentioned reaction of the compounds of the formula (IV) with the compounds of the formula (XXIX) can be carried out in a suitable diluting agent, and examples of the diluting agent used in this procedure include aliphatic, cyclic aliphatic and aromatic hydrocarbons (optionally chlorinated in some cases), for example, pentane, hexane, cyclohexane, petroleum ether, benzene, toluene, xylene, dichloromethane, dichloroethane and the like; ethers, for example, ethyl ether, methyl ethyl ether, isopropyl ether, butyl ether, dioxane, dimethoxyethane (DME), tetrahydrofuran (THF), diethylene glycol dimethyl ether (DGM) and the like; ketones, for example, acetone, methyl ethyl ketone (MEK), methyl isopropyl ketone, methyl isobutyl ketone (MIBK) and the like; nitriles, for example, acetonitrile, propionitrile, acrylonitriles and the like; esters, for example, ethyl acetate, amyl acetate and the like; acid amides, for example, dimethylformamide (DMF), dimethylacetamide (DMA), N-methylpyrrolidone, 1,3-dimethyl-2-imidazolidinone, hexamethylphosphoric triamide (HMPA) and the like.

This reaction can be carried out in the presence of a base, and as examples of the base, inorganic bases include hydroxides, carbonates, bicarbonates and the like of alkali metals, for example, sodium hydrogencarbonate, potassium hydrogencarbonate, sodium carbonate, potassium carbonate, lithium hydroxide, sodium hydroxide, potassium hydroxide and the like; and organic bases include alcoholates, tertiary amines, dialkylaminoanilines and pyridines, for example, triethylamine, 1,1,4,4-tetramethylethylenediamine (TMEDA), N,N-dimethylaniline, N,N-diethylaniline, pyridine, 4-dimethylaminopyridine (DMAP), 1,4-diazabicyclo[2,2,2]octane (DABCO) and 1,8-diazabicyclo[5,4,0]undec-7-ene (DBU) and the like.

This reaction can be carried out in a substantially wide temperature range. In general, it can be carried out between about -40°C to about 100°C, preferably about 0°C to about 40°C. It is desirable that the reaction is carried out under normal pressure, however, it can also be operated under increased pressure or reduced pressure.

In carrying out the reaction, the compounds of the formula (XXIX) in an amount of 1 mol or slightly excess amount and triethylamine as a base in an amount of 1 mol or slightly excess amount are used per mol of the compounds of the formula (IV) and dichloromethane is used as a diluting agent, and they are stirred at room temperature, thus, the compounds of the formula (XXVIII) can be obtained.

The reaction of the compounds of the formula (XXVIII) with triphenylphosphine and carbon tetrachloride can be carried out in a suitable diluting agent, and examples of the diluting agent used in this procedure include aliphatic halogenated hydrocarbons, for example, dichloromethane, dichloroethane, chloroform and the like.

This reaction can be carried out in a substantially wide temperature range. In general, it can be carried out between about 0°C to about 100°C, preferably about 20°C to about 60°C. It is desirable that the reaction is carried out under normal pressure, however, it can also be operated under increased pressure or reduced pressure.

In carrying out the reaction, triphenylphosphine in slightly excess amount and carbon tetrachloride in slightly excess amount per mol of compounds of the formula (XXVIII) can be heated while stirring using dichloromethane as a diluting agent to obtain the compounds of the formula (VI).

The compounds of the formula (VII) which are another raw material in the production method (d) are well known compounds, and examples thereof include ammonia, methylamine, dimethylamine, ethylamine, 2,2,2-trifluoroethylamine, n-propylamine, isopropylamine, methylethylamine, diethylamine, cyclopropylamine, allylamine, propargylamine, pyrrolidine, piperidine, morpholine, cyclohexylethylamine, aniline, 4-trifluoroaniline, benzylamine, 4-trifluorobenzylamine, O-methylhydroxylamine, N,O-dimethylhydroxylamine, 2-methoxyethylamine, N,N-dimethylethylenediamine and the like.

The reaction in the production method (d) can be carried out in a suitable diluting agent, and examples of the diluting agent used in this procedure include aliphatic, cyclic aliphatic and aromatic hydrocarbons (optionally chlorinated in some cases), for example, pentane, hexane, cyclohexane, petroleum ether, benzene, toluene, xylene, dichloromethane, dichloroethane and the like; ethers, for example, ethyl ether, methyl ethyl ether, isopropyl ether, butyl ether, dioxane, dimethoxyethane (DME), tetrahydrofuran (THF), diethylene glycol dimethyl ether (DGM) and the like; ketones, for example, acetone, methyl ethyl ketone (MEK), methyl isopropyl ketone, methyl isobutyl ketone (MIBK) and the like; nitriles, for example, acetonitrile, propionitrile, acrylonitriles and the like; esters, for example, ethyl acetate, amyl acetate and the like; acid amides, for example, dimethylformamide (DMF), dimethylacetamide (DMA), N-methylpyrrolidone, 1,3-dimethyl-2-imidazolidinone, hexamethylphosphoric triamide (HMPA) and the like.

The reaction in the production method (d) can be carried out in the presence of a base, and as examples of the base, inorganic bases include hydrides, hydroxides, carbonates, bicarbonates and the like of alkali metals, for example, sodium hydride, lithium hydride, sodium hydrogencarbonate, potassium hydrogencarbonate, sodium carbonate, potassium carbonate, lithium hydroxide, sodium hydroxide, potassium hydroxide and the like; and organic bases include alcoholates, tertiary amines, dialkylaminoanilines and pyridines, for example, triethylamine, 1,1,4,4-tetramethylethylenediamine (TMEDA), N,N-dimethylaniline, N,N-diethylaniline, pyridine, 4-dimethylaminopyridine (DMAP), 1,4-diazabicyclo[2,2,2]octane (DABCO) and 1,8-diazabicyclo[5,4,0]undec-7-ene (DBU) and the like, and by using the compounds of the formula (VII) in an amount of 2 equivalents or more, one equivalent portion can be used as a base.

The production method (d) can be carried out in a substantially wide temperature range. In general, it can be carried out between about -40°C to about 100°C, preferably about 0°C to about 40°C. It is desirable that the reaction is carried out under normal pressure, however, it can also be operated under increased pressure or reduced pressure.

In carrying out the production method (d), the compounds of the formula (VII) in excess amount of 2 mol or more per mol of the compounds of the formula (VI) can be stirred at room temperature using acetonitrile as a diluting agent to obtain aimed compounds.

Compounds of the formula (VIII) in the production method (e) are obtained by reacting compounds of the formula (IV) with compounds of the formula (XXX) : 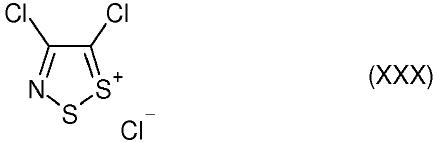 in the presence of a base to obtain compounds of the formula (XXXI) : 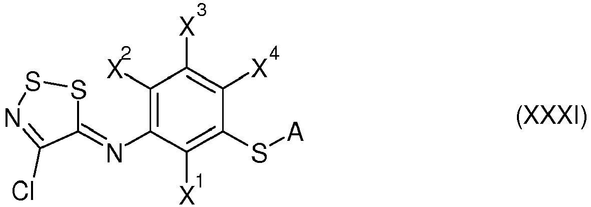 (wherein, X¹, X², X³, X⁴ and A are as defined above.) then, thermally decomposing the above-mentioned compounds of the formula (XXXI). The above-mentioned compounds of the formula (XXX) are obtained by a method described in Chem. Ber., 118, 1985, 1632-1643.

The above-mentioned reaction of compounds of the formula (IV) with compounds of the formula (XXX) can be carried out in a suitable diluting agent, and examples of the diluting agent used in this procedure include aliphatic, cyclic aliphatic and aromatic hydrocarbons (optionally chlorinated in some cases), for example, pentane, hexane, cyclohexane, petroleum ether, benzene, toluene, xylene, dichloromethane, dichloroethane and the like; ethers, for example, ethyl ether, methyl ethyl ether, isopropyl ether, butyl ether, dioxane, dimethoxyethane (DME), tetrahydrofuran (THF), diethylene glycol dimethyl ether (DGM) and the like; ketones, for example, acetone, methyl ethyl ketone (MEK), methyl isopropyl ketone, methyl isobutyl ketone (MIBK) and the like; nitriles, for example, acetonitrile, propionitrile, acrylonitriles and the like; esters, for example, ethyl acetate, amyl acetate and the like.

This reaction can be carried out in the presence of a base, and organic bases as examples of the base include pyridines, for example, pyridine, 4-dimethylaminopyridine (DMAP) and the like.

This reaction can be carried out in a substantially wide temperature range. In general, it can be carried out between about -40°C to about 100°C, preferably about 0°C to about 40°C. It is desirable that the reaction is carried out under normal pressure, however, it can also be operated under increased pressure or reduced pressure.

In carrying out the reaction, compounds of the formula (XXX) in an amount of 1 mol or slightly excess amount and pyridine as a base in an amount of 2 mol or slightly excess amount per mol of compounds of the formula (IV) can be stirred at room temperature using dichloromethane as a diluting agent to obtain compounds of the formula (XXXI).

The thermal decomposition reaction can be carried out in a substantially wide temperature range. In general, it can be carried out between about 150°C to about 300°C, preferably about 180°C to about 220°C. It is desirable that the reaction is carried out under normal pressure, however, it can also be operated under increased pressure or reduced pressure.

In carrying out the reaction, compounds of the formula (XXXI) can be directly heated while stirring without using a diluting agent to obtain the compounds of the formula (VIII).

The reaction in the production method (e) can be carried out under the same conditions as for the above-mentioned production method (c) .

The compounds of the formula (If) which are a raw material in the production method (f) are included in the compounds (I) of the present invention which can be produced by the production methods (a), (c), (d) or (e).

The compounds of the formula (IX) which are another raw material are well known compounds and examples thereof include ethyl isocyanate, phenyl isocyanate and the like.

The reaction of the production method (f) can be carried out in a suitable diluting agent, and examples of the diluting agent used in this procedure include ethers, for example, tetrahydrofuran (THF) and the like; acid amides, for example, dimethylformamide (DMF), dimethylacetamide (DMA), N-methylpyrrolidone, 1,3-dimethyl-2-imidazolidinone, hexamethylphosphoric triamide (HMPA) and the like.

The production method (f) can be carried out in the presence of a base, and inorganic bases as examples of the base, hydrides and the like of alkali metals, for example, sodium hydride, lithium hydride and the like can be used.

The production method (f) can be carried out in a substantially wide temperature range. In general, it can be carried out between about -20°C to about 200°C, preferably about 0°C to about 100°C. It is desirable that the reaction is carried out under normal pressure, however, it can also be operated under increased pressure or reduced pressure.

In carrying out the production method (f), the compounds of the formula (IX) in an amount of 1 mol or slightly excess amount and sodium hydride in an amount of 1 mol or slightly excess amount per mol of the compounds of the formula (If) can be stirred at room temperature using DMF as a diluting agent to obtain aimed compounds.

The compounds of the formula (I), according to the present invention, show a strong insecticidal action. Therefore, the compounds of the formula (I) can be used as insecticides. If appropriate, the compounds of the formula (I) can be present in different polymorphic forms or as a mixture of different polymorphic forms. Both the pure polymorphs and the polymorph mixtures are provided by the invention and can be used according to the invention.

The active compounds according to the invention, in combination with good plant tolerance and favourable toxicity to warm-blooded animals and being tolerated well by the environment, are suitable for protecting plants and plant organs, for increasing the harvest yields, for improving the quality of the harvested material and for controlling animal pests, in particular insects, arachnids, helminths, nematodes and molluscs, which are encountered in agriculture, in horticulture, in animal husbandry, in forests, in gardens and leisure facilities, in the protection of stored products and of materials, and in the hygiene sector. They may be preferably employed as plant protection agents. They are active against normally sensitive and resistant species and against all or some stages of development. The abovementioned pests include: From the order of the Anoplura (Phthiraptera), for example, Damalinia spp., Haematopinus spp., Linognathus spp., Pediculus spp., Trichodectes spp.

From the class of the Arachnida, for example, Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

From the class of the Bivalva, for example, Dreissena spp. From the order of the Chilopoda, for example, Geophilus spp., Scutigera spp.

From the order of the Coleoptera, for example, Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

From the order of the Collembola, for example, Onychiurus armatus.

From the order of the Dermaptera, for example, Forficula auricularia.

From the order of the Diplopoda, for example, Blaniulus guttulatus.

From the order of the Diptera, for example, Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp., Lucilia spp., Musca spp., Nezara spp., Oestrusspp., Oscinellafrit, Pegomyia hyoscyami, Phorbiaspp., Stomoxys spp., Tabanus spp., Tannia spp., Tipula paludosa, Wohlfahrtia spp.

From the class of the Gastropoda, for example, Arion spp., Biomphalaria spp., Bulinus spp., Deroceras spp., Galba spp., Lymnaea spp., Oncomelania spp., Succinea spp.

From the class of the helminths, for example, Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

It is furthermore possible to control protozoa, such as Eimeria. From the order of the Heteroptera, for example, Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygusspp., Macropes excavatus, Miridae, Nezaraspp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

From the order of the Homoptera, for example, Acyrthosipon spp. , Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Lao-delphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

From the order of the Hymenoptera, for example, Diprion spp., Hoplocampa spp., Lasius spp., Monomorium pharaonis, Vespa spp. From the order of the Isopoda, for example, Armadillidium vulgare, Oniscus asellus, Porcellio scaber.

From the order of the Isoptera, for example, Reticulitermes spp. , Odontotermes spp.

From the order of the Lepidoptera, for example, Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp. From the order of the Orthoptera, for example, Acheta domesticus, Blatta orientalis, Blattella germanica, Gryllotalpa spp., Leucophaea maderae, Locusta spp., Melanoplus spp., Periplaneta americana, Schistocerca gregaria.

From the order of the Siphonaptera, for example, Ceratophyllus spp., Xenopsylla cheopis.

From the order of the Symphyla, for example, Scutigerella immaculata.

From the order of the Thysanoptera, for example, Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

From the order of the Thysanura, for example, Lepisma saccharina.

The phytoparasitic nematodes include, for example, Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

If appropriate, the active compounds according to the invention can, at certain concentrations or application rates, also be used as herbicides, safeners, growth regulators or agents to improve plant properties, or as microbicides, for example as fungicides, antimycotics, bactericides, viricides (including agents against viroids) or as agents against MLO (Mycoplasma-like organisms) and RLO (Rickettsia-like organisms). If appropriate, they can also be employed as intermediates or precursors for the synthesis of other active compounds.

The active compounds can be converted to the customary formulations, such as solutions, emulsions, wettable powders, water- and oil-based suspensions, powders, dusts, pastes, soluble powders, soluble granules, granules for broadcasting, suspension-emulsion concentrates, natural materials impregnated with active compound, synthetic materials impregnated with active compound, fertilizers and microencapsulations in polymeric substances.

These formulations are produced in a known manner, for example by mixing the active compounds with extenders, that is liquid solvents and/or solid carriers, optionally with the use of surfactants, that is emulsifiers and/or dispersants and/or foam-formers. The formulations are prepared either in suitable plants or else before or during the application.

Suitable for use as auxiliaries are substances which are suitable for imparting to the active compounds itself and/or to preparations derived therefrom (for example spray liquors, seed dressings) particular properties such as certain technical properties and/or also particular biological properties. Typical suitable auxiliaries are: extenders, solvents and carriers.

Suitable extenders are, for example, water, polar and non-polar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulphones and sulphoxides (such as dimethyl sulphoxide).

If the extender used is water, it is also possible to employ, for example, organic solvents as auxiliary solvents. Essentially, suitable liquid solvents are: aromatics such as xylene, toluene or alkylnaphthalenes, chlorinated aromatics and chlorinated aliphatic hydrocarbons such as chlorobenzenes, chloroethylenes or methylene chloride, aliphatic hydrocarbons such as cyclohexane or paraffins, for example petroleum fractions, mineral and vegetable oils, alcohols such as butanol or glycol and also their ethers and esters, ketones such as acetone, methyl ethyl ketone, methyl isobutyl ketone or cyclohexanone, strongly polar solvents such as dimethyl sulphoxide, and also water.

Suitable solid carriers are:
for example, ammonium salts and ground natural minerals such as kaolins, clays, talc, chalk, quartz, attapulgite, montmorillonite or diatomaceous earth, and ground synthetic minerals, such as finely divided silica, alumina and silicates; suitable solid carriers for granules are: for example, crushed and fractionated natural rocks such as calcite, marble, pumice, sepiolite and dolomite, and also synthetic granules of inorganic and organic meals, and granules of organic material such as paper, sawdust, coconut shells, maize cobs and tobacco stalks; suitable emulsifiers and/or foam-formers are: for example, nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates and also protein hydrolysates; suitable dispersants are nonionic and/or ionic substances, for example from the classes of the alcohol-POE and/or -POP ethers, acid and/or POP-POE esters, alkyl aryl and/or POP-POE ethers, fat and/or POP-POE adducts, POE- and/or POP-polyol derivatives, POE- and/or POP-sorbitan- or -sugar adducts, alkyl or aryl sulphates, alkyl- or arylsulphonates and alkyl or aryl phosphates or the corresponding PO-ether adducts. Furthermore, suitable oligo- or polymers, for example those derived from vinylic monomers, from acrylic acid, from EO and/or PO alone or in combination with, for example, (poly) alcohols or (poly)amines. It is also possible to employ lignin and its sulphonic acid derivatives, unmodified and modified celluloses, aromatic and/or aliphatic sulphonic acids and their adducts with formaldehyde.

Tackifiers such as carboxymethylcellulose and natural and synthetic polymers in the form of powders, granules or latices, such as gum arabic, polyvinyl alcohol and polyvinyl acetate, as well as natural phospholipids such as cephalins and lecithins, and synthetic phospholipids, can be used in the formulations. It is possible to use colorants such as inorganic pigments, for example iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

Other possible additives are perfumes, mineral or vegetable, optionally modified oils, waxes and nutrients (including trace nutrients), such as salts of iron, manganese, boron, copper, cobalt, molybdenum and zinc.

Stabilizers, such as low-temperature stabilizers, preservatives, antioxidants, light stabilizers or other agents which improve chemical and/or physical stability may also be present.

The formulations generally comprise between 0.01 and 98% by weight of active compound, preferably between 0.5 and 90%. The active compound according to the invention can be used in its commercially available formulations and in the use forms, prepared from these formulations, as a mixture with other active compounds, such as insecticides, attractants, sterilizing agents, bactericides, acaricides, nematicides, fungicides, growth-regulating substances, herbicides, safeners, fertilizers or semiochemicals.

Particularly favourable mixing components are, for example, the following compounds:
Fungicides:
   Inhibitors of nucleic acid synthesis benalaxyl, benalaxyl-M, bupirimate, chiralaxyl, clozylacon, dimethirimol, ethirimol, furalaxyl, hymexazol, metalaxyl, metalaxyl-M, ofurace, oxadixyl, oxolinic acid Inhibitors of mitosis and cell division benomyl, carbendazim, diethofencarb, fuberidazole, pencycuron, thiabendazole, thiophanat-methyl, zoxamide Inhibitors of respiratory chain complex I diflumetorim Inhibitors of respiratory chain complex II boscalid, carboxin, fenfuram, flutolanil, furametpyr, mepronil, oxycarboxin, penthiopyrad, thifluzamide Inhibitors of respiratory chain complex III azoxystrobin, cyazofamid, dimoxystrobin, enestrobin, famoxadone, fenamidone, fluoxastrobin, kresoxim-methyl, metominostrobin, orysastrobin, pyraclostrobin, picoxystrobin Decouplers dinocap, fluazinam Inhibitors of ATP production fentin acetate, fentin chloride, fentin hydroxide, silthiofam Inhibitors of amino acid biosynthesis and protein biosynthesis andoprim, blasticidin-S, cyprodinil, kasugamycin, kasugamycin hydrochloride hydrate, mepanipyrim, pyrimethanil Inhibitors of signal transduction fenpiclonil, fludioxonil, quinoxyfen Inhibitors of lipid and membrane synthesis chlozolinate, iprodione, procymidone, vinclozolin ampropylfos, potassium-ampropylfos, edifenphos, iprobenfos (IBP), isoprothiolane, pyrazophos tolclofos-methyl, biphenyl iodocarb, propamocarb, propamocarb hydrochloride Inhibitors of ergosterol biosynthesis fenhexamid, azaconazole, bitertanol, bromuconazole, cyproconazole, diclobutrazole, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, paclobutrazole, penconazole, propiconazole, prothioconazole, simeconazole, tebuconazole, tetraconazole, triadimefon, triadimenol, triticonazole, uniconazole, voriconazole, imazalil, imazalil sulphate, oxpoconazole, fenarimol, flurprimidole, nuarimol, pyrifenox, triforine, pefurazoate, prochloraz, triflumizole, viniconazole, aldimorph, dodemorph, dodemorph acetate, fenpropimorph, tridemorph, fenpropidin, spiroxamine, naftifine, pyributicarb, terbinafine Inhibitors of cell wall synthesis benthiavalicarb, bialaphos, dimethomorph, flumorph, iprovalicarb, polyoxins, polyoxorim, validamycin A Inhibitors of melanin biosynthesis capropamid, diclocymet, fenoxanil, phthalid, pyroquilon, tricyclazole Resistance inductors acibenzolar-S-methyl, probenazole, tiadinil Multisite captafol, captan, chlorothalonil, copper salts such as: copper hydroxide, copper naphthenate, copper oxychloride, copper sulphate, copper oxide, oxine-copper and Bordeaux mixture, dichlofluanid, dithianon, dodine, dodine free base, ferbam, folpet, fluorofolpet, guazatine, guazatine acetate, iminoctadine, iminoctadine albesilate, iminoctadine triacetate, mancopper, mancozeb, maneb, metiram, metiram zinc, propineb, sulphur and sulphur preparations containing calcium polysulphide, thiram, tolylfluanid, zineb, ziram Unknown mechanism amibromdol, benthiazol, bethoxazin, capsimycin, carvone, chinomethionat, chloropicrin, cufraneb, cyflufenamid, cymoxanil, dazomet, debacarb, diclomezine, dichlorophen, dicloran, difenzoquat, difenzoquat methyl sulphate, diphenylamine, ethaboxam, ferimzone, flumetover, flusulphamide, fluopicolide, fluoroimide, hexachlorobenzene, 8-hydroxyquinoline sulphate, irumamycin, methasulphocarb, metrafenone, methyl isothiocyanate, mildiomycin, natamycin, nickel dimethyl dithiocarbamate, nitrothal-isopropyl, octhilinone, oxamocarb, oxyfenthiin, pentachlorophenol and salts, 2-phenylphenol and salts, piperalin, propanosine-sodium, proquinazid, pyrrol nitrin, quintozene, tecloftalam, tecnazene, triazoxide, trichlamide, zarilamid and 2,3,5,6-tetrachloro-4-(methylsulphonyl)pyridine, N-(4-chloro-2-nitrophenyl)-N-ethyl-4-methylbenzenesulphonam ide, 2-amino-4-methyl-N-phenyl-5-thiazolecarboxamide, 2-chloro-N-(2,3-dihydro-1,1,3-trimethyl-1H-inden-4-yl)-3-py ridinecarboxamide, 3-[5-(4-chlorophenyl)-2,3-dimethylisoxazolidin-3-yl]pyridin e, cis-1-(4-chloro-phenyl)-2-(1H-1,2,4-triazol-1-yl)cycloheptanol, 2,4-dihydro-5-methoxy-2-methyl-4-[[[[1-[3-(trifluoromethyl) phenyl]ethylidene]amino]oxy]methyl]phenyl]-3H-1,2,3-triazol -3-one (185336-79-2), methyl 1-(2,3-dihydro-2,2-dimethyl-1H-inden-1-yl)-1H-imidazole-5-c arboxylate, 3,4,5-trichloro-2,6-pyridinedicarbonitrile, methyl 2-[[[cyclopropyl[(4-methoxy-phenyl)imino]methyl]thio]methyl]-.alpha.-(methoxymethylene) benzacetate, 4-chloro-alpha-propynyloxy-N-[2-[3-methoxy-4-(2-propynyloxy )phenyl]ethyl]benzacetamide, (2S)-N-[2-[4-[[3-(4-chlorophenyl)-2-propynyl]oxy]-3-methoxy phenyl]ethyl]-3-methyl-2-[(methylsulphonyl)amino]butanamide, 5-chloro-7-(4-methylpiperidin-1-yl)-6-(2,4,6-trifluoropheny l)[1,2,4]triazolo[1,5-a]pyrimidine, 5-chloro-6-(2,4,6-trifluoro-phenyl)-N-[(1R)-1,2,2-trimethylpropyl]-[1,2,4]triazolo[1,5-a]pyrimidin-7-amine, 5-chloro-N-[(1R)-1,2-dimethylpropyl]-6-(2,4,6-trifluorophen yl)[1,2,4]triazolo[1,5-a]pyrimidin-7-amine, N-[1-(5-bromo-3-chloropyridin-2-yl)ethyl]-2,4-dichloronicot inamide, N-(5-bromo-3-chloropyridin-2-yl)methyl-2,4-dichloro-nicotinamide, 2-butoxy-6-iodo-3-propylbenzopyranon-4-one, N-{(Z)-[(cyclopropylmethoxy)-imino][6-(difluoromethoxy)-2,3-difluorophenyl]methyl}-2-ben zacetamide, N-(3-ethyl-3,5,5-trimethylcyclohexyl)-3-formylamino-2-hydro xybenzamide, 2-[[[[1-[3(1-fluoro-2-phenyl-ethyl)oxy]phenyl]ethylidene]amino]oxy]methyl]-alpha-(methox yimino)-N-methyl-alphaE-benzacetamide, N-{2-[3-chloro-5-(trifluoromethyl)pyridin-2-yl]ethyl}-2-(tr ifluoromethyl)benzamide, N-(3',4'-dichloro-5-fluorobiphenyl-2-yl)-3-(difluoromethyl) -1-methyl-1H-pyrazole-4-carboxamide, N-(6-methoxy-3-pyridinyl)cyclopropanecarboxamide, 1-[(4-methoxyphenoxy)methyl]-2,2-dimethylpropyl-1H-imidazol e-1-carboxylic acid, O-[1-[(4-methoxyphenoxy)methyl]-2,2-dimethylpropyl]-1H-imid azole-1-carbothioic acid, 2-(2-{[6-(3-chloro-2-methylphenoxy)-5-fluoropyrimidin-4-yl] oxy}phenyl)-2-(methoxyimino)-N-methylacetamide
Bactericides:
   bronopol, dichlorophen, nitrapyrin, nickel dimethyldithiocarbamate, kasugamycin, octhilinone, furancarboxylic acid, oxytetracycline, probenazole, streptomycin, tecloftalam, copper sulphate and other copper preparations.
Insecticides/acaricides/nematicides:
   Acetylcholine esterase (AChE) inhibitors carbamates, for example alanycarb, aldicarb, aldoxycarb, allyxycarb, aminocarb, bendiocarb, benfuracarb, bufencarb, butacarb, butocarboxim, butoxycarboxim, carbaryl, carbofuran, carbosulphan, cloethocarb, dimetilan, ethiofencarb, fenobucarb, fenothiocarb, formetanate, furathiocarb, isoprocarb, metam-sodium, methiocarb, methomyl, metolcarb, oxamyl, pirimicarb, promecarb, propoxur, thiodicarb, thiofanox, trimethacarb, XMC, xylylcarb, triazamate organophosphates, for example acephate, azamethiphos, azinphos (-methyl, -ethyl), bromophos-ethyl, bromfenvinfos (-methyl), butathiofos, cadusafos, carbophenothion, chlorethoxyfos, chlorfenvinphos, chlormephos, chlorpyrifos (-methyl/-ethyl), coumaphos, cyanofenphos, cyanophos, chlorfenvinphos, demeton-S-methyl, demeton-S-methylsulphone, dialifos, diazinon, dichlofenthion, dichlorvos/DDVP, dicrotophos, dimethoate, dimethylvinphos, dioxabenzofos, disulphoton, EPN, ethion, ethoprophos, etrimfos, famphur, fenamiphos, fenitrothion, fensulphothion, fenthion, flupyrazofos, fonofos, formothion, fosmethilan, fosthiazate, heptenophos, iodofenphos, iprobenfos, isazofos, isofenphos, isopropyl O-salicylate, isoxathion, malathion, mecarbam, methacrifos, methamidophos, methidathion, mevinphos, monocrotophos, naled, omethoate, oxydemeton-methyl, parathion (-methyl/-ethyl), phenthoate, phorate, phosalone, phosmet, phosphamidon, phosphocarb, phoxim, pirimiphos (-methyl/-ethyl), profenofos, propaphos, propetamphos, prothiofos, prothoate, pyraclofos, pyridaphenthion, pyridathion, quinalphos, sebufos, sulphotep, sulprofos, tebupirimfos, temephos, terbufos, tetrachlorvinphos, thiometon, triazophos, triclorfon, vamidothion Sodium channel modulators / voltage-dependent sodium channel blockers pyrethroids, for example acrinathrin, allethrin (d-cis-trans, d-trans), beta-cyfluthrin, bifenthrin, bioallethrin, bioallethrin-S-cyclopentyl isomer, bioethanomethrin, biopermethrin, bioresmethrin, chlovaporthrin, cis-cypermethrin, cis-resmethrin, cis-permethrin, clocythrin, cycloprothrin, cyfluthrin, cyhalothrin, cypermethrin (alpha-, beta-, theta-, zeta-), cyphenothrin, deltamethrin, empenthrin (1R isomer), esfenvalerate, etofenprox, fenfluthrin, fenpropathrin, fenpyrithrin, fenvalerate, flubrocythrinate, flucythrinate, flufenprox, flumethrin, fluvalinate, fubfenprox, gamma-cyhalothrin, imiprothrin, kadethrin, lambda-cyhalothrin, metofluthrin, permethrin (cis-, trans-), phenothrin (1R-trans-isomer), prallethrin, profluthrin, protrifenbute, pyresmethrin, resmethrin, RU 15525, silafluofen, tau-fluvalinate, tefluthrin, terallethrin, tetramethrin (1R isomer), tralomethrin, transfluthrin, ZXI 8901, pyrethrins (pyrethrum)
      DDT
      oxadiazines,
      for example indoxacarb
      semicarbazones,
      for example metaflumizone (BAS3201) Acetylcholine receptor agonists/antagonists chloronicotinyls,
      for example acetamiprid, clothianidin, dinotefuran, imidacloprid, nitenpyram, nithiazine, thiacloprid, imidaclothiz, AKD-1022, thiamethoxam nicotine, bensultap, cartap Acetylcholine receptor modulators spinosyns,
      for example spinosad, spinetoram (XDE-175) GABA-controlled chloride channel antagonists organochlorines,
      for example camphechlor, chlordane, endosulphan, gamma-HCH, HCH, heptachlor, lindane, methoxychlor fiprols,
      for example acetoprole, ethiprole, fipronil, pyrafluprole, pyriprole, vaniliprole Chloride channel activators mectins,
      for example abarmectin, emamectin, emamectin-benzoate, ivermectin, lepimectin, milbemycin Juvenile hormone mimetics,
      for example diofenolan, epofenonane, fenoxycarb, hydroprene, kinoprene, methoprene, pyriproxifen, triprene Ecdysone agonists/disruptors diacylhydrazines,
      for example chromafenozide, halofenozide, methoxyfenozide, tebufenozide Chitin biosynthesis inhibitors benzoylureas,
      for example bistrifluron, chlofluazuron, diflubenzuron, fluazuron, flucycloxuron, flufenoxuron, hexaflumuron, lufenuron, novaluron, noviflumuron, penfluron, teflubenzuron, triflumuron buprofezin cyromazine
      Oxidative phosphorylation inhibitors, ATP disruptors diafenthiuron organotin compounds,
      for example azocyclotin, cyhexatin, fenbutatin-oxide Oxidative phosphorylation decouplers acting by interrupting the H-proton gradient pyrroles,
      for example chlorfenapyr dinitrophenols, for example binapacyrl, dinobuton, dinocap, DNOC Site-I electron transport inhibitors METIs,
      for example fenazaquin, fenpyroximate, pyrimidifen, pyridaben, tebufenpyrad, tolfenpyrad hydramethylnon dicofol Site-II electron transport inhibitors rotenone Site-III electron transport inhibitors acequinocyl, fluacrypyrim Microbial disruptors of the insect gut membrane Bacillus thuringiensis strains Lipid synthesis inhibitors tetronic acids, for example spirodiclofen, spiromesifen tetramic acids,
      for example spirotetramat carboxamides,
      for example flonicamid octopaminergic agonists,
      for example amitraz Inhibitors of magnesium-stimulated ATPase, propargite nereistoxin analogues,
      for example thiocyclam hydrogen oxalate, thiosultap-sodium Ryanodin receptor agonists benzoic acid dicarboxamides,
      for example flubendiamid anthronilamides,
      for example pynaxypyr (3-bromo-N-{4-chloro-2-methyl-6-[(methylamino)carbonyl]phe-nyl}-1-(3-chloropyridin-2-yl)-1H-pyrazole-5-carboxamide) Biologicals, hormones or pheromones azadirachtin, Bacillus spec., Beauveria spec., codlemone, Metarrhizium spec., Paecilomyces spec., thuringiensin, Verticillium spec.
      Active compounds with unknown or unspecific mechanisms of action fumigants,
      for example aluminium phosphide, methyl bromide, sulphuryl fluoride antifeedants,
      for example cryolite, flonicamid, pymetrozine mite growth inhibitors,
      for example clofentezine, etoxazole, hexythiazox amidoflumet, benclothiaz, benzoximate, bifenazate, bromopropylate, buprofezin, chinomethionat, chlordimeform, chlorobenzilate, chloropicrin, clothiazoben, cycloprene, cyflumetofen, dicyclanil, fenoxacrim, fentrifanil, flubenzimine, flufenerim, flutenzin, gossyplure, hydramethylnone, japonilure, metoxadiazone, petroleum, piperonyl butoxide, potassium oleate, pyridalyl, sulphluramid, tetradifon, tetrasul, triarathene, verbutin A mixture with other known active compounds, such as herbicides, fertilizers, growth regulators, safeners, semiochemicals, or else with agents for improving the plant properties, is also possible.

When used as insecticides, the active compounds according to the invention can furthermore be present in their commercially available formulations and in the use forms, prepared from these formulations, as a mixture with synergists. Synergists are compounds which increase the action of the active compounds, without it being necessary for the synergistic agent added to be active itself.

When used as insecticides, the active compounds according to the invention can furthermore be present in their commercially available formulations and in the use forms, prepared from these formulations, as a mixture with inhibitors which reduce degradation of the active compound after use in the environment of the plant, on the surface of parts of plants or in plant tissues.

The active compound content of the use forms prepared from the commercially available formulations can vary within wide limits. The active compound concentration of the use forms can be from 0.00000001 to 95% by weight of active compound, preferably between 0.00001 and 1% by weight.

The active compounds are employed in a customary manner appropriate for the use forms.

All plants and plant parts can be treated in accordance with the invention. Plants are to be understood as meaning in the present context all plants and plant populations such as desired and undesired wild plants or crop plants (including naturally occurring crop plants). Crop plants can be plants which can be obtained by conventional plant breeding and optimization methods or by biotechnological and genetic engineering methods or by combinations of these methods, including the transgenic plants and including the plant cultivars protectable or not protectable by plant breeders' rights. Plant parts are to be understood as meaning all parts and organs of plants above and below the ground, such as shoot, leaf, flower and root, examples which may be mentioned being leaves, needles, stalks, stems, flowers, fruit bodies, fruits, seeds, roots, tubers and rhizomes. The plant parts also include harvested material, and vegetative and generative propagation material, for example cuttings, tubers, rhizomes, offshoots and seeds. Treatment according to the invention of the plants and plant parts with the active compounds is carried out directly or by allowing the compounds to act on the surroundings, habitat or storage space by the customary treatment methods, for example by immersion, spraying, evaporation, fogging, scattering, painting on, injection and, in the case of propagation material, in particular in the case of seeds, also by applying one or more coats.

The active compounds according to the invention are particularly suitable for treating seed. Here, the active compounds according to the invention mentioned above as preferred or particularly preferred may be mentioned as being preferred. Thus, a large part of the damage to crop plants which is caused by pests occurs as early as when the seed is attacked during storage and after the seed is introduced into the soil, during and immediately after germination of the plants. This phase is particularly critical since the roots and shoots of the growing plant are particularly sensitive and even minor damage can lead to the death of the whole plant. Protecting the seed and the germinating plant by the use of suitable active compounds is therefore of particularly great interest.

The control of pests by treating the seeds of plants has been known for a long time and is the subject of continuous improvements. However, the treatment of seed entails a series of problems which cannot always be solved in a satisfactory manner. Thus, it is desirable to develop methods for protecting the seed and the germinating plant which dispense with the additional application of crop protection agents after sowing or after the emergence of the plants. It is furthermore desirable to optimize the amount of active compound employed in such a way as to provide maximum protection for the seed and the germinating plant from attack by pests, but without damaging the plant itself by the active compound employed. In particular, methods for the treatment of seed should also take into consideration the intrinsic insecticidal properties of transgenic plants in order to achieve optimum protection of the seed and the germinating plant with a minimum of crop protection agents being employed.

The present invention therefore in particular also relates to a method for the protection of seed and germinating plants from attack by pests, by treating the seed with an active compound according to the invention. The invention likewise relates to the use of the active compounds according to the invention for the treatment of seed for protecting the seed and the resultant plant from pests. Furthermore, the invention relates to seed which has been treated with an active compound according to the invention so as to afford protection from pests.

One of the advantages of the present invention is that the particular systemic properties of the active compounds according to the invention mean that treatment of the seed with these active compounds not only protects the seed itself, but also the resulting plants after emergence, from pests. In this manner, the immediate treatment of the crop at the time of sowing or shortly thereafter can be dispensed with.

Furthermore, it must be considered as advantageous that the active compounds according to the invention can also be employed in particular in transgenic seed, the plants arising from this seed being capable of expressing a protein directed against pests. By treating such seed with the active compounds according to the invention, certain pests can be controlled merely by the expression of the, for example, insecticidal protein, and additionally be protected by the active compounds according to the invention against damage.

The active compounds according to the invention are suitable for protecting seed of any plant variety as already mentioned above which is employed in agriculture, in the greenhouse, in forests or in horticulture. In particular, this takes the form of seed of maize, peanut, canola, oilseed rape, poppy, soya beans, cotton, beet (for example sugar beet and fodder beet), rice, sorghum and millet, wheat, barley, oats, rye, sunflower, tobacco, potatoes or vegetables (for example tomatoes, cabbage plants). The active compounds according to the invention are likewise suitable for treating the seed of fruit plants and vegetables as already mentioned above. The treatment of the seed of maize, soya beans, cotton, wheat and canola or oilseed rape is of particular importance.

As already mentioned above, the treatment of transgenic seed with an active compound according to the invention is also of particular importance. This takes the form of seed of plants which, as a rule, comprise at least one heterologous gene which governs the expression of a polypeptide with in particular insecticidal properties. In this context, the heterologous genes in transgenic seed may be derived from microorganisms such as Bacillus, Rhizobium, Pseudomonas, Serratia, Trichoderma, Clavibacter, Glomus or Gliocladium. The present invention is particularly suitable for the treatment of transgenic seed which comprises at least one heterologous gene orignating from Bacillus sp. and whose gene product shows activity against the European corn borer and/or the corn root worm. It is particularly preferably a heterologous gene derived from Bacillus thuringiensis.

In the context of the present invention, the active compound according to the invention is applied to the seed either alone or in a suitable formulation. Preferably, the seed is treated in a state which is stable enough to avoid damage during treatment. In general, the seed may be treated at any point in time between harvest and sowing. The seed usually used has been separated from the plant and freed from cobs, shells, stalks, coats, hairs or the flesh of the fruits.

When treating the seed, care must generally be taken that the amount of the active compound according to the invention applied to the seed and/or the amount of further additives is chosen in such a way that the germination of the seed is not adversely affected, or that the resulting plant is not damaged. This must be borne in mind in particular in the case of active compounds which may have phytotoxic effects at certain application rates. As already mentioned above, it is possible to treat all plants and their parts according to the invention. In a preferred embodiment, wild plant species and plant cultivars, or those obtained by conventional biological breeding methods, such as crossing or protoplast fusion, and parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts", "parts of plants" and "plant parts" have been explained above.

Particularly preferably, plants of the plant cultivars which are in each case commercially available or in use are treated according to the invention. Plant cultivars are to be understood as meaning plants having novel properties ("traits") which have been obtained by conventional breeding, by mutagenesis or by recombinant DNA techniques. These can be cultivars, bio- or genotypes.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, higher quality and/or a higher nutritional value of the harvested products, better storage stability and/or processability of the harvested products are possible, which exceed the effects which were actually to be expected. The transgenic plants or plant cultivars (obtained by genetic engineering) which are preferably to be treated according to the invention include all plants which, by virtue of the genetic modification, received genetic material which imparted particularly advantageous, useful traits to these plants. Examples of such traits are better plant growth, increased tolerance to high or low temperatures, increased tolerance to drought or to water or soil salt content, increased flowering performance, easier harvesting, accelerated maturation, higher harvest yields, higher quality and/or a higher nutritional value of the harvested products, better storage stability and/or processability of the harvested products. Further and particularly emphasized examples of such traits are a better defence of the plants against animal and microbial pests, such as against insects, mites, phytopathogenic fungi, bacteria and/or viruses, and also increased tolerance of the plants to certain herbicidally active compounds. Examples of transgenic plants which may be mentioned are the important crop plants, such as cereals (wheat, rice), maize, soya beans, potatoes, sugar beet, tomatoes, peas and other vegetable varieties, cotton, tobacco, oilseed rape and also fruit plants (with the fruits apples, pears, citrus fruits and grapes), and particular emphasis is given to maize, soya beans, potatoes, cotton, tobacco and oilseed rape. Traits that are emphasized are in particular increased defence of the plants against insects, arachnids, nematodes and slugs and snails by virtue of toxins formed in the plants, in particular those formed in the plants by the genetic material from Bacillus thuringiensis (for example by the genes CryIA(a), CryIA(b), CryIA(c), CryIIA, CryIIIA, CryIIIB2, Cry9c, Cry2Ab, Cry3Bb and CryIF and also combinations thereof) (referred to hereinbelow as "Bt plants") . Traits that are also particularly emphasized are the increased defence of the plants against fungi, bacteria and viruses by systemic acquired resistance (SAR), systemin, phytoalexins, elicitors and resistance genes and correspondingly expressed proteins and toxins. Traits that are furthermore particularly emphasized are the increased tolerance of the plants to certain herbicidally active compounds, for example imidazolinones, sulphonylureas, glyphosate or phosphinotricin (for example the "PAT" gene). The genes which impart the desired traits in question can also be present in combination with one another in the transgenic plants. Examples of "Bt plants" which may be mentioned are maize varieties, cotton varieties, soya bean varieties and potato varieties which are sold under the trade names YIELD GARD® (for example maize, cotton, soya beans), KnockOut® (for example maize), StarLink® (for example maize), Bollgard® (cotton), Nucotn® (cotton) and NewLeaf® (potato). Examples of herbicide-tolerant plants which may be mentioned are maize varieties, cotton varieties and soya bean varieties which are sold under the trade names Roundup Ready® (tolerance to glyphosate, for example maize, cotton, soya bean), Liberty Link® (tolerance to phosphinotricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize) . Of course, these statements also apply to plant cultivars having these genetic traits or genetic traits still to be developed, which plant cultivars will be developed and/or marketed in the future. The plants listed can be treated according to the invention in a particularly advantageous manner with the active compounds of the general formula I according to the invention. The preferred ranges stated above for the active compounds also apply to the treatment of these plants. Particular emphasis is given to the treatment of plants with the active compounds specifically mentioned in the present text.

The active compounds according to the invention act not only against plant, hygiene and stored product pests, but also in the veterinary medicine sector against animal parasites (ecto- and endoparasites), such as hard ticks, soft ticks, mange mites, leaf mites, flies (biting and licking), parasitic fly larvae, lice, hair lice, feather lice and fleas. These parasites include:
From the order of the Anoplurida, for example, Haematopinus spp. , Linognathus spp., Pediculus spp., Phtirus spp., Solenopotes spp.
From the order of the Mallophagida and the suborders Amblycerina and Ischnocerina, for example, Trimenopon spp., Menopon spp., Trinoton spp., Bovicola spp., Werneckiella spp., Lepikentron spp., Damalina spp., Trichodectes spp., Felicola spp.
From the order of the Diptera and the suborders Nematocerina and Brachycerina, for example, Aedes spp., Anopheles spp., Culex spp., Simulium spp., Eusimulium spp., Phlebotomus spp., Lutzomyia spp. , Culicoides spp., Chrysops spp., Hybomitra spp. , Atylotus spp., Tabanus spp., Haematopota spp., Philipomyia spp. , Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
From the order of the Siphonapterida, for example, Pulex spp., Ctenocephalides spp., Xenopsylla spp., Ceratophyllus spp. From the order of the Heteropterida, for example, Cimex spp., Triatoma spp., Rhodnius spp., Panstrongylus spp.
From the order of the Blattarida, for example, Blatta orientalis, Periplaneta americana, Blattela germanica, Supella spp. From the subclass of the Acari (Acarina) and the orders of the Meta- and Mesostigmata, for example, Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.
From the order of the Actinedida (Prostigmata) and Acaridida (Astigmata), for example, Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

The active compounds of the formula (I) according to the invention are also suitable for controlling arthropods which infest agricultural productive livestock, such as, for example, cattle, sheep, goats, horses, pigs, donkeys, camels, buffalo, rabbits, chickens, turkeys, ducks, geese and bees, other pets, such as, for example, dogs, cats, caged birds and aquarium fish, and also so-called test animals, such as, for example, hamsters, guinea pigs, rats and mice. By controlling these arthropods, cases of death and reduction in productivity (for meat, milk, wool, hides, eggs, honey etc.) should be diminished, so that more economic and easier animal husbandry is possible by use of the active compounds according to the invention.

The active compounds according to the invention are used in the veterinary sector and in animal husbandry in a known manner by enteral administration in the form of, for example, tablets, capsules, potions, drenches, granules, pastes, boluses, the feed-through process and suppositories, by parenteral administration, such as, for example, by injection (intramuscular, subcutaneous, intravenous, intraperitoneal and the like), implants, by nasal administration, by dermal use in the form, for example, of dipping or bathing, spraying, pouring on and spotting on, washing and powdering, and also with the aid of moulded articles containing the active compound, such as collars, ear marks, tail marks, limb bands, halters, marking devices and the like.

When used for cattle, poultry, pets and the like, the active compounds of the formula (I) can be used as formulations (for example powders, emulsions, free-flowing compositions), which comprise the active compounds in an amount of 1 to 80% by weight, directly or after 100 to 10 000-fold dilution, or they can be used as a chemical bath.

It has furthermore been found that the active compounds according to the invention also have a strong insecticidal action against insects which destroy industrial materials. The following insects may be mentioned as examples and as preferred - but without any limitation:
Beetles, such as Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hymenopterons, such as Sirex juvencus, Urocerus gigas, Urocerus gigas taignus, Urocerus augur;
Termites, such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Bristletails, such as Lepisma saccharina.

Industrial materials in the present connection are to be understood as meaning non-living materials, such as, preferably, plastics, adhesives, sizes, papers and cardboards, leather, wood and processed wood products and coating compositions. The ready-to-use compositions may, if appropriate, comprise further insecticides and, if appropriate, one or more fungicides.

With respect to possible additional additives, reference may be made to the insecticides and fungicides mentioned above. The active compounds according to the invention can likewise be employed for protecting objects which come into contact with seawater or brackish water, such as hulls, screens, nets, buildings, moorings and signalling systems, against fouling. Furthermore, the active compounds according to the invention, alone or in combinations with other active compounds, may be employed as antifouling agents.

In domestic, hygiene and stored-product protection, the active compounds are also suitable for controlling animal pests, in particular insects, arachnids and mites, which are found in enclosed spaces such as, for example, dwellings, factory halls, offices, vehicle cabins and the like. They can be employed alone or in combination with other active compounds and auxiliaries in domestic insecticide products for controlling these pests. They are active against sensitive and resistant species and against all developmental stages. These pests include: From the order of the Scorpionidea, for example, Buthus occitanus.

From the order of the Acarina, for example, Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae. From the order of the Araneae, for example, Aviculariidae, Araneidae.

From the order of the Opiliones, for example, Pseudoscorpiones chelifer, Pseudoscorpiones cheiridium, Opiliones phalangium. From the order of the Isopoda, for example, Oniscus asellus, Porcellio scaber.

From the order of the Diplopoda, for example, Blaniulus guttulatus, Polydesmus spp.

From the order of the Chilopoda, for example, Geophilus spp. From the order of the Zygentoma, for example, Ctenolepisma spp. , Lepisma saccharina, Lepismodes inquilinus.

From the order of the Blattaria, for example, Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

From the order of the Saltatoria, for example, Acheta domesticus.

From the order of the Dermaptera, for example, Forficula auricularia.

From the order of the Isoptera, for example, Kalotermes spp., Reticulitermes spp.

From the order of the Psocoptera, for example, Lepinatus spp., Liposcelis spp.

From the order of the Coleoptera, for example, Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum. From the order of the Diptera, for example, Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa. From the order of the Lepidoptera, for example, Achroia grisella, Galleria mellonella, Plodia interpunctella, Tinea cloacella, Tinea pellionella, Tineola bisselliella.

From the order of the Siphonaptera, for example, Ctenocephalides canis, Ctenocephalides felis, Pulex irritans, Tunga penetrans, Xenopsylla cheopis.

From the order of the Hymenoptera, for example, Camponotus herculeanus, Lasius fuliginosus, Lasius niger, Lasius umbratus, Monomorium pharaonis, Paravespula spp., Tetramorium caespitum.

From the order of the Anoplura, for example, Pediculus humanus capitis, Pediculus humanus corporis, Pemphigus spp., Phylloera vastatrix, Phthirus pubis.

From the order of the Heteroptera, for example, Cimex hemipterus, Cimex lectularius, Rhodinus prolixus, Triatoma infestans.

In the field of household insecticides, the active compounds according to the invention are used alone or in combination with other suitable active compounds, such as phosphoric esters, carbamates, pyrethroids, neonicotinoids, growth regulators or active compounds from other known classes of insecticides. The active compounds according to the invention are used in aerosols, pressure-free spray products, for example pump and atomizer sprays, automatic fogging systems, foggers, foams, gels, evaporator products with evaporator tablets made of cellulose or polymer, liquid evaporators, gel and membrane evaporators, propeller-driven evaporators, energy-free, or passive, evaporation systems, moth papers, moth bags and moth gels, as granules or dusts, in baits for spreading or in bait stations.

Next, the present invention will be illustrated further specifically by examples but the present invention should not be limited to only these examples.

### Synthesis Example 1 (final substance synthesis)

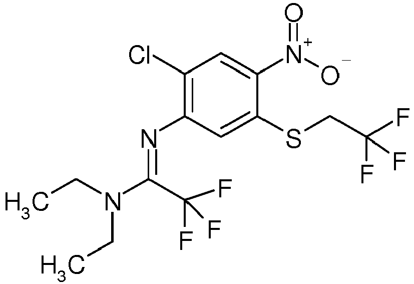

### Synthesis of N'-{2-chloro-4-nitro-5-[(2,2,2-trifluoroethyl)thio]phenyl}-N,N-diethyl-2,2,2-trifluoroethaneimideamide

To a 50 ml DMF solution of 0.8 g (2.2 mmol) of N'-(2,5-dichloro-4-nitrophenyl)-N,N-diethyl-2,2,2-trifluoro ethaneimideamide was added 0.4 g (3.4 mmol) of 2,2,2-trifluoroethanethiol and 0.5 g (3.6 mmol) of potassium carbonate, and stirred at room temperature for 15 hours. The reaction solution was poured into ice water, extracted with ethyl acetate, and the solvent was distilled off under reduced pressure, then, the residue was purified by silica gel column chromatography, to obtain 0.85 g of the aimed compound in the form of pale yellow oil (yield: 87%, nD20 1.5743).

### Synthesis Example 2 (final substance synthesis)

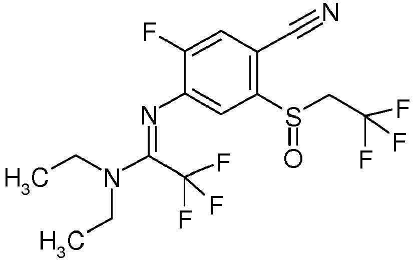

### Synthesis of N'-{4-cyano-2-fluoro-5-[(2,2,2-trifluoroethyl)sulfinyl]phen yl}-N,N-diethyl-2,2,2-trifluoroethaneimideamide

To a 50 ml dichloromethane solution of 0.4 g (1.0 mmol) of N'-{4-cyano-2-fluoro-5-[(2,2,2-trifluoroethyl)thio]phenyl}-N,N-diethyl-2,2,2-trifluoroethaneimideamide was added 0.3 g (purity 70%, 1.2 mmol) of m-chloroperbenzoic acid under ice cooling and the temperature was returned to room temperature without any other treatment and stirred for 10 hours. After completion of the reaction, the reaction solution was washed twice with saturated aqueous sodium hydrogen carbonate solution, and the solvent was distilled off under reduced pressure, then, the residue was purified by silica gel column chromatography, to obtain 0.3 g of the aimed compound in the form of white crystal (yield: 720, mp 103 to 105°C).

### Synthesis Example 3 (final substance synthesis)

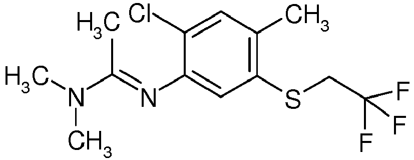

### Synthesis of N'-{2-chloro-4-methyl-5-[(2,2,2-trifluoroethyl)thio]phenyl} -N,N-dimethylethaneimideamide

A mixed solution of 0.58 g (2.2 mmol) of 2-chloro-4-methyl-2-[(2,2,2-trifluoroethyl)thio]aniline, 0.4 g (4.5 mmol) of N,N-dimethylacetamide and 1 ml (11 mmol) of phosphorus oxychloride was stirred at 100°C for 3 hours. The reaction solution was cooled, then, poured into ice water and made alkaline with a NaOH aqueous solution, then, extracted with ethyl acetate. The solvent was distilled off under reduced pressure, then, the residue was purified by silica gel column chromatography, to obtain 0.18 g of the aimed compound in the form of white crystal (yield: 24%, mp 66 to 71°C).

### Synthesis Example 4-1 (raw material synthesis)

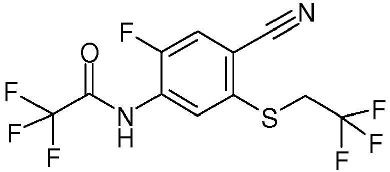

### Synthesis of N-{4-cyano-2-fluoro-5-[(2,2,2-trifluoroethyl)thio]phenyl}-2 ,2,2-trifluoroacetamide

To a 50 ml dichloromethane solution of 4.0 g (16.0 mmol) of 4-amino-5-fluoro-2-[(2,2,2-trifluoroethyl)thio]benzonitrile was added 2.0 g (19.8 mmol) of triethylamine, and a 20 ml dichloromethane solution of 4.0 g (19.0 mmol) of trifluoroacetic anhydride was dropped under ice cooling and the temperature was returned to room temperature without any other treatment and stirred for 1 hour. After completion of the reaction, the reaction solution was washed with saturated saline, and the solvent was distilled off under reduced pressure, then, the residue was purified by silica gel column chromatography, to obtain 4.0 g of the aimed compound in the form of pale yellow oil (yield: 78%).

### Synthesis Example 4-2 (raw material synthesis)

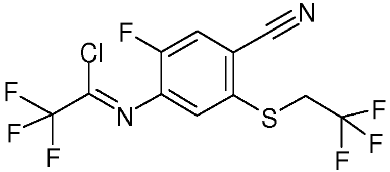

### Synthesis of N-{4-cyano-2-fluoro-5-[(2,2,2-trifluoroethyl)thio]phenyl}-2 ,2,2-trifluoroethaneimideyl chloride

To a 100 ml dichloromethane solution of 11.5 g (33.2 mmol) of N-{4-cyano-2-fluoro-5-[(2,2,2-trifluoroethyl)thio]phenyl}-2 ,2,2-trifluoroacetamide obtained above was added 12.0 g (45.8 mmol) of triphenyl phosphine and 8.0 g (52.0 mmol) of carbon tetrachloride was dropped at about 30°C, then , the mixture was heated under reflux for 5 hours. After completion of the reaction, the solvent was distilled off under reduced pressure, then, the residue was purified by silica gel column chromatography, to obtain 10.2 g of the aimed compound in the form of pale yellow oil (yield: 84%).

### Synthesis Example 4-3 (final substance synthesis)

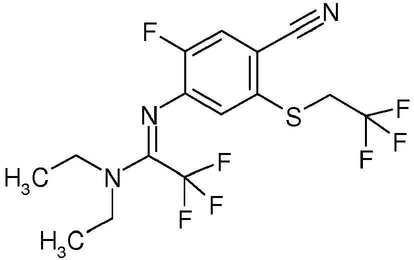

### Synthesis of N'-{4-cyano-2-fluoro-5-[(2,2,2-trifluoroethyl)thio]phenyl}-N,N-diethyl-2,2,2-trifluoroethaneimideamide

To a 30 ml acetonitrile solution of 0.2 g (2.7 mmol) of diethylamine was added a 10 ml acetonitrile solution of 0.2 g (0.5 mmol) of N-{4-cyano-2-fluoro-5-[(2,2,2-trifluoroethyl)thio]phenyl}-2 ,2,2-trifluoroethaneimideyl chloride obtained above, and the mixture was stirred at room temperature for 1 hour. After completion of the reaction, the solvent was distilled off under reduced pressure, then, the residue was purified by silica gel column chromatography, to obtain 0.15 g of the aimed compound in the form of colorless oil (yield: 68%, nD20 1.5187).

### Synthesis Example 5-1 (raw material synthesis)

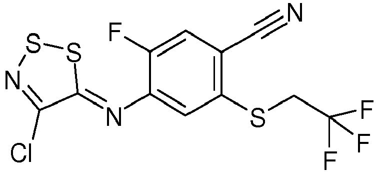

### Synthesis of 4-[(4-chloro-5H-1,2,3-dithiazol-5-ylidene)amino]-5-fluoro-2 -[(2,2,2-trifluoroethyl)thio]benzonitrile

To a 50 ml dichloromethane solution of 3.2 g (12.8 mmol) of 4-amino-5-fluoro-2-[(2,2,2-trifluoroethyl)thio]benzonitrile was added 3.0 g (14.4 mmol) of 4,5-dichloro-1,2,3-dithiazol-1-ium chloride and the mixture was stirred at room temperature for 2 hours. Then, at room temperature, a 20 ml dichloromethane solution of 2.2 g (27.8 mmol) of pyridine was dropped, and the mixture was stirred at room temperature for 1 hour without any other operation. After completion of the reaction, the reaction solution was washed with saturated saline, the solvent was distilled off under reduced pressure, then, the residue was purified by silica gel column chromatography, to obtain 4.1 g of the aimed compound in the form of yellow crystal (yield: 83%).

### Synthesis Example 5-2 (raw material synthesis)

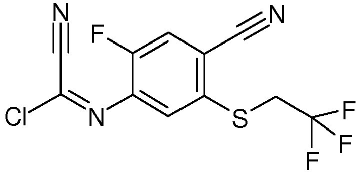

### Synthesis of 1-{[chloro(cyano)methylene]amino}-4-cyano-2-fluoro-5-[(2,2, 2-trifluoroethyl)thio]benzene

3 g of 4-[(4-chloro-5H-1,2,3-dithiazol-5-ylidene)amino]-5-fluoro-2 -[(2,2,2-trifluoroethyl)thio]benzonitrile obtained above was heated at 200°C while stirring for 5 minutes. After cooling, the reaction substance was dissolved in dichloromethane, and insoluble substances were removed by filtration. The solvent was distilled off under reduced pressure, then, the residue was purified by silica gel column chromatography, to obtain 1.8 g of the aimed compound in the form of white crystal (yield: 72%) .

### Synthesis Example 5-3 (final substance synthesis)

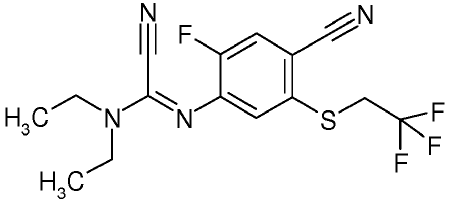

### Synthesis of 1-cyano-4-{[cyano(diethylamino)methylene]amino}-5-fluoro-2-[(2,2,2-trifluoroethyl)thio]benzene

Into a 30 ml acetonitrile solution of 0.2 g (2.7 mmol) of diethylamine was dropped a 10 ml acetonitrile solution of 0.3 g (0.9 mmol) of 1-{[chloro(cyano)methylene]amino}-4-cyano-2-fluoro-5-[(2,2, 2-trifluoroethyl)thio]benzene, and the mixture was stirred at room temperature for 1 hour. After completion of the reaction, the solvent was distilled off under reduced pressure, then, the residue was purified by silica gel column chromatography, to obtain 0.2 g of the aimed compound in the form of colorless oil (yield: 60%, nD20 1.5538).

### Synthesis Example 6-1 (raw material synthesis)

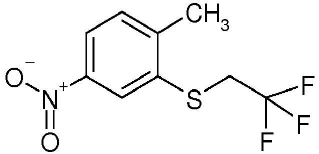

### Synthesis of 1-methyl-4-nitro-2-[(2,2,2-trifluoroethyl)thio]benzene

A DMF solution of 1.1 g (7.1 mmol) of 2-fluoro-1-methyl-4-nitrobenzene, 1.0 g (8.6 mol) of 2,2,2-trifluoroethanethiol and 1.19 g (8.6 mmol) of potassium carbonate was stirred at 50°C for 1 hour. The reaction solution was poured into water, and extracted with ethyl acetate. The solvent was distilled off under reduced pressure, then, the residue was purified by silica gel column chromatography, to obtain 0.49 g of the aimed compound in the form of white crystal (yield: 27%).

### Synthesis Example 6-2 (raw material synthesis)

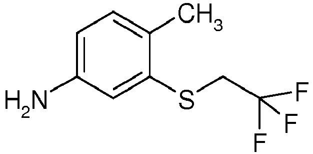

### Synthesis of 4-methyl-3-[(2,2,2-trifluoroethyl)thio]aniline

A methanol solution of 1.0 g (4 mmol) of 1-methyl-4-nitro-2-[(2,2,2-trifluoroethyl)thio]benzene obtained above and 1.9 g (8 mmol) of nickel chloride was cooled to 0°C, and 0.6 g (16 mmol) of sodium borohydride was added portion-wise. The mixture was stirred at room temperature for 1 hour. The solvent was distilled off, and 2 N hydrochloric acid was added to the residue. Then, 28% ammonia water was added and extracted with ethyl acetate. The solvent was distilled off under reduced pressure, then, the residue was washed with hexane to obtain 0.4 g (yield: 45%) of crystal.

### Synthesis Example 7-1 (raw material synthesis)

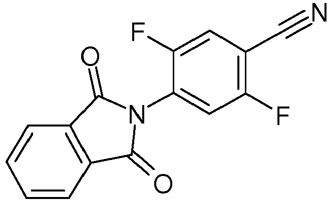

### Synthesis of 4-(1,3-dioxo-1,3-dihydro-2H-isoindol-2-yl)-2,5-difluorobenz onitrile

To a 100 ml DMF solution of 16.0 g (101.6 mmol) of 2,4,5-trifluorobenzonitrile was added 20.0 g (108.0 mmol) of potassium phthalimide, and the mixture was heated at 100 °C while stirring for 5 hours. After cooling, the reaction solution was poured into ice water, and extracted with an ethyl acetate, THF mixed solvent, and the solvent was distilled off under reduced pressure. The remaining crystal was washed with a small amount of ethyl acetate to obtain 24.7 g of the aimed compound in the form of white crystal (yield: 85%).

### Synthesis Example 7-2 (raw material synthesis)

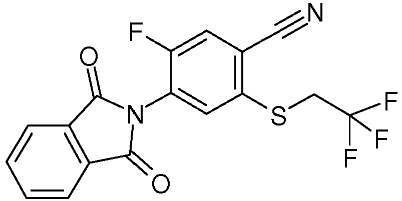

### Synthesis of 4-(1,3-dioxo-1,3-dihydro-2H-isoindol-2-yl)-5-fluoro-2-[(2,2 ,2-trifluoroethyl)thio]benzonitrile

To a 100 ml DMF solution of 8.5 g (29.9 mmol) of 4-(1,3-dioxo-1,3-dihydro-2H-isoindol-2-yl)-2,5-difluorobenz onitrile obtained above was added 5.0 g (36.2 mmol) of potassium carbonate, and a 100 ml DMF solution of 3.5 g (30.1 mmol) of 2,2,2-trifluoroethanethiol was dropped under ice cooling over 1 hour. The temperature was returned to room temperature and the mixture was stirred for 1 hour, then, the reaction solution was poured into ice water, and extracted with ethyl acetate. The solvent was distilled off under reduced pressure to give a residue which was used in the subsequent reaction without purification, obtaining 11.0 g (yield: 97%) of the aimed compound in the form of brown slurry.

### Synthesis Example 7-3 (raw material synthesis)

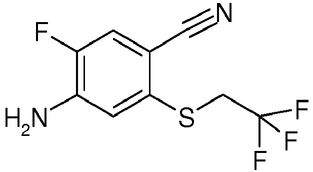

### Synthesis of 4-amino-5-fluoro-2-[(2,2,2-trifluoroethyl)thio]benzonitrile

To a 150 ml ethanol solution of 40.0 g (105.2 mmol) of 4-(1,3-dioxo-1,3-dihydro-2H-isoindol-2-yl)-5-fluoro-2-[(2,2 ,2-trifluoroethyl)thio]benzonitrile obtained above was added 7.0 g (139.8 mmol) of hydrazine-hydrate, and the mixture was heated under reflux for 5 hours, then, the temperature was returned to room temperature, and insoluble materials were filtrated. The solvent was distilled off, and the residue was dissolved in ethyl acetate, and washed with a IN sodium hydroxide solution. The solvent was distilled off under reduced pressure, and the residue was purified by silica gel column chromatography, to obtain 10.0 g (yield: 38%) of the aimed compound in the form of white crystal.

### Synthesis Example 8-1 (raw material synthesis)

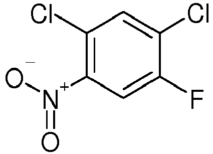

### Synthesis of 1,5-dichloro-2-fluoro-4-nitrobenzene

5.0 g (30 mmol) of 2,4-dichloro-1-fluorobenzene was stirred in 40 ml of concentrated sulfuric acid under ice cooling, and 3.1 g (30 mmol) of potassium nitrate was added to this stirring solution. The temperature was returned to room temperature, and the mixture was stirred for 12 hours, then, poured into ice water, and extracted with ethyl acetate. The organic layer was washed with water and saturated saline, then, dried over sodium sulfate, and the solvent was distilled off under reduced pressure to obtain 6.2 g (yield: 97%) of the aimed compound.

### Synthesis Example 8-2 (raw material synthesis)

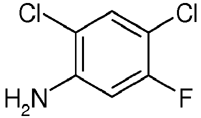

### Synthesis of 2,4-dichloro-5-fluoroaniline

To a 500 ml methanol solution of 10.7 g (45 mmol) of nickel chloride hexa-hydrate was gradually added 5.1 g (135 mmol) of sodium borohydride under ice cooling. The temperature was returned to room temperature, and the mixture was stirred for 1 hour, then, 19.0 g (90 mmol) of 1,5-dichloro-2-fluoro-4-nitrobenzene obtained above was added, and cooled with ice. To this was gradually added 12.0 g (317 mmol) of sodium borohydride. The mixture was stirred for 1 hour, then, the solvent was distilled off under reduced pressure. To the residue was added 150 ml of a 1 N hydrochloric acid aqueous solution, 150 ml of ammonia water and 200 ml of ethyl acetate in this order and the mixture was stirred. Insoluble materials were filtrated through celite, and an organic layer was washed with saturated saline, dried over sodium sulfate, and distilled off under reduced pressure. The residue was purified by silica gel column chromatograph to obtain 14.8 g (yield: 91%) of the aimed compound.

### Synthesis Example 8-3 (raw material synthesis)

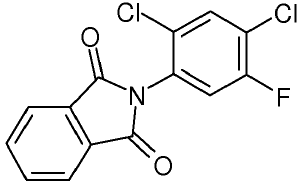

### Synthesis of 2-(2,4-dichloro-5-fluorophenyl)-1H-isoindol-1,3(2H)-dione

A mixture of 3.0 g (16.7 mmol) of 2, 4-dichloro-5-fluoroaniline and 2.5 g (16.7 mmol) of phthalic anhydride was heated under reflux for 4 hours in 40 ml of acetic acid. The mixture was cooled to room temperature, then, the solvent was distilled off under reduced pressure. The resulting residue was washed with hexane, and filtrated to obtain 3.5 g (yield: 68%) of the aimed compound.

### Synthesis Example 8-4 (raw material synthesis)

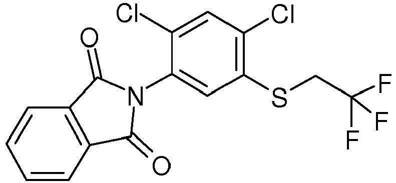

### Synthesis of 2-{2,4-dichloro-5-[(2,2,2-trifluoroethyl)thio]phenyl}-1H-is oindol-1,3(2H)-dione

To a 20 ml DMF solution of 2.6 g (8.6 mmol) of 2-(2,4-dichloro-5-fluorophenyl)-1H-isoindol-1,3(2H)-dione obtained above and 1.79 g (12.9 mmol) of potassium carbonate was added 1.0 g (8.6 mmol) of 2,2,2-trifluoroethanethiol while cooling with ice under an argon atmosphere, and the mixture was stirred at room temperature for 12 hours. The solution was diluted with ethyl acetate, then, washed with water and saturated saline, then, dried over sodium sulfate. The solvent was distilled off under reduced pressure, to obtain 2.8 g (yield: 80%) of the aimed compound.

### Synthesis Example 8-5 (raw material synthesis)

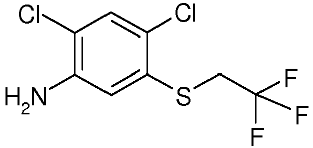

### Synthesis of 2,4-dichloro-5-[(2,2,2-trifluoroethyl)thio]aniline

To a 30 ml ethanol solution of 2.8 g (6.9 mmol) of 2-{2,4-dichloro-5-[(2,2,2-trifluoroethyl)thio]phenyl}-1H-is oindol-1,3 (2H) -dione obtained above was added 0.38 g (7.6 mmol) of hydrazine mono-hydrate, and the mixture was stirred at room temperature for 12 hours. The precipitate was filtrated, and the solvent was distilled off under reduced pressure, and the residue was purified by silica gel chromatography to obtain 1.4 g (yield: 73%) of the aimed compound.

### Synthesis Example 9-1 (raw material synthesis)

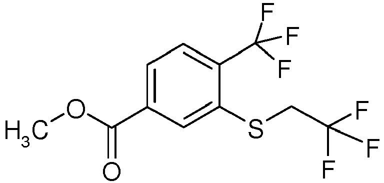

### Synthesis of methyl 3-[(2,2,2-trifluoroethyl)thio]-4-(trifluoromethyl)benzoate

To a 200 ml acetonitrile solution of 14.0 g (63.0 mmol) of methyl 3-fluoro-4-(trifluoromethyl)benzoate was added 10.0 g (86.1 mmol) of 2,2,2-trifluoroethanethiol and 12.0 g (86.8 mmol) of potassium carbonate, and the mixture was stirred at room temperature for 15 hours. The reaction solution was filtrated to remove insoluble materials, then, the filtrate was distilled under reduced pressure, and the residue was purified by silica gel column chromatography to obtain 10.9 g (yield: 54%) of the aimed compound in the form of pale brown oil.

### Synthesis Example 9-2 (raw material synthesis)

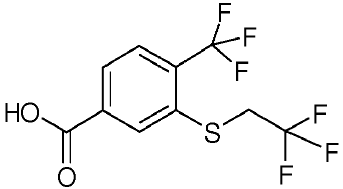

### Synthesis of 3-[(2,2,2-trifluoroethyl)thio]-4-(trifluoromethyl)benzoic acid

To a 10 ml dioxane solution of 7.2g (22.6 mmol) of methyl 3-[(2,2,2-trifluoroethyl)thio]-4-(trifluoromethyl)benzoate obtained above was added 100 ml of water containing 2.0 g (50.0 mmol) of sodium hydroxide dissolved therein, and the mixture was stirred at room temperature for 15 hours. The reaction solution was washed with toluene, then, concentrated hydrochloric acid was added to an aqueous layer to attain pH 1, and the deposited crystal was filtrated. The filtrated crystal was dried, to obtain 5.2 g (yield: 75%) of the aimed compound in the form of white crystal.

### Synthesis Example 9-3 (raw material synthesis)

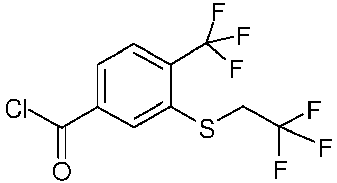

### Synthesis of 3-[(2,2,2-trifluoroethyl)thio]-4-(trifluoromethyl)benzoic chloride

To a 50 ml 1,2-dichloroethane solution of 7.1g (23.3 mmol) of 3-[(2,2,2-trifluoroethyl)thio]-4-(trifluoromethyl)benzoic acid obtained above was added 3 drops of DMF and 4.0 g (33.6 mmol) of thionyl chloride, and the mixture was heated under reflux for 5 hours. After completion of the reaction, the solvent was distilled off under reduced pressure, to obtain 7.4 g (yield: 98%) of a pale yellow aimed compound.

### Synthesis Example 9-4 (raw material synthesis)

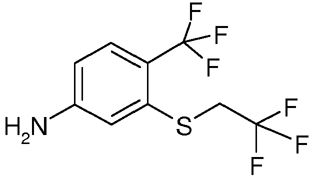

### Synthesis of 3-[(2,2,2-trifluoroethyl)thio]-4-(trifluoromethyl)aniline

To a 50 ml aqueous solution of 1.4 g (21.5 mmol) of sodium azide was added 30 ml of toluene and 200 mg of triethylbenzylammonium chloride, and a 30 ml toluene solution of 6.0 g (18.6 mmol) of 3-[(2,2,2-trifluoroethyl)thio]-4-(trifluoromethyl)benzoic chloride obtained above was dropped slowly while stirring vigorously under ice cooling. After completion of dropping, the temperature was returned to room temperature and the mixture was stirred vigorously for 1 hour at the same temperature, and an organic layer was separated and dried over magnesium sulfate. Magnesium sulfate was removed by filtration, then, the filtrate toluene solution was heated under reflux for 5 hours without any other operation, and the temperature was returned to room temperature, then, a mixed solution of 20 ml of acetic acid and 20 ml of water was dropped slowly. After completion of dropping, the mixture was heated under reflux again for 1 hour without any other operation, and the temperature of the reaction solution was returned to room temperature, then, the reaction solution was washed with saturated saline, then, with saturated sodium hydrogen carbonate solution. The solvent was distilled off under reduced pressure, then, the residue was purified by silica gel column chromatography, to obtain 2.0 g (yield: 39%) of the aimed compound in the form of white crystal.

### Synthesis Example 10 (raw material synthesis)

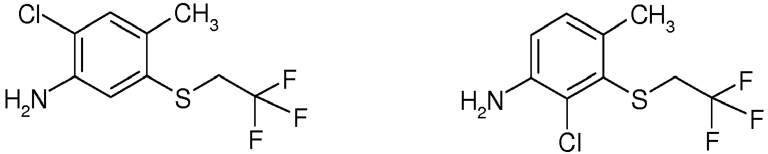

### Synthesis of 2-chloro-4-methyl-5-[(2,2,2-trifluoroethyl)thio]aniline and 2-chloro-4-methyl-3-[(2,2,2-trifluoroethyl)thio]aniline

An acetic acid solution of 0.56 g (2.5 mmol) of 4-methy-3-[(2,2,2-trifluoroethyl)thio]aniline and 0.41 g (3 mmol) of N-chlorosuccinic imide was stirred at 70°C for 2 hours. The solvent was distilled off under reduced pressure, then, the residue was separated by silica gel column chromatography, to obtain each 0.07 g (yield: 11%) of two kinds of isomers of the aimed compound.

### Synthesis Example 11 (final substance synthesis)

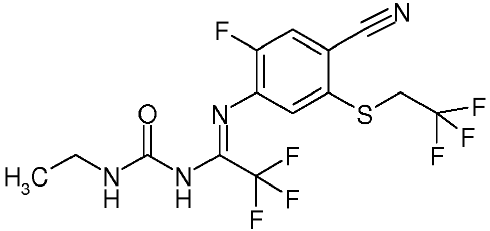

### Synthesis of N'-{4-cyano-2-fluoro-5-[(2,2,2-trifluoroethyl)thio]phenyl}-N-[(ethylamino)carbonyl]-2,2,2-trifluoroethaneimideamide

To a 50 ml DMF solution of 0.2 g (0.6 mmol) of N'-{4-cyano-2-fluoro-5-[(2,2,2-trifluoroethyl)thio]phenyl}-2,2,2-trifluoroethaneimideamide was added 0.05 g (0.7 mmol) of ethyl isocyanate, and at room temperature, 0.05 g (1.3 mmol) of sodium hydride (purity: 60%) was added portion-wise. The mixture was stirred for 15 hours at room temperature without any other operation, then, the reaction solution was poured into ice water, and extracted with ethyl acetate, and the solvent was distilled off under reduced pressure, then, the residue was purified by silica gel column chromatography, to obtain 0.15 g (yield: 62%, nD20 1.5215) of the aimed compound in the form of colorless oil.

The compounds of the formula (I) which can be obtained in the same manner as in the above-exemplified synthesis examples of raw material and final substance are shown in Table 1 and Table 2, and compounds of the formula (IV-A) are shown in Table 3.

In the above-mentioned synthesis examples, also aimed compounds correlated with final substance synthesis are shown in Table 1.
[TABLE 1]

**Table 1**

| | | | | | | | | | |
|---|---|---|---|---|---|---|---|---|---|
| 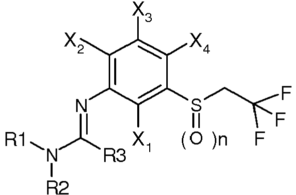 | | | | | | | | | |

| Comp. No. | R1 | R2 | R3 | X1 | X2 | X3 | X4 | n | nD20 or m.p. |
|---|---|---|---|---|---|---|---|---|---|
| I-1 | H | H | CF3 | H | H | H | CN | 0 | |
| I-2 | H | H | CF3 | H | H | H | CN | 1 | |
| I-3 | CH3 | H | CF3 | H | H | H | CN | 0 | 1.5312 |
| I-4 | CH3 | H | CF3 | H | H | H | CN | 1 | 143-145 |
| I-5 | CH3 | CH3 | CF3 | H | H | H | CN | 0 | |
| I-6 | CH3 | CH3 | CF3 | H | H | H | CN | 1 | |
| I-7 | CH2CF3 | H | CF3 | H | H | H | CN | 0 | 1.4942 |
| I-8 | CH2CF3 | H | CF3 | H | H | H | CN | 1 | |
| I-9 | i-Pr | H | CF3 | H | H | H | CN | 0 | 51-53 |
| I-10 | i-Pr | H | CF3 | H | H | H | CN | 1 | 96-98 |
| I-11 | CH2CH3 | CH2CH3 | CF3 | H | H | H | CN | 0 | 1.5465 |
| I-12 | CH2CH3 | CH2CH3 | CF3 | H | H | H | CN | 1 | |
| I-13 | -CH2CH=CH2 | H | CF3 | H | H | H | CN | 0 | |
| I-14 | -CH2CH=CH2 | H | CF3 | H | H | H | CN | 1 | |
| I-15 | -CH2CΞCH | H | CF3 | H | H | H | CN | 0 | 85-86 |
| I-16 | -CH2CΞCH | H | CF3 | H | H | H | CN | 1 | 147-149 |
| I-17 | CH3 | CH3 | CN | H | H | H | CN | 0 | |
| I-18 | CH3 | CH3 | CN | H | H | H | CN | 1 | |
| I-19 | CH3 | CH3 | H | H | H | H | CN | 0 | |
| I-20 | CH3 | CH3 | H | H | H | H | CN | 1 | |
| I-21 | CH3 | CH3 | CH3 | H | H | H | CN | 0 | |
| I-22 | CH3 | CH3 | CH3 | H | H | H | CN | 1 | |
| I-23 | CH3 | CH3 | Ph | H | H | H | CN | 0 | |
| I-24 | CH3 | CH3 | Ph | H | H | H | CN | 1 | |
| I-25 | -C2H4- | CH3 | -C2H4- | H | H | H | CN | 0 | |
| I-26 | -C2H4- | CH3 | -C2H4- | H | H | H | CN | 1 | |
| I-27 | -CO- | CH3 | -C2H4- | H | H | H | CN | 0 | |
| I-28 | -CO- | CH3 | -C2H4- | H | H | H | CN | 1 | |
| I-29 | H | H | CF3 | H | F | H | CN | 0 | 145-146 |
| I-30 | H | H | CF3 | H | F | H | CN | 1 | 190-192 |
| I-31 | CH3 | H | CF3 | H | F | H | CN | 0 | 1.5217 |
| I-32 | CH3 | H | CF3 | H | F | H | CN | 1 | 109-112 |
| I-33 | CH3 | CH3 | CF3 | H | F | H | CN | 0 | 1.53 |
| I-34 | CH3 | CH3 | CF3 | H | F | H | CN | 1 | 158-159 |
| I-35 | C2H5 | H | CF3 | H | F | H | CN | 0 | |
| I-36 | C2H5 | H | CF3 | H | F | H | CN | 1 | |
| I-37 | CH2CF3 | H | CF3 | H | F | H | CN | 0 | 1.4853 |
| I-38 | CH2CF3 | H | CF3 | H | F | H | CN | 1 | 120-124 |
| I-39 | n-Pr | H | CF3 | H | F | H | CN | 0 | 87-89 |
| I-40 | n-Pr | H | CF3 | H | F | H | CN | 1 | 87-90 |
| I-41 | i-Pr | H | CF3 | H | F | H | CN | 0 | 1.5109 |
| I-42 | i-Pr | H | CF3 | H | F | H | CN | 1 | 146-148 |
| I-43 | CH2CH3 | CH2CH3 | CF3 | H | F | H | CN | 0 | 1.5187 |
| I-44 | CH2CH3 | CH2CH3 | CF3 | H | F | H | CN | 1 | 103-105 |
| I-45 | -CH2CH2- | -CH2CH2- | CF3 | H | F | H | CN | 0 | 1.5433 |
| I-46 | -CH2CH2- | -CH2CH2- | CF3 | H | F | H | CN | 1 | |
| I-47 | -CH2CH2O- | -CH2CH2- | CF3 | H | F | H | CN | 0 | 1.533 |
| I-48 | -CH2CH2O- | -CH2CH2- | CF3 | H | F | H | CN | 1 | |
| I-49 | cyc-Pr | H | CF3 | H | F | H | CN | 0 | 77-78 |
| I-50 | cyc-Pr | H | CF3 | H | F | H | CN | 1 | 129-131 |
| I-51 | -CH2CH=CH2 | H | CF3 | H | F | H | CN | 0 | 79-88 |
| I-52 | -CH2CH=CH2 | H | CF3 | H | F | H | CN | 1 | 50-54 |
| I-53 | -CH2C≡CH | H | CF3 | H | F | H | CN | 0 | 77-78 |
| I-54 | -CH2C≡CH | H | CF3 | H | F | H | CN | 1 | 1.5103 |
| I-55 | C2H5 | CH3 | CF3 | H | F | H | CN | 0 | 79-88 |
| I-56 | C2H5 | CH3 | CF3 | H | F | H | CN | 1 | |
| I-57 | cyc-C6H11 | C2H5 | CF3 | H | F | H | CN | 0 | 1.5305 |
| I-58 | cyc-C6H11 | C2H5 | CF3 | H | F | H | CN | 1 | |
| I-59 | Ph | H | CF3 | H | F | H | CN | 0 | |
| I-60 | Ph | H | CF3 | H | F | H | CN | 1 | |
| I-61 | 4-CF3-Ph | H | CF3 | H | F | H | CN | 0 | 141-144 |
| I-62 | 4-CF3-Ph | H | CF3 | H | F | H | CN | 1 | 197-200 |
| I-63 | -CH2Ph | H | CF3 | H | F | H | CN | 0 | |
| I-64 | -CH2Ph | H | CF3 | H | F | H | CN | 1 | |
| I-65 | -CH2-4-CF3-Ph | H | CF3 | H | F | H | CN | 0 | 99-103 |
| I-66 | -CH2-4-CF3-Ph | H | CF3 | H | F | H | CN | 1 | |
| I-67 | -OCH3 | H | CF3 | H | F | H | CN | 0 | 86-90 |
| I-68 | -OCH3 | H | CF3 | H | F | H | CN | 1 | 112-119 |
| I-69 | -OCH3 | CH3 | CF3 | H | F | H | CN | 0 | 1.5123 |
| I-70 | -OCH3 | CH3 | CF3 | H | F | H | CN | 1 | 144-145 |
| I-71 | -CH2CH2OCH3 | H | CF3 | H | F | H | CN | 0 | 76-78 |
| I-72 | -CH2CH2OCH3 | H | CF3 | H | F | H | CN | 1 | 62-63 |
| I-73 | -CH2CH2N(CH3)2 | H | CF3 | H | F | H | CN | 0 | 73-76 |
| I-74 | -CH2CH2N(CH3)2 | H | CF3 | H | F | H | CN | 1 | |
| I-75 | -CONHC2H5 | H | CF3 | H | F | H | CN | 0 | 1.5215 |
| I-76 | -CONHC2H5 | H | CF3 | H | F | H | CN | 1 | |
| I-77 | -CONHPh | H | CF3 | H | F | H | CN | 0 | |
| I-78 | -CONHPh | H | CF3 | H | F | H | CN | 1 | |
| I-79 | H | H | C2F5 | H | F | H | CN | 0 | |
| I-80 | H | H | C2F5 | H | F | H | CN | 1 | |
| I-81 | CH3 | H | C2F5 | H | F | H | CN | 0 | 1.5 |
| I-82 | CH3 | H | C2F5 | H | F | H | CN | 1 | 135-137 |
| I-83 | CH3 | CH3 | C2F5 | H | F | H | CN | 0 | |
| I-84 | CH3 | CH3 | C2F5 | H | F | H | CN | 1 | |
| I-85 | C2H5 | H | C2F5 | H | F | H | CN | 0 | |
| I-86 | C2H5 | H | C2F5 | H | F | H | CN | 1 | |
| I-87 | CH₂CF3 | H | C2F5 | H | F | H | CN | 0 | |
| I-88 | CH2CF3 | H | C2F5 | H | F | H | CN | 1 | |
| I-89 | n-Pr | H | C2F5 | H | F | H | CN | 0 | |
| I-90 | n-Pr | H | C2F5 | H | F | H | CN | 1 | |
| I-91 | i-Pr | H | C2F5 | H | F | H | CN | 0 | 1.4922 |
| I-92 | i-Pr | H | C2F5 | H | F | H | CN | 1 | 122-124 |
| I-93 | CH2CH3 | CH2CH3 | C2F5 | H | F | H | CN | 0 | 1.4998 |
| I-94 | CH2CH3 | CH2CH3 | C2F5 | H | F | H | CN | 1 | 97-100 |
| I-95 | -CH2CH2- | -CH2CH2- | C2F5 | H | F | H | CN | 0 | |
| I-96 | -CH2CH2- | -CH2CH2- | C2F5 | H | F | H | CN | 1 | |
| I-97 | cyc-Pr | H | C2F5 | H | F | H | CN | 0 | |
| I-98 | cyc-Pr | H | C2F5 | H | F | H | CN | 1 | |
| I-99 | -CH2CH=CH2 | H | C2F5 | H | F | H | CN | 0 | |
| I-100 | -CH2CH=CH2 | H | C2F5 | H | F | H | CN | 1 | |
| I-101 | -CH2C≡CH | H | C2F5 | H | F | H | CN | 0 | 1.5505 |
| I-102 | -CH2C≡CH | H | C2F5 | H | F | H | CN | 1 | 127-129 |
| I-103 | H | H | C3F7 | H | F | H | CN | 0 | |
| I-104 | H | H | C3F7 | H | F | H | CN | 1 | |
| I-105 | CH3 | H | C3F7 | H | F | H | CN | 0 | 83-84 |
| I-106 | CH3 | H | C3F7 | H | F | H | CN | 1 | 122-125 |
| I-107 | CH3 | CH3 | C3F7 | H | F | H | CN | 0 | |
| I-108 | CH3 | CH3 | C3F7 | H | F | H | CN | 1 | |
| I-109 | C2H5 | H | C3F7 | H | F | H | CN | 0 | |
| I-110 | C2H5 | H | C3F7 | H | F | H | CN | 1 | |
| I-111 | CH2CF3 | H | C3F7 | H | F | H | CN | 0 | |
| I-112 | CH2CF3 | H | C3F7 | H | F | H | CN | 1 | |
| I-113 | n-Pr | H | C3F7 | H | F | H | CN | 0 | |
| I-114 | n-Pr | H | C3F7 | H | F | H | CN | 1 | |
| I-115 | i-Pr | H | C3F7 | H | F | H | CN | 0 | 1.479 |
| I-116 | i-Pr | H | C3F7 | H | F | H | CN | 1 | 95-98 |
| I-117 | CH2CH3 | CH2CH3 | C3F7 | H | F | H | CN | 0 | 1.487 |
| I-118 | CH2CH3 | CH2CH3 | C3F7 | H | F | H | CN | 1 | 99-101 |
| I-119 | -CH2CH2- | -CH2CH2- | C3F7 | H | F | H | CN | 0 | |
| I-120 | -CH2CH2- | -CH2CH2- | C3F7 | H | F | H | CN | 1 | |
| I-121 | cyc-Pr | H | C3F7 | H | F | H | CN | 0 | |
| I-122 | cyc-Pr | H | C3F7 | H | F | H | CN | 1 | |
| I-123 | -CH2CH=CH2 | H | C3F7 | H | F | H | CN | 0 | |
| I-124 | -CH2CH=CH2 | H | C3F7 | H | F | H | CN | 1 | |
| I-125 | -CH2C≡CH | H | C3F7 | H | F | H | CN | 0 | |
| I-126 | -CH2C≡CH | H | C3F7 | H | F | H | CN | 1 | |
| I-127 | H | H | CN | H | F | H | CN | 0 | 107-111 |
| I-128 | H | H | CN | H | F | H | CN | 1 | |
| I-129 | CH3 | H | CN | H | F | H | CN | 0 | 1.5505 |
| I-130 | CH3 | H | CN | H | F | H | CN | 1 | 182-185 |
| I-131 | i-Pr | H | CN | H | F | H | CN | 0 | 1.5415 |
| I-132 | i-Pr | H | CN | H | F | H | CN | 1 | 167-168 |
| I-133 | C2H5 | C2H5 | CN | H | F | H | CN | 0 | 1.5538 |
| I-134 | C2H5 | C2H5 | CN | H | F | H | CN | 1 | 157-159 |
| I-135 | CH3 | CH3 | H | H | F | H | CN | 0 | 86-91 |
| I-136 | CH3 | CH3 | H | H | F | H | CN | 1 | |
| I-137 | C2H5 | C2H5 | H | H | F | H | CN | 0 | 1.5782 |
| I-138 | C2H5 | C2H5 | H | H | F | H | CN | 1 | |
| I-139 | -C2H2-O- | -C2H4- | H | H | F | H | CN | 0 | |
| I-140 | -C2H2-O- | -C2H4- | H | H | F | H | CN | 1 | |
| I-141 | -C2H4- | -C2H4- | H | H | F | H | CN | 0 | |
| I-142 | -C2H4- | -C2H4- | H | H | F | H | CN | 1 | |
| I-143 | -C2H4- | -C2H4- | H | H | F | H | CN | 0 | |
| I-144 | -C2H4- | -C2H4- | H | H | F | H | CN | 1 | |
| I-145 | CH3 | CH3 | CH3 | H | F | H | CN | 0 | 109-111 |
| I-146 | CH3 | CH3 | CH3 | H | F | H | CN | 1 | 131 -133 |
| I-147 | C2H5 | C2H5 | CH3 | H | F | H | CN | 0 | 1.559 |
| I-148 | C2H5 | C2H5 | CH3 | H | F | H | CN | 1 | 101-102 |
| I-149 | -C2H4-O- | -C2H4- | CH3 | H | F | H | CN | 0 | 102-103 |
| I-150 | -C2H4-O- | -C2H4- | CH3 | H | F | H | CN | 1 | 137-145 |
| I-151 | -C2H4- | -C2H4- | CH3 | H | F | H | CN | 0 | 1.5865 |
| I-152 | -C2H4- | -C2H4- | CH3 | H | F | H | CN | 1 | 94-96 |
| I-153 | -C2H4- | -C2H4- | CH3 | H | F | H | CN | 0 | 1.5792 |
| I-154 | -C2H4- | -C2H4- | CH3 | H | F | H | CN | 1 | 132-134 |
| I-155 | CH3 | CH3 | Ph | H | F | H | CN | 0 | 1.5798 |
| I-156 | CH3 | CH3 | Ph | H | F | H | CN | 1 | |
| I-157 | C2H5 | C2H5 | Ph | H | F | H | CN | 0 | |
| I-158 | C2H5 | C2H5 | Ph | H | F | H | CN | 1 | |
| 1-159 | -C2H4-O- | -C2H4- | Ph | H | F | H | CN | 0 | |
| I-160 | -C2H4-O- | -C2H4- | Ph | H | F | H | CN | 1 | |
| I-161 | -C2H4- | -C2H4- | Ph | H | F | H | CN | 0 | |
| I-162 | -C2H4- | -C2H4- | Ph | H | F | H | CN | 1 | |
| I-163 | -C2H4- | -C2H4- | Ph | H | F | H | CN | 0 | |
| I-164 | -C2H4- | -C2H4- | Ph | H | F | H | CN | 1 | |
| I-165 | -C2H4- | CH3 | -CH2- | H | F | H | CN | 0 | 94-100 |
| I-166 | -C2H4- | CH3 | -CH2- | H | F | H | CN | 1 | 131-133 |
| I-167 | -C2H4- | CH3 | -C2H4- | H | F | H | CN | 0 | 93-98 |
| I-168 | -C2H4- | CH3 | -C2H4- | H | F | H | CN | 1 | 145-147 |
| I-169 | -CO- | CH3 | -C2H4- | H | F | H | CN | 0 | 124-127 |
| I-170 | -CO- | CH3 | -C2H4- | H | F | H | CN | 1 | |
| I-171 | H | H | CF3 | H | Cl | H | CN | 0 | |
| I-172 | H | H | CF3 | H | Cl | H | CN | 1 | |
| I-173 | CH3 | H | CF3 | H | Cl | H | CN | 0 | |
| I-174 | CH3 | H | CF3 | H | Cl | H | CN | 1 | |
| I-175 | CH3 | CH3 | CF3 | H | Cl | H | CN | 0 | |
| I-176 | CH3 | CH3 | CF3 | H | Cl | H | CN | 1 | |
| I-177 | CH2CF3 | H | CF3 | H | Cl | H | CN | 0 | |
| I-178 | CH2CF3 | H | CF3 | H | Cl | H | CN | 1 | |
| I-179 | i-Pr | H | CF3 | H | Cl | H | CN | 0 | |
| I-180 | i-Pr | H | CF3 | H | Cl | H | CN | 1 | |
| I-181 | CH2CH3 | CH2CH3 | CF3 | H | Cl | H | CN | 0 | |
| I-182 | CH2CH3 | CH2CH3 | CF3 | H | Cl | H | CN | 1 | |
| I-183 | -CH2CH=CH2 | H | CF3 | H | Cl | H | CN | 0 | |
| I-184 | -CH2CH=CH2 | H | CF3 | H | Cl | H | CN | 1 | |
| I-185 | -CH2C≡CH | H | CF3 | H | Cl | H | CN | 0 | |
| I-186 | -CH2C≡CH | H | CF3 | H | Cl | H | CN | 1 | |
| I-187 | CH3 | CH3 | CN | H | Cl | H | CN | 0 | |
| I-188 | CH3 | CH3 | CN | H | Cl | H | CN | 1 | |
| I-189 | CH3 | CH3 | H | H | Cl | H | CN | 0 | |
| I-190 | CH3 | CH3 | H | H | Cl | H | CN | 1 | |
| I-191 | CH3 | CH3 | CH3 | H | Cl | H | CN | 0 | |
| I-192 | CH3 | CH3 | CH3 | H | Cl | H | CN | 1 | |
| I-193 | CH3 | CH3 | Ph | H | Cl | H | CN | 0 | |
| I-194 | CH3 | CH3 | Ph | H | Cl | H | CN | 1 | |
| I-195 | -C2H4- | CH3 | -C2H4- | H | Cl | H | CN | 0 | |
| I-196 | -C2H4- | CH3 | -C2H4- | H | Cl | H | CN | 1 | |
| I-197 | -CO- | CH3 | -C2H4- | H | Cl | H | CN | 0 | |
| I-198 | -CO- | CH3 | -C2H4- | H | Cl | H | CN | 1 | |
| I-199 | H | H | CF3 | H | Br | H | CN | 0 | |
| I-200 | H | H | CF3 | H | Br | H | CN | 1 | |
| I-201 | CH3 | H | CF3 | H | Br | H | CN | 0 | |
| I-202 | CH3 | H | CF3 | H | Br | H | CN | 1 | |
| I-203 | CH3 | CH3 | CF3 | H | Br | H | CN | 0 | |
| I-204 | CH3 | CH3 | CF3 | H | Br | H | CN | 1 | |
| I-205 | CH2CF3 | H | CF3 | H | Br | H | CN | 0 | |
| I-206 | CH2CF3 | H | CF3 | H | Br | H | CN | 1 | |
| I-207 | i-Pr | H | CF3 | H | Br | H | CN | 0 | |
| I-208 | i-Pr | H | CF3 | H | Br | H | CN | 1 | |
| I-209 | CH2CH3 | CH2CH3 | CF3 | H | Br | H | CN | 0 | |
| I-210 | CH2CH3 | CH2CH3 | CF3 | H | Br | H | CN | 1 | |
| I-211 | -CH2CH=CH2 | H | CF3 | H | Br | H | CN | 0 | |
| I-212 | -CH2CH=CH2 | H | CF3 | H | Br | H | CN | 1 | |
| I-213 | -CH2CΞCH | H | CF3 | H | Br | H | CN | 0 | |
| I-214 | -CH2C≡CH | H | CF3 | H | Br | H | CN | 1 | |
| I-215 | CH3 | CH3 | CN | H | Br | H | CN | 0 | |
| I-216 | CH3 | CH3 | CN | H | Br | H | CN | 1 | |
| I-217 | CH3 | CH3 | H | H | Br | H | CN | 0 | |
| I-218 | CH3 | CH3 | H | H | Br | H | CN | 1 | |
| I-219 | CH3 | CH3 | CH3 | H | Br | H | CN | 0 | |
| I-220 | CH3 | CH3 | CH3 | H | Br | H | CN | 1 | |
| I-221 | CH3 | CH3 | Ph | H | Br | H | CN | 0 | |
| I-222 | CH3 | CH3 | Ph | H | Br | H | CN | 1 | |
| I-223 | -C2H4- | CH3 | -C2H4- | H | Br | H | CN | 0 | |
| I-224 | -C2H4- | CH3 | -C2H4- | H | Br | H | CN | 1 | |
| I-225 | -CO- | CH3 | -C2H4- | H | Br | H | CN | 0 | |
| I-226 | -CO- | CH3 | -C2H4- | H | Br | H | CN | 1 | |
| I-227 | H | H | CF3 | H | I | H | CN | 0 | |
| I-228 | H | H | CF3 | H | I | H | CN | 1 | |
| I-229 | CH3 | H | CF3 | H | I | H | CN | 0 | |
| I-230 | CH3 | H | CF3 | H | I | H | CN | 1 | |
| I-231 | CH3 | CH3 | CF3 | H | I | H | CN | 0 | |
| I-232 | CH3 | CH3 | CF3 | H | I | H | CN | 1 | |
| I-233 | CH2CF3 | H | CF3 | H | I | H | CN | 0 | |
| I-234 | CH2CF3 | H | CF3 | H | I | H | CN | 1 | |
| I-235 | i-Pr | H | CF3 | H | I | H | CN | 0 | |
| I-236 | i-Pr | H | CF3 | H | I | H | CN | 1 | |
| I-237 | CH2CH3 | CH2CH3 | CF3 | H | I | H | CN | 0 | |
| I-238 | CH2CH3 | CH2CH3 | CF3 | H | I | H | CN | 1 | |
| I-239 | -CH2CH=CH2 | H | CF3 | H | I | H | CN | 0 | |
| I-240 | -CH2CH=CH2 | H | CF3 | H | I | H | CN | 1 | |
| I-241 | -CH2C≡CH | H | CF3 | H | I | H | CN | 0 | |
| I-242 | -CH2C≡CH | H | CF3 | H | I | H | CN | 1 | |
| I-243 | CH3 | CH3 | CN | H | I | H | CN | 0 | |
| I-244 | CH3 | CH3 | CN | H | I | H | CN | 1 | |
| I-245 | CH3 | CH3 | H | H | I | H | CN | 0 | |
| I-246 | CH3 | CH3 | H | H | I | H | CN | 1 | |
| I-247 | CH3 | CH3 | CH3 | H | I | H | CN | 0 | |
| I-248 | CH3 | CH3 | CH3 | H | I | H | CN | 1 | |
| I-249 | CH3 | CH3 | Ph | H | I | H | CN | 0 | |
| I-250 | CH3 | CH3 | Ph | H | I | H | CN | 1 | |
| I-251 | -C2H4- | CH3 | -C2H4- | H | I | H | CN | 0 | |
| I-252 | -C2H4- | CH3 | -C2H4- | H | I | H | CN | 1 | |
| I-253 | -CO- | CH3 | -C2H4- | H | I | H | CN | 0 | |
| I-254 | -CO- | CH3 | -C2H4- | H | I | H | CN | 1 | |
| I-255 | H | H | CF3 | CI | H | H | CN | 0 | |
| I-256 | H | H | CF3 | Cl | H | H | CN | 1 | |
| I-257 | CH3 | H | CF3 | Cl | H | H | CN | 0 | |
| I-258 | CH3 | H | CF3 | Cl | H | H | CN | 1 | |
| I-259 | CH3 | CH3 | CF3 | Cl | H | H | CN | 0 | |
| I-260 | CH3 | CH3 | CF3 | Cl | H | H | CN | 1 | |
| I-261 | CH2CF3 | H | CF3 | Cl | H | H | CN | 0 | |
| I-262 | CH2CF3 | H | CF3 | Cl | H | H | CN | 1 | |
| I-263 | i-Pr | H | CF3 | Cl | H | H | CN | 0 | |
| I-264 | i-Pr | H | CF3 | Cl | H | H | CN | 1 | |
| I-265 | CH2CH3 | CH2CH3 | CF3 | Cl | H | H | CN | 0 | |
| I-266 | CH2CH3 | CH2CH3 | CF3 | Cl | H | H | CN | 1 | |
| I-267 | -CH2CH=CH2 | H | CF3 | Cl | H | H | CN | 0 | |
| I-268 | -CH2CH=CH2 | H | CF3 | Cl | H | H | CN | 1 | |
| I-269 | -CH2C≡CH | H | CF3 | Cl | H | H | CN | 0 | |
| I-270 | -CH2C≡CH | H | CF3 | Cl | H | H | CN | 1 | |
| I-271 | CH3 | CH3 | CN | Cl | H | H | CN | 0 | |
| I-272 | CH3 | CH3 | CN | Cl | H | H | CN | 1 | |
| I-273 | CH3 | CH3 | H | Cl | H | H | CN | 0 | |
| I-274 | CH3 | CH3 | H | Cl | H | H | CN | 1 | |
| I-275 | CH3 | CH3 | CH3 | Cl | H | H | CN | 0 | |
| I-276 | CH3 | CH3 | CH3 | Cl | H | H | CN | 1 | |
| I-277 | CH3 | CH3 | Ph | Cl | H | H | CN | 0 | |
| I-278 | CH3 | CH3 | Ph | Cl | H | H | CN | 1 | |
| I-279 | -C2H4- | CH3 | -C2H4- | Cl | H | H | CN | 0 | |
| I-280 | -C2H4- | CH3 | -C2H4- | Cl | H | H | CN | 1 | |
| I-281 | -CO- | CH3 | -C2H4- | Cl | H | H | CN | 0 | |
| I-282 | -CO- | CH3 | -C2H4- | Cl | H | H | CN | 1 | |
| I-283 | H | H | CF3 | H | CN | H | H | 0 | |
| I-284 | H | H | CF3 | H | CN | H | H | 1 | |
| I-285 | CH3 | H | CF3 | H | CN | H | H | 0 | |
| I-286 | CH3 | H | CF3 | H | CN | H | H | 1 | |
| I-287 | CH3 | CH3 | CF3 | H | CN | H | H | 0 | |
| I-288 | CH3 | CH3 | CF3 | H | CN | H | H | 1 | |
| I-289 | CH2CF3 | H | CF3 | H | CN | H | H | 0 | |
| I-290 | CH2CF3 | H | CF3 | H | CN | H | H | 1 | |
| I-291 | i-Pr | H | CF3 | H | CN | H | H | 0 | 73-76 |
| I-292 | i-Pr | H | CF3 | H | CN | H | H | 1 | |
| I-293 | CH2CH3 | CH2CH3 | CF3 | H | CN | H | H | 0 | |
| I-294 | CH2CH3 | CH2CH3 | CF3 | H | CN | H | H | 1 | |
| I-295 | -CH2CH=CH2 | H | CF3 | H | CN | H | H | 0 | |
| I-296 | -CH2CH=CH2 | H | CF3 | H | CN | H | H | 1 | |
| I-297 | -CH2C≡CH | H | CF3 | H | CN | H | H | 0 | |
| I-298 | -CH2C≡CH | H | CF3 | H | CN | H | H | 1 | |
| I-299 | CH3 | CH3 | CN | H | CN | H | H | 0 | |
| I-300 | CH3 | CH3 | CN | H | CN | H | H | 1 | |
| I-301 | CH3 | CH3 | H | H | CN | H | H | 0 | |
| I-302 | CH3 | CH3 | H | H | CN | H | H | 1 | |
| I-303 | CH3 | CH3 | CH3 | H | CN | H | H | 0 | |
| I-304 | CH3 | CH3 | CH3 | H | CN | H | H | 1 | |
| I-305 | CH3 | CH3 | Ph | H | CN | H | H | 0 | |
| I-306 | CH3 | CH3 | Ph | H | CN | H | H | 1 | |
| I-307 | -C2H4- | CH₃ | -C2H4- | H | CN | H | H | 0 | |
| I-308 | -C2H4- | CH3 | -C2H4- | H | CN | H | H | 1 | |
| I-309 | -CO- | CH3 | -C2H4- | H | CN | H | H | 0 | |
| I-310 | -CO- | CH3 | -C2H4- | H | CN | H | H | 1 | |
| I-311 | H | H | CF3 | H | CN | H | Cl | 0 | |
| I-312 | H | H | CF3 | H | CN | H | Cl | 1 | |
| I-313 | CH3 | H | CF3 | H | CN | H | Cl | 0 | |
| I-314 | CH3 | H | CF3 | H | CN | H | Cl | 1 | |
| I-315 | CH3 | CH3 | CF3 | H | CN | H | Cl | 0 | |
| I-316 | CH3 | CH3 | CF3 | H | CN | H | Cl | 1 | |
| I-317 | CH2CF3 | H | CF3 | H | CN | H | Cl | 0 | |
| I-318 | CH2CF3 | H | CF3 | H | CN | H | Cl | 1 | |
| I-319 | i-Pr | H | CF3 | H | CN | H | Cl | 0 | |
| I-320 | i-Pr | H | CF3 | H | CN | H | Cl | 1 | |
| I-321 | CH2CH3 | CH2CH3 | CF3 | H | CN | H | Cl | 0 | |
| I-322 | CH2CH3 | CH2CH3 | CF3 | H | CN | H | Cl | 1 | |
| I-323 | -CH2CH=CH2 | H | CF3 | H | CN | H | Cl | 0 | |
| I-324 | -CH2CH=CH2 | H | CF3 | H | CN | H | Cl | 1 | |
| I-325 | -CH2C≡CH | H | CF3 | H | CN | H | Cl | 0 | |
| I-326 | -CH2C≡CH | H | CF3 | H | CN | H | Cl | 1 | |
| I-327 | CH3 | CH3 | CN | H | CN | H | Cl | 0 | |
| I-328 | CH3 | CH3 | CN | H | CN | H | Cl | 1 | |
| I-329 | CH3 | CH3 | H | H | CN | H | Cl | 0 | |
| I-330 | CH3 | CH3 | H | H | CN | H | Cl | 1 | |
| I-331 | CH3 | CH3 | CH3 | H | CN | H | Cl | 0 | |
| I-332 | CH3 | CH3 | CH3 | H | CN | H | Cl | 1 | |
| I-333 | CH3 | CH3 | Ph | H | CN | H | Cl | 0 | |
| I-334 | CH3 | CH3 | Ph | H | CN | H | Cl | 1 | |
| I-335 | -C2H4- | CH3 | -C2H4- | H | CN | H | Cl | 0 | |
| I-336 | -C2H4- | CH3 | -C2H4- | H | CN | H | Cl | 1 | |
| I-337 | -CO- | CH3 | -C2H4- | H | CN | H | Cl | 0 | |
| I-338 | -CO- | CH3 | -C2H4- | H | CN | H | Cl | 1 | |
| I-339 | H | H | CF3 | H | CN | H | Br | 0 | |
| I-340 | H | H | CF3 | H | CN | H | Br | 1 | |
| I-341 | CH3 | H | CF3 | H | CN | H | Br | 0 | |
| I-342 | CH3 | H | CF3 | H | CN | H | Br | 1 | |
| I-343 | CH3 | CH₃ | CF3 | H | CN | H | Br | 0 | |
| I-344 | CH3 | CH3 | CF3 | H | CN | H | Br | 1 | |
| I-345 | CH2CF3 | H | CF3 | H | CN | H | Br | 0 | |
| I-346 | CH2CF3 | H | CF3 | H | CN | H | Br | 1 | |
| I-347 | i-Pr | H | CF3 | H | CN | H | Br | 0 | |
| I-348 | i-Pr | H | CF3 | H | CN | H | Br | 1 | |
| I-349 | CH2CH3 | CH2CH3 | CF3 | H | CN | H | Br | 0 | |
| I-350 | CH2CH3 | CH2CH3 | CF3 | H | CN | H | Br | 1 | |
| I-351 | -CH2CH=CH2 | H | CF3 | H | CN | H | Br | 0 | |
| I-352 | -CH2CH=CH2 | H | CF3 | H | CN | H | Br | 1 | |
| I-353 | -CH2C≡CH | H | CF3 | H | CN | H | Br | 0 | |
| I-354 | -CH2C≡CH | H | CF3 | H | CN | H | Br | 1 | |
| I-355 | CH3 | CH3 | CN | H | CN | H | Br | 0 | |
| I-356 | CH3 | CH3 | CN | H | CN | H | Br | 1 | |
| I-357 | CH3 | CH3 | H | H | CN | H | Br | 0 | |
| I-358 | CH3 | CH3 | H | H | CN | H | Br | 1 | |
| I-359 | CH3 | CH3 | CH3 | H | CN | H | Br | 0 | |
| I-360 | CH3 | CH3 | CH3 | H | CN | H | Br | 1 | |
| I-361 | CH3 | CH3 | Ph | H | CN | H | Br | 0 | |
| I-362 | CH3 | CH3 | Ph | H | CN | H | Br | 1 | |
| I-363 | -C2H4- | CH3 | -C2H4- | H | CN | H | Br | 0 | |
| I-364 | -C2H4- | CH3 | -C2H4- | H | CN | H | Br | 1 | |
| I-365 | -CO- | CH3 | -C2H4- | H | CN | H | Br | 0 | |
| I-366 | -CO- | CH3 | -C2H4- | H | CN | H | Br | 1 | |
| I-367 | H | H | CF3 | H | CN | H | I | 0 | |
| I-368 | H | H | CF3 | H | CN | H | I | 1 | |
| I-369 | CH3 | H | CF3 | H | CN | H | I | 0 | |
| I-370 | CH3 | H | CF3 | H | CN | H | I | 1 | |
| I-371 | CH3 | CH3 | CF3 | H | CN | H | I | 0 | |
| I-372 | CH3 | CH3 | CF3 | H | CN | H | I | 1 | |
| I-373 | CH2CF3 | H | CF3 | H | CN | H | I | 0 | |
| I-374 | CH2CF3 | H | CF3 | H | CN | H | I | 1 | |
| I-375 | i-Pr | H | CF3 | H | CN | H | I | 0 | |
| I-376 | i-Pr | H | CF3 | H | CN | H | I | 1 | |
| I-377 | CH2CH3 | CH2CH3 | CF3 | H | CN | H | I | 0 | |
| I-378 | CH2CH3 | CH2CH3 | CF3 | H | CN | H | I | 1 | |
| I-379 | -CH2CH=CH2 | H | CF3 | H | CN | H | I | 0 | |
| I-380 | -CH2CH=CH2 | H | CF3 | H | CN | H | I | 1 | |
| I-381 | -CH2C≡CH | H | CF3 | H | CN | H | I | 0 | |
| I-382 | -CH2C≡CH | H | CF3 | H | CN | H | I | 1 | |
| I-383 | CH3 | CH3 | CN | H | CN | H | I | 0 | |
| I-384 | CH3 | CH3 | CN | H | CN | H | I | 1 | |
| I-385 | CH3 | CH3 | H | H | CN | H | I | 0 | |
| I-386 | CH3 | CH3 | H | H | CN | H | I | 1 | |
| I-387 | CH3 | CH3 | CH3 | H | CN | H | I | 0 | |
| I-388 | CH3 | CH3 | CH3 | H | CN | H | I | 1 | |
| I-389 | CH3 | CH3 | Ph | H | CN | H | I | 0 | |
| I-390 | CH3 | CH3 | Ph | H | CN | H | I | 1 | |
| I-391 | -C2H4- | CH3 | -C2H4- | H | CN | H | I | 0 | |
| I-392 | -C2H4- | CH3 | -C2H4- | H | CN | H | I | 1 | |
| I-393 | -CO- | CH3 | -C2H4- | H | CN | H | I | 0 | |
| I-394 | -CO- | CH3 | -C2H4- | H | CN | H | I | 1 | |
| I-395 | H | H | CF3 | Cl | CN | H | H | 0 | |
| I-396 | H | H | CF3 | Cl | CN | H | H | 1 | |
| I-397 | CH3 | H | CF3 | Cl | CN | H | H | 0 | |
| I-398 | CH3 | H | CF3 | Cl | CN | H | H | 1 | |
| I-399 | CH3 | CH3 | CF3 | Cl | CN | H | H | 0 | |
| I-400 | CH3 | CH3 | CF3 | Cl | CN | H | H | 1 | |
| I-401 | CH2CF3 | H | CF3 | Cl | CN | H | H | 0 | |
| I-402 | CH2CF3 | H | CF3 | Cl | CN | H | H | 1 | |
| I-403 | i-Pr | H | CF3 | Cl | CN | H | H | 0 | |
| I-404 | i-Pr | H | CF3 | Cl | CN | H | H | 1 | |
| I-405 | CH2CH3 | CH2CH3 | CF3 | Cl | CN | H | H | 0 | |
| I-406 | CH2CH3 | CH2CH3 | CF3 | Cl | CN | H | H | 1 | |
| I-407 | -CH2CH=CH2 | H | CF3 | Cl | CN | H | H | 0 | |
| I-408 | -CH2CH=CH2 | H | CF3 | Cl | CN | H | H | 1 | |
| I-409 | -CH2C≡CH | H | CF3 | Cl | CN | H | H | 0 | |
| I-410 | -CH2C≡CH | H | CF3 | Cl | CN | H | H | 1 | |
| I-411 | CH3 | CH3 | CN | Cl | CN | H | H | 0 | |
| I-412 | CH3 | CH3 | CN | Cl | CN | H | H | 1 | |
| I-413 | CH3 | CH3 | H | Cl | CN | H | H | 0 | |
| I-414 | CH3 | CH3 | H | Cl | CN | H | H | 1 | |
| I-415 | CH3 | CH3 | CH3 | Cl | CN | H | H | 0 | |
| I-416 | CH3 | CH3 | CH3 | Cl | CN | H | H | 1 | |
| I-417 | CH3 | CH3 | Ph | Cl | CN | H | H | 0 | |
| I-418 | CH3 | CH3 | Ph | Cl | CN | H | H | 1 | |
| I-419 | -C2H4- | CH3 | -C2H4- | Cl | CN | H | H | 0 | |
| I-420 | -C2H4- | CH3 | -C2H4- | Cl | CN | H | H | 1 | |
| I-421 | -CO- | CH3 | -C2H4- | Cl | CN | H | H | 0 | |
| I-422 | -CO- | CH₃ | -C2H4- | Cl | CN | H | H | 1 | |
| I-423 | H | H | CF3 | H | CN | H | CF3 | 0 | 1.4625 |
| I-424 | H | H | CF3 | H | CN | H | CF3 | 1 | 193-195 |
| I-425 | CH3 | H | CF3 | H | CN | H | CF3 | 0 | 1.4713 |
| I-426 | CH3 | H | CF3 | H | CN | H | CF3 | 1 | 81-82 |
| I-427 | CH3 | CH3 | CF3 | H | CN | H | CF3 | 0 | |
| I-428 | CH3 | CH3 | CF3 | H | CN | H | CF3 | 1 | |
| I-429 | CH2CF3 | H | CF3 | H | CN | H | CF3 | 0 | |
| I-430 | CH2CF3 | H | CF3 | H | CN | H | CF3 | 1 | |
| I-431 | i-Pr | H | CF3 | H | CN | H | CF3 | 0 | |
| I-432 | i-Pr | H | CF3 | H | CN | H | CF3 | 1 | |
| I-433 | CH2CH3 | CH2CH3 | CF3 | H | CN | H | CF3 | 0 | |
| I-434 | CH2CH3 | CH2CH3 | CF3 | H | CN | H | CF3 | 1 | |
| I-435 | -CH2CH=CH2 | H | CF3 | H | CN | H | CF3 | 0 | |
| I-436 | -CH2CH=CH2 | H | CF3 | H | CN | H | CF3 | 1 | |
| I-437 | -CH2C≡CH | H | CF3 | H | CN | H | CF3 | 0 | |
| I-438 | -CH2C≡CH | H | CF3 | H | CN | H | CF3 | 1 | |
| I-439 | CH3 | CH3 | CN | H | CN | H | CF3 | 0 | |
| I-440 | CH3 | CH3 | CN | H | CN | H | CF3 | 1 | |
| I-441 | CH3 | CH3 | H | H | CN | H | CF3 | 0 | |
| I-442 | CH3 | CH3 | H | H | CN | H | CF3 | 1 | |
| I-443 | CH3 | CH3 | CH3 | H | CN | H | CF3 | 0 | |
| I-444 | CH3 | CH3 | CH3 | H | CN | H | CF3 | 1 | |
| I-445 | CH3 | CH3 | Ph | H | CN | H | CF3 | 0 | |
| I-446 | CH3 | CH3 | Ph | H | CN | H | CF3 | 1 | |
| I-447 | -C2H4- | CH3 | -C2H4- | H | CN | H | CF3 | 0 | |
| I-448 | -C2H4- | CH3 | -C2H4- | H | CN | H | CF3 | 1 | |
| I-449 | -CO- | CH3 | -C2H4- | H | CN | H | CF3 | 0 | |
| I-450 | -CO- | CH3 | -C2H4- | H | CN | H | CF3 | 1 | |
| I-451 | H | H | CF3 | H | CN | H | CF3 | 0 | |
| I-452 | H | H | CF3 | H | CN | H | CF3 | 1 | |
| I-453 | CH3 | H | CF3 | H | CN | H | CF3 | 0 | |
| I-454 | CH3 | H | CF3 | H | CN | H | CF3 | 1 | |
| I-455 | CH3 | CH3 | CF3 | H | CN | H | CF3 | 0 | |
| I-456 | CH3 | CH3 | CF3 | H | CN | H | CF3 | 1 | |
| I-457 | CH2CF3 | H | CF3 | H | CN | H | CF3 | 0 | |
| I-458 | CH2CF3 | H | CF3 | H | CN | H | CF3 | 1 | |
| I-459 | i-Pr | H | CF3 | H | CN | H | CF3 | 0 | |
| I-460 | i-Pr | H | CF3 | H | CN | H | CF3 | 1 | |
| I-461 | CH2CH3 | CH2CH3 | CF3 | H | CN | H | CF3 | 0 | |
| I-462 | CH2CH3 | CH2CH3 | CF3 | H | CN | H | CF3 | 1 | |
| I-463 | -CH2CH=CH2 | H | CF3 | H | CN | H | CF3 | 0 | |
| I-464 | -CH2CH=CH2 | H | CF3 | H | CN | H | CF3 | 1 | |
| I-465 | -CH2C≡CH | H | CF3 | H | CN | H | CF3 | 0 | |
| I-466 | -CH2C≡CH | H | CF3 | H | CN | H | CF3 | 1 | |
| I-467 | CH3 | CH3 | CN | H | CN | H | CF3 | 0 | |
| I-468 | CH3 | CH3 | CN | H | CN | H | CF3 | 1 | |
| I-469 | CH3 | CH3 | H | H | CN | H | CF3 | 0 | |
| I-470 | CH3 | CH3 | H | H | CN | H | CF3 | 1 | |
| I-471 | CH3 | CH3 | CH3 | H | CN | H | CF3 | 0 | |
| I-472 | CH3 | CH3 | CH3 | H | CN | H | CF3 | 1 | |
| I-473 | CH3 | CH3 | Ph | H | CN | H | CF3 | 0 | |
| I-474 | CH3 | CH3 | Ph | H | CN | H | CF3 | 1 | |
| I-475 | -C2H4- | CH3 | -C2H4- | H | CN | H | CF3 | 0 | |
| I-476 | -C2H4- | CH3 | -C2H4- | H | CN | H | CF3 | 1 | |
| I-477 | -CO- | CH3 | -C2H4- | H | CN | H | CF3 | 0 | |
| I-478 | -CO- | CH3 | -C2H4- | H | CN | H | CF3 | 1 | |
| I-479 | H | H | CF3 | H | CN | H | CF3 | 0 | |
| I-480 | H | H | CF3 | H | CN | H | CF3 | 1 | |
| I-481 | CH3 | H | CF3 | H | CN | H | CF3 | 0 | |
| I-482 | CH3 | H | CF3 | H | CN | H | CF3 | 1 | |
| I-483 | CH3 | CH3 | CF3 | H | CN | H | CF3 | 0 | |
| I-484 | CH3 | CH3 | CF3 | H | CN | H | CF3 | 1 | |
| I-485 | CH2CF3 | H | CF3 | H | CN | H | CF3 | 0 | |
| I-486 | CH2CF3 | H | CF3 | H | CN | H | CF3 | 1 | |
| I-487 | i-Pr | H | CF3 | H | CN | H | CF3 | 0 | |
| I-488 | i-Pr | H | CF3 | H | CN | H | CF3 | 1 | |
| I-489 | CH2CH3 | CH2CH3 | CF3 | H | CN | H | CF3 | 0 | |
| I-490 | CH2CH3 | CH2CH3 | CF3 | H | CN | H | CF3 | 1 | |
| I-491 | -CH2CH=CH2 | H | CF3 | H | CN | H | CF3 | 0 | |
| I-492 | -CH2CH=CH2 | H | CF3 | H | CN | H | CF3 | 1 | |
| I-493 | -CH2C≡CH | H | CF3 | H | CN | H | CF3 | 0 | |
| I-494 | -CH2C≡CH | H | CF3 | H | CN | H | CF3 | 1 | |
| I-495 | CH3 | CH3 | CN | H | CN | H | CF3 | 0 | |
| I-496 | CH3 | CH3 | CN | H | CN | H | CF3 | 1 | |
| I-497 | CH3 | CH3 | H | H | CN | H | CF3 | 0 | |
| I-498 | CH3 | CH3 | H | H | CN | H | CF3 | 1 | |
| I-499 | CH3 | CH3 | CH3 | H | CN | H | CF3 | 0 | |
| I-500 | CH3 | CH3 | CH3 | H | CN | H | CF3 | 1 | |
| I-501 | CH3 | CH3 | Ph | H | CN | H | CF3 | 0 | |
| I-502 | CH3 | CH3 | Ph | H | CN | H | CF3 | 1 | |
| I-503 | -C2H4- | CH3 | -C2H4- | H | CN | H | CF3 | 0 | |
| I-504 | -C2H4- | CH3 | -C2H4- | H | CN | H | CF3 | 1 | |
| I-505 | -CO- | CH3 | -C2H4- | H | CN | H | CF3 | 0 | |
| I-506 | -CO- | CH3 | -C2H4- | H | CN | H | CF3 | 1 | |
| I-507 | H | H | CF3 | H | CN | H | CF3 | 0 | |
| I-508 | H | H | CF3 | H | CN | H | CF3 | 1 | |
| I-509 | CH3 | H | CF3 | H | CN | H | CF3 | 0 | |
| I-510 | CH3 | H | CF3 | H | CN | H | CF3 | 1 | |
| I-511 | CH3 | CH3 | CF3 | H | CN | H | CF3 | 0 | |
| I-512 | CH3 | CH3 | CF3 | H | CN | H | CF3 | 1 | |
| I-513 | CH2CF3 | H | CF3 | H | CN | H | CF3 | 0 | |
| I-514 | CH2CF3 | H | CF3 | H | CN | H | CF3 | 1 | |
| I-515 | i-Pr | H | CF3 | H | CN | H | CF3 | 0 | |
| I-516 | i-Pr | H | CF3 | H | CN | H | CF3 | 1 | |
| I-517 | CH2CH3 | CH2CH3 | CF3 | H | CN | H | CF3 | 0 | |
| I-518 | CH2CH3 | CH2CH3 | CF3 | H | CN | H | CF3 | 1 | |
| I-519 | -CH2CH=CH2 | H | CF3 | H | CN | H | CF3 | 0 | |
| I-520 | -CH2CH=CH2 | H | CF3 | H | CN | H | CF3 | 1 | |
| I-521 | -CH2C≡CH | H | CF3 | H | CN | H | CF3 | 0 | |
| I-522 | -CH2C≡CH | H | CF3 | H | CN | H | CF3 | 1 | |
| I-523 | CH3 | CH3 | CN | H | CN | H | CF3 | 0 | |
| I-524 | CH3 | CH3 | CN | H | CN | H | CF3 | 1 | |
| I-525 | CH3 | CH3 | H | H | CN | H | CF3 | 0 | |
| I-526 | CH3 | CH3 | H | H | CN | H | CF3 | 1 | |
| I-527 | CH3 | CH3 | CH3 | H | CN | H | CF3 | 0 | |
| I-528 | CH3 | CH3 | CH3 | H | CN | H | CF3 | 1 | |
| I-529 | CH3 | CH3 | Ph | H | CN | H | CF3 | 0 | |
| I-530 | CH3 | CH3 | Ph | H | CN | H | CF3 | 1 | |
| I-531 | -C2H4- | CH3 | -C2H4- | H | CN | H | CF3 | 0 | |
| I-532 | -C2H4- | CH3 | -C2H4- | H | CN | H | CF3 | 1 | |
| I-533 | -CO- | CH3 | -C2H4- | H | CN | H | CF3 | 0 | |
| I-534 | -CO- | CH3 | -C2H4- | H | CN | H | CF3 | 1 | |
| I-535 | H | H | CF3 | Cl | CN | H | CF3 | 0 | |
| I-536 | H | H | CF3 | Cl | CN | H | CF3 | 1 | |
| I-537 | CH3 | H | CF3 | Cl | CN | H | CF3 | 0 | |
| I-538 | CH3 | H | CF3 | Cl | CN | H | CF3 | 1 | |
| I-539 | CH3 | CH3 | CF3 | Cl | CN | H | CF3 | 0 | |
| I-540 | CH3 | CH3 | CF3 | Cl | CN | H | CF3 | 1 | |
| I-541 | CH2CF3 | H | CF3 | Cl | CN | H | CF3 | 0 | |
| I-542 | CH2CF3 | H | CF3 | Cl | CN | H | CF3 | 1 | |
| I-543 | i-Pr | H | CF3 | Cl | CN | H | CF3 | 0 | |
| I-544 | i-Pr | H | CF3 | Cl | CN | H | CF3 | 1 | |
| I-545 | CH2CH3 | CH2CH3 | CF3 | Cl | CN | H | CF3 | 0 | |
| I-546 | CH2CH3 | CH2CH3 | CF3 | Cl | CN | H | CF3 | 1 | |
| I-547 | -CH2CH=CH2 | H | CF3 | Cl | CN | H | CF3 | 0 | |
| I-548 | -CH2CH=CH2 | H | CF3 | Cl | CN | H | CF3 | 1 | |
| I-549 | -CH2C≡CH | H | CF3 | Cl | CN | H | CF3 | 0 | |
| I-550 | -CH2C≡CH | H | CF3 | Cl | CN | H | CF3 | 1 | |
| I-551 | CH3 | CH3 | CN | Cl | CN | H | CF3 | 0 | |
| I-552 | CH3 | CH3 | CN | Cl | CN | H | CF3 | 1 | |
| I-553 | CH3 | CH3 | H | Cl | CN | H | CF3 | 0 | |
| I-554 | CH3 | CH3 | H | Cl | CN | H | CF3 | 1 | |
| I-555 | CH3 | CH3 | CH3 | Cl | CN | H | CF3 | 0 | |
| I-556 | CH3 | CH3 | CH3 | Cl | CN | H | CF3 | 1 | |
| I-557 | CH3 | CH3 | Ph | Cl | CN | H | CF3 | 0 | |
| I-558 | CH3 | CH3 | Ph | Cl | CN | H | CF3 | 1 | |
| I-559 | -C2H4- | CH3 | -C2H4- | Cl | CN | H | CF3 | 0 | |
| I-560 | -C2H4- | CH3 | -C2H4- | Cl | CN | H | CF3 | 1 | |
| I-561 | -CO- | CH3 | -C2H4- | Cl | CN | H | CF3 | 0 | |
| I-562 | -CO- | CH3 | -C2H4- | Cl | CN | H | CF3 | 1 | |
| I-563 | H | H | CF3 | H | CN | H | H | 0 | |
| I-564 | H | H | CF3 | H | CN | H | H | 1 | |
| I-565 | CH3 | H | CF3 | H | CN | H | H | 0 | |
| I-566 | CH3 | H | CF3 | H | CN | H | H | 1 | |
| I-567 | CH3 | CH3 | CF3 | H | CN | H | H | 0 | |
| I-568 | CH3 | CH3 | CF3 | H | CN | H | H | 1 | |
| I-569 | CH2CF3 | H | CF3 | H | CN | H | H | 0 | |
| I-570 | CH2CF3 | H | CF3 | H | CN | H | H | 1 | |
| I-571 | i-Pr | H | CF3 | H | CN | H | H | 0 | |
| I-572 | i-Pr | H | CF3 | H | CN | H | H | 1 | |
| I-573 | CH2CH3 | CH2CH3 | CF3 | H | CN | H | H | 0 | |
| I-574 | CH2CH3 | CH2CH3 | CF3 | H | CN | H | H | 1 | |
| I-575 | -CH2CH=CH2 | H | CF3 | H | CN | H | H | 0 | |
| I-576 | -CH2CH=CH2 | H | CF3 | H | CN | H | H | 1 | |
| I-577 | -CH2C≡CH | H | CF3 | H | CN | H | H | 0 | |
| I-578 | -CH2C≡CH | H | CF3 | H | CN | H | H | 1 | |
| I-579 | CH3 | CH3 | CN | H | CN | H | H | 0 | |
| I-580 | CH3 | CH3 | CN | H | CN | H | H | 1 | |
| I-581 | CH3 | CH3 | H | H | CN | H | H | 0 | |
| I-582 | CH3 | CH3 | H | H | CN | H | H | 1 | |
| I-583 | CH3 | CH3 | CH3 | H | CN | H | H | 0 | |
| I-584 | CH3 | CH3 | CH3 | H | CN | H | H | 1 | |
| I-585 | CH3 | CH3 | Ph | H | CN | H | H | 0 | |
| I-586 | CH3 | CH3 | Ph | H | CN | H | H | 1 | |
| I-587 | -C2H4- | CH3 | -C2H4- | H | CN | H | H | 0 | |
| I-588 | -C2H4- | CH3 | -C2H4- | H | CN | H | H | 1 | |
| I-589 | -CO- | CH3 | -C2H4- | H | CN | H | H | 0 | |
| I-590 | -CO- | CH3 | -C2H4- | H | CN | H | H | 1 | |
| I-591 | H | H | CF3 | H | CN | H | Cl | 0 | |
| I-592 | H | H | CF3 | H | CN | H | Cl | 1 | |
| I-593 | CH3 | H | CF3 | H | CN | H | Cl | 0 | |
| I-594 | CH3 | H | CF3 | H | CN | H | Cl | 1 | |
| I-595 | CH3 | CH3 | CF3 | H | CN | H | Cl | 0 | |
| I-596 | CH3 | CH3 | CF3 | H | CN | H | Cl | 1 | |
| I-597 | CH2CF3 | H | CF3 | H | CN | H | Cl | 0 | |
| I-598 | CH2CF3 | H | CF3 | H | CN | H | Cl | 1 | |
| I-599 | i-Pr | H | CF3 | H | CN | H | Cl | 0 | |
| I-600 | i-Pr | H | CF3 | H | CN | H | Cl | 1 | |
| I-601 | CH2CH3 | CH2CH3 | CF3 | H | CN | H | Cl | 0 | |
| I-602 | CH2CH3 | CH2CH3 | CF3 | H | CN | H | Cl | 1 | |
| I-603 | -CH2CH=CH2 | H | CF3 | H | CN | H | Cl | 0 | |
| I-604 | -CH2CH=CH2 | H | CF3 | H | CN | H | Cl | 1 | |
| I-605 | -CH2C≡CH | H | CF3 | H | CN | H | Cl | 0 | |
| I-606 | -CH2C≡CH | H | CF3 | H | CN | H | Cl | 1 | |
| I-607 | CH3 | CH3 | CN | H | CN | H | Cl | 0 | |
| I-608 | CH3 | CH3 | CN | H | CN | H | Cl | 1 | |
| I-609 | CH3 | CH3 | H | H | CN | H | Cl | 0 | |
| I-610 | CH3 | CH3 | H | H | CN | H | Cl | 1 | |
| I-611 | CH3 | CH3 | CH3 | H | CN | H | Cl | 0 | |
| I-612 | CH3 | CH3 | CH3 | H | CN | H | Cl | 1 | |
| I-613 | CH3 | CH3 | Ph | H | CN | H | Cl | 0 | |
| I-614 | CH3 | CH3 | Ph | H | CN | H | Cl | 1 | |
| I-615 | -C2H4- | CH3 | -C2H4- | H | CN | H | Cl | 0 | |
| I-616 | -C2H4- | CH3 | -C2H4- | H | CN | H | Cl | 1 | |
| I-617 | -CO- | CH3 | -C2H4- | H | CN | H | Cl | 0 | |
| I-618 | -CO- | CH3 | -C2H4- | H | CN | H | Cl | 1 | |
| I-619 | H | H | CF3 | H | CN | H | Br | 0 | |
| I-620 | H | H | CF3 | H | CN | H | Br | 1 | |
| I-621 | CH3 | H | CF3 | H | CN | H | Br | 0 | |
| I-622 | CH3 | H | CF3 | H | CN | H | Br | 1 | |
| I-623 | CH3 | CH3 | CF3 | H | CN | H | Br | 0 | |
| I-624 | CH3 | CH3 | CF3 | H | CN | H | Br | 1 | |
| I-625 | CH2CF3 | H | CF3 | H | CN | H | Br | 0 | |
| I-626 | CH2CF3 | H | CF3 | H | CN | H | Br | 1 | |
| I-627 | i-Pr | H | CF3 | H | CN | H | Br | 0 | |
| I-628 | i-Pr | H | CF3 | H | CN | H | Br | 1 | |
| I29 | CH2CH3 | CH2CH3 | CF3 | H | CN | H | Br | 0 | |
| I-630 | CH2CH3 | CH2CH3 | CF3 | H | CN | H | Br | 1 | |
| I-631 | -CH2CH=CH2 | H | CF3 | H | CN | H | Br | 0 | |
| I-632 | -CH2CH=CH2 | H | CF3 | H | CN | H | Br | 1 | |
| I-633 | -CH2C≡CH | H | CF3 | H | CN | H | Br | 0 | |
| I-634 | -CH2C≡CH | H | CF3 | H | CN | H | Br | 1 | |
| I-635 | CH3 | CH3 | CN | H | CN | H | Br | 0 | |
| I-636 | CH3 | CH3 | CN | H | CN | H | Br | 1 | |
| I-637 | CH3 | CH3 | H | H | CN | H | Br | 0 | |
| I-638 | CH3 | CH3 | H | H | CN | H | Br | 1 | |
| I-639 | CH3 | CH3 | CH3 | H | CN | H | Br | 0 | |
| I-640 | CH3 | CH3 | CH3 | H | CN | H | Br | 1 | |
| I-641 | CH3 | CH3 | Ph | H | CN | H | Br | 0 | |
| I-642 | CH3 | CH3 | Ph | H | CN | H | Br | 1 | |
| I-643 | -C2H4- | CH3 | -C2H4- | H | CN | H | Br | 0 | |
| I-644 | -C2H4- | CH3 | -C2H4- | H | CN | H | Br | 1 | |
| I-645 | -CO- | CH3 | -C2H4- | H | CN | H | Br | 0 | |
| I-646 | -CO- | CH3 | -C2H4- | H | CN | H | Br | 1 | |
| I-647 | H | H | CF3 | H | CN | H | I | 0 | |
| I-648 | H | H | CF3 | H | CN | H | I | 1 | |
| I-649 | CH3 | H | CF3 | H | CN | H | I | 0 | |
| I-650 | CH3 | H | CF3 | H | CN | H | I | 1 | |
| I-651 | CH3 | CH3 | CF3 | H | CN | H | I | 0 | |
| I-652 | CH3 | CH3 | CF3 | H | CN | H | I | 1 | |
| I-653 | CH2CF3 | H | CF3 | H | CN | H | I | 0 | |
| I-654 | CH2CF3 | H | CF3 | H | CN | H | I | 1 | |
| I-655 | i-Pr | H | CF3 | H | CN | H | I | 0 | |
| I-656 | i-Pr | H | CF3 | H | CN | H | I | 1 | |
| I-657 | CH2CH3 | CH2CH3 | CF3 | H | CN | H | I | 0 | |
| I-658 | CH2CH3 | CH2CH3 | CF3 | H | CN | H | I | 1 | |
| I-659 | -CH2CH=CH2 | H | CF3 | H | CN | H | I | 0 | |
| I-660 | -CH2CH=CH2 | H | CF3 | H | CN | H | I | 1 | |
| I-661 | -CH2C≡CH | H | CF3 | H | CN | H | I | 0 | |
| I-662 | -CH2C≡CH | H | CF3 | H | CN | H | I | 1 | |
| I-663 | CH3 | CH3 | CN | H | CN | H | I | 0 | |
| I-664 | CH3 | CH3 | CN | H | CN | H | I | 1 | |
| I-665 | CH3 | CH3 | H | H | CN | H | I | 0 | |
| I-666 | CH3 | CH3 | H | H | CN | H | I | 1 | |
| I-667 | CH3 | CH3 | CH3 | H | CN | H | I | 0 | |
| I-668 | CH3 | CH3 | CH3 | H | CN | H | I | 1 | |
| I-669 | CH3 | CH3 | Ph | H | CN | H | I | 0 | |
| I-670 | CH3 | CH3 | Ph | H | CN | H | I | 1 | |
| I-671 | -C2H4- | CH3 | -C2H4- | H | CN | H | I | 0 | |
| I-672 | -C2H4- | CH3 | -C2H4- | H | CN | H | I | 1 | |
| I-673 | -CO- | CH3 | -C2H4- | H | CN | H | I | 0 | |
| I-674 | -CO- | CH3 | -C2H4- | H | CN | H | I | 1 | |
| I-675 | H | H | CF3 | Cl | CN | H | H | 0 | |
| I-676 | H | H | CF3 | Cl | CN | H | H | 1 | |
| I-677 | CH3 | H | CF3 | Cl | CN | H | H | 0 | |
| I-678 | CH3 | H | CF3 | Cl | CN | H | H | 1 | |
| I-679 | CH3 | CH3 | CF3 | Cl | CN | H | H | 0 | |
| I-680 | CH3 | CH3 | CF3 | Cl | CN | H | H | 1 | |
| I-681 | CH2CF3 | H | CF3 | Cl | CN | H | H | 0 | |
| I-682 | CH2CF3 | H | CF3 | Cl | CN | H | H | 1 | |
| I-683 | i-Pr | H | CF3 | Cl | CN | H | H | 0 | |
| I-684 | i-Pr | H | CF3 | Cl | CN | H | H | 1 | |
| I-685 | CH2CH3 | CH2CH3 | CF3 | Cl | CN | H | H | 0 | |
| I-686 | CH2CH3 | CH2CH3 | CF3 | Cl | CN | H | H | 1 | |
| I-687 | -CH2CH=CH2 | H | CF3 | Cl | CN | H | H | 0 | |
| I-688 | -CH2CH=CH2 | H | CF3 | Cl | CN | H | H | 1 | |
| I-689 | -CH2C≡CH | H | CF3 | Cl | CN | H | H | 0 | |
| I-690 | -CH2C≡CH | H | CF3 | Cl | CN | H | H | 1 | |
| I-691 | CH3 | CH3 | CN | Cl | CN | H | H | 0 | |
| I-692 | CH3 | CH3 | CN | Cl | CN | H | H | 1 | |
| I-693 | CH3 | CH3 | H | Cl | CN | H | H | 0 | |
| I-694 | CH3 | CH3 | H | Cl | CN | H | H | 1 | |
| I-695 | CH3 | CH3 | CH3 | Cl | CN | H | H | 0 | |
| I-696 | CH3 | CH3 | CH3 | Cl | CN | H | H | 1 | |
| I-697 | CH3 | CH3 | Ph | Cl | CN | H | H | 0 | |
| I-698 | CH3 | CH3 | Ph | Cl | CN | H | H | 1 | |
| I-699 | -C2H4- | CH3 | -C2H4- | Cl | CN | H | H | 0 | |
| I-700 | -C2H4- | CH3 | -C2H4- | Cl | CN | H | H | 1 | |
| I-701 | -CO- | CH3 | -C2H4- | Cl | CN | H | H | 0 | |
| I-702 | -CO- | CH3 | -C2H4- | Cl | CN | H | H | 1 | |
| I-703 | H | H | CF3 | H | CN | H | CHF2 | 0 | |
| I-704 | H | H | CF3 | H | CN | H | CHF2 | 1 | |
| I-705 | CH3 | H | CF3 | H | CN | H | CHF2 | 0 | |
| I-706 | CH3 | H | CF3 | H | CN | H | CHF2 | 1 | |
| I-707 | CH3 | CH3 | CF3 | H | CN | H | CHF2 | 0 | |
| I-708 | CH3 | CH3 | CF3 | H | CN | H | CHF2 | 1 | |
| I-709 | CH2CF3 | H | CF3 | H | CN | H | CHF2 | 0 | |
| I-710 | CH2CF3 | H | CF3 | H | CN | H | CHF2 | 1 | |
| I-711 | i-Pr | H | CF3 | H | CN | H | CHF2 | 0 | |
| I-712 | i-Pr | H | CF3 | H | CN | H | CHF2 | 1 | |
| I-713 | CH2CH3 | CH2CH3 | CF3 | H | CN | H | CHF2 | 0 | |
| I-714 | CH2CH3 | CH2CH3 | CF3 | H | CN | H | CHF2 | 1 | |
| I-715 | -CH2CH=CH2 | H | CF3 | H | CN | H | CHF2 | 0 | |
| I-716 | -CH2CH=CH2 | H | CF3 | H | CN | H | CHF2 | 1 | |
| I-717 | -CH2C≡CH | H | CF3 | H | CN | H | CHF2 | 0 | |
| I-718 | -CH2C≡CH | H | CF3 | H | CN | H | CHF2 | 1 | |
| I-719 | CH3 | CH3 | H | H | CN | H | CHF2 | 0 | |
| I-720 | CH3 | CH3 | H | H | CN | H | CHF2 | 1 | |
| I-721 | CH3 | CH3 | CH3 | H | CN | H | CHF2 | 0 | |
| I-722 | CH3 | CH3 | CH3 | H | CN | H | CHF2 | 1 | |
| I-723 | CH3 | CH3 | Ph | H | CN | H | CHF2 | 0 | |
| I-724 | CH3 | CH3 | Ph | H | CN | H | CHF2 | 1 | |
| I-725 | -C2H4- | CH3 | -C2H4- | H | CN | H | CHF2 | 0 | |
| I-726 | -C2H4- | CH3 | -C2H4- | H | CN | H | CHF2 | 1 | |
| I-727 | -CO- | CH3 | -C2H4- | H | CN | H | CHF2 | 0 | |
| I-728 | -CO- | CH3 | -C2H4- | H | CN | H | CHF2 | 1 | |
| I-729 | H | H | CF3 | H | CN | H | CHF2 | 0 | |
| I-730 | H | H | CF3 | H | CN | H | CHF2 | 1 | |
| I-731 | CH3 | H | CF3 | H | CN | H | CHF2 | 0 | |
| I-732 | CH3 | H | CF3 | H | CN | H | CHF2 | 1 | |
| I-733 | CH3 | CH3 | CF3 | H | CN | H | CHF2 | 0 | |
| I-734 | CH3 | CH3 | CF3 | H | CN | H | CHF2 | 1 | |
| I-735 | CH2CF3 | H | CF3 | H | CN | H | CHF2 | 0 | |
| I-736 | CH2CF3 | H | CF3 | H | CN | H | CHF2 | 1 | |
| I-737 | i-Pr | H | CF3 | H | CN | H | CHF2 | 0 | |
| I-738 | i-Pr | H | CF3 | H | CN | H | CHF2 | 1 | |
| I-739 | CH2CH3 | CH2CH3 | CF3 | H | CN | H | CHF2 | 0 | |
| I-740 | CH2CH3 | CH2CH3 | CF3 | H | CN | H | CHF2 | 1 | |
| I-741 | -CH2CH=CH2 | H | CF3 | H | CN | H | CHF2 | 0 | |
| I-742 | -CH2CH=CH2 | H | CF3 | H | CN | H | CHF2 | 1 | |
| I-743 | -CH2C≡CH | H | CF3 | H | CN | H | CHF2 | 0 | |
| I-744 | -CH2C≡CH | H | CF3 | H | CN | H | CHF2 | 1 | |
| I-745 | CH3 | CH3 | H | H | CN | H | CHF2 | 0 | |
| I-746 | CH3 | CH3 | H | H | CN | H | CHF2 | 1 | |
| I-747 | CH3 | CH3 | CH3 | H | CN | H | CHF2 | 0 | |
| I-748 | CH3 | CH3 | CH3 | H | CN | H | CHF2 | 1 | |
| I-749 | CH3 | CH3 | Ph | H | CN | H | CHF2 | 0 | |
| I-750 | CH3 | CH3 | Ph | H | CN | H | CHF2 | 1 | |
| I-751 | -C2H4- | CH3 | -C2H4- | H | CN | H | CHF2 | 0 | |
| I-752 | -C2H4- | CH3 | -C2H4- | H | CN | H | CHF2 | 1 | |
| I-753 | -CO- | CH3 | -C2H4- | H | CN | H | CHF2 | 0 | |
| I-754 | -CO- | CH3 | -C2H4- | H | CN | H | CHF2 | 1 | |
| I-755 | H | H | CF3 | H | CN | H | CHF2 | 0 | |
| I-756 | H | H | CF3 | H | CN | H | CHF2 | 1 | |
| I-757 | CH3 | H | CF3 | H | CN | H | CHF2 | 0 | |
| I-758 | CH3 | H | CF3 | H | CN | H | CHF2 | 1 | |
| I-759 | CH3 | CH3 | CF3 | H | CN | H | CHF2 | 0 | |
| I-760 | CH3 | CH3 | CF3 | H | CN | H | CHF2 | 1 | |
| I-761 | CH2CF3 | H | CF3 | H | CN | H | CHF2 | 0 | |
| I-762 | CH2CF3 | H | CF3 | H | CN | H | CHF2 | 1 | |
| I-763 | i-Pr | H | CF3 | H | CN | H | CHF2 | 0 | |
| I-764 | i-Pr | H | CF3 | H | CN | H | CHF2 | 1 | |
| I-765 | CH2CH3 | CH2CH3 | CF3 | H | CN | H | CHF2 | 0 | |
| I-766 | CH2CH3 | CH2CH3 | CF3 | H | CN | H | CHF2 | 1 | |
| I-767 | -CH2CH=CH2 | H | CF3 | H | CN | H | CHF2 | 0 | |
| I-768 | -CH2CH=CH2 | H | CF3 | H | CN | H | CHF2 | 1 | |
| I-769 | -CH2C≡CH | H | CF3 | H | CN | H | CHF2 | 0 | |
| I-770 | -CH2C≡CH | H | CF3 | H | CN | H | CHF2 | 1 | |
| I-771 | CH3 | CH3 | H | H | CN | H | CHF2 | 0 | |
| I-772 | CH3 | CH3 | H | H | CN | H | CHF2 | 1 | |
| I-773 | CH3 | CH3 | CH3 | H | CN | H | CHF2 | 0 | |
| I-774 | CH3 | CH3 | CH3 | H | CN | H | CHF2 | 1 | |
| I-775 | CH3 | CH3 | Ph | H | CN | H | CHF2 | 0 | |
| I-776 | CH3 | CH3 | Ph | H | CN | H | CHF2 | 1 | |
| I-777 | -C2H4- | CH3 | -C2H4- | H | CN | H | CHF2 | 0 | |
| I-778 | -C2H4- | CH3 | -C2H4- | H | CN | H | CHF2 | 1 | |
| I-779 | -CO- | CH3 | -C2H4- | H | CN | H | CHF2 | 0 | |
| I-780 | -CO- | CH3 | -C2H4- | H | CN | H | CHF2 | 1 | |
| I-781 | H | H | CF3 | H | CN | H | CHF2 | 0 | |
| I-782 | H | H | CF3 | H | CN | H | CHF2 | 1 | |
| I-783 | CH3 | H | CF3 | H | CN | H | CHF2 | 0 | |
| I-784 | CH3 | H | CF3 | H | CN | H | CHF2 | 1 | |
| I-785 | CH3 | CH3 | CF3 | H | CN | H | CHF2 | 0 | |
| I-786 | CH3 | CH3 | CF3 | H | CN | H | CHF2 | 1 | |
| I-787 | CH2CF3 | H | CF3 | H | CN | H | CHF2 | 0 | |
| I-788 | CH2CF3 | H | CF3 | H | CN | H | CHF2 | 1 | |
| I-789 | i-Pr | H | CF3 | H | CN | H | CHF2 | 0 | |
| I-790 | i-Pr | H | CF3 | H | CN | H | CHF2 | 1 | |
| I-791 | CH2CH3 | CH2CH3 | CF3 | H | CN | H | CHF2 | 0 | |
| I-792 | CH2CH3 | CH2CH3 | CF3 | H | CN | H | CHF2 | 1 | |
| I-793 | -CH2CH=CH2 | H | CF3 | H | CN | H | CHF2 | 0 | |
| I-794 | -CH2CH=CH2 | H | CF3 | H | CN | H | CHF2 | 1 | |
| I-795 | -CH2C≡CH | H | CF3 | H | CN | H | CHF2 | 0 | |
| I-796 | -CH2C≡CH | H | CF3 | H | CN | H | CHF2 | 1 | |
| I-797 | CH3 | CH3 | H | H | CN | H | CHF2 | 0 | |
| I-798 | CH3 | CH3 | H | H | CN | H | CHF2 | 1 | |
| I-799 | CH3 | CH3 | CH3 | H | CN | H | CHF2 | 0 | |
| I-800 | CH3 | CH3 | CH3 | H | CN | H | CHF2 | 1 | |
| I-801 | CH3 | CH3 | Ph | H | CN | H | CHF2 | 0 | |
| I-802 | CH3 | CH3 | Ph | H | CN | H | CHF2 | 1 | |
| I-803 | -C2H4- | CH3 | -C2H4- | H | CN | H | CHF2 | 0 | |
| I-804 | -C2H4- | CH3 | -C2H4- | H | CN | H | CHF2 | 1 | |
| I-805 | -CO- | CH3 | -C2H4- | H | CN | H | CHF2 | 0 | |
| I-806 | -CO- | CH3 | -C2H4- | H | CN | H | CHF2 | 1 | |
| I-807 | H | H | CF3 | Cl | CN | H | CHF2 | 0 | |
| I-808 | H | H | CF3 | Cl | CN | H | CHF2 | 1 | |
| I-809 | CH3 | H | CF3 | Cl | CN | H | CHF2 | 0 | |
| I-810 | CH3 | H | CF3 | Cl | CN | H | CHF2 | 1 | |
| I-811 | CH3 | CH3 | CF3 | Cl | CN | H | CHF2 | 0 | |
| I-812 | CH3 | CH3 | CF3 | Cl | CN | H | CHF2 | 1 | |
| I-813 | CH2CF3 | H | CF3 | Cl | CN | H | CHF2 | 0 | |
| I-814 | CH2CF3 | H | CF3 | Cl | CN | H | CHF2 | 1 | |
| I-815 | i-Pr | H | CF3 | Cl | CN | H | CHF2 | 0 | |
| I-816 | i-Pr | H | CF3 | Cl | CN | H | CHF2 | 1 | |
| I-817 | CH2CH3 | CH2CH3 | CF3 | Cl | CN | H | CHF2 | 0 | |
| I-818 | CH2CH3 | CH2CH3 | CF3 | Cl | CN | H | CHF2 | 1 | |
| I-819 | -CH2CH=CH2 | H | CF3 | Cl | CN | H | CHF2 | 0 | |
| I-820 | -CH2CH=CH2 | H | CF3 | Cl | CN | H | CHF2 | 1 | |
| I-821 | -CH2C≡CH | H | CF3 | Cl | CN | H | CHF2 | 0 | |
| I-822 | -CH2C≡CH | H | CF3 | Cl | CN | H | CHF2 | 1 | |
| I-823 | CH3 | CH3 | H | Cl | CN | H | CHF2 | 0 | |
| I-824 | CH3 | CH3 | H | Cl | CN | H | CHF2 | 1 | |
| I-825 | CH3 | CH3 | CH3 | Cl | CN | H | CHF2 | 0 | |
| I-826 | CH3 | CH3 | CH3 | Cl | CN | H | CHF2 | 1 | |
| I-827 | CH3 | CH3 | Ph | Cl | CN | H | CHF2 | 0 | |
| I-828 | CH3 | CH3 | Ph | Cl | CN | H | CHF2 | 1 | |
| I-829 | -C2H4- | CH3 | -C2H4- | Cl | CN | H | CHF2 | 0 | |
| I-830 | -C2H4- | CH3 | -C2H4- | Cl | CN | H | CHF2 | 1 | |
| I-831 | -CO- | CH3 | -C2H4- | Cl | CN | H | CHF2 | 0 | |
| I-832 | -CO- | CH3 | -C2H4- | Cl | CN | H | CHF2 | 1 | |
| I-833 | H | H | CF3 | H | CN | H | H | 0 | |
| I-834 | H | H | CF3 | H | CN | H | H | 1 | |
| I-835 | CH3 | H | CF3 | H | CN | H | H | 0 | |
| I-836 | CH3 | H | CF3 | H | CN | H | H | 1 | |
| I-837 | CH3 | CH3 | CF3 | H | CN | H | H | 0 | |
| I-838 | CH3 | CH3 | CF3 | H | CN | H | H | 1 | |
| I-839 | CH2CF3 | H | CF3 | H | CN | H | H | 0 | |
| I-840 | CH2CF3 | H | CF3 | H | CN | H | H | 1 | |
| I-841 | i-Pr | H | CF3 | H | CN | H | H | 0 | |
| I-842 | i-Pr | H | CF3 | H | CN | H | H | 1 | |
| I-843 | CH2CH3 | CH2CH3 | CF3 | H | CN | H | H | 0 | |
| I-844 | CH2CH3 | CH2CH3 | CF3 | H | CN | H | H | 1 | |
| I-845 | -CH2CH=CH2 | H | CF3 | H | CN | H | H | 0 | |
| I-846 | -CH2CH=CH2 | H | CF3 | H | CN | H | H | 1 | |
| I-847 | -CH2C≡CH | H | CF3 | H | CN | H | H | 0 | |
| I-848 | -CH2C≡CH | H | CF3 | H | CN | H | H | 1 | |
| I-849 | CH3 | CH3 | H | H | CN | H | H | 0 | |
| I-850 | CH3 | CH3 | H | H | CN | H | H | 1 | |
| I-851 | CH3 | CH3 | CH3 | H | CN | H | H | 0 | |
| I-852 | CH3 | CH3 | CH3 | H | CN | H | H | 1 | |
| I-853 | CH3 | CH3 | Ph | H | CN | H | H | 0 | |
| I-854 | CH3 | CH3 | Ph | H | CN | H | H | 1 | |
| I-855 | -C2H4- | CH3 | -C2H4- | H | CN | H | H | 0 | |
| I-856 | -C2H4- | CH3 | -C2H4- | H | CN | H | H | 1 | |
| I-857 | -CO- | CH3 | -C2H4- | H | CN | H | H | 0 | |
| I-858 | -CO- | CH3 | -C2H4- | H | CN | H | H | 1 | |
| I-859 | H | H | CF3 | H | CN | H | Cl | 0 | |
| I-860 | H | H | CF3 | H | CN | H | Cl | 1 | |
| I-861 | CH3 | H | CF3 | H | CN | H | Cl | 0 | |
| I-862 | CH3 | H | CF3 | H | CN | H | Cl | 1 | |
| I-863 | CH3 | CH3 | CF3 | H | CN | H | Cl | 0 | |
| I-864 | CH3 | CH3 | CF3 | H | CN | H | Cl | 1 | |
| I-865 | CH2CF3 | H | CF3 | H | CN | H | Cl | 0 | |
| I-866 | CH2CF3 | H | CF3 | H | CN | H | Cl | 1 | |
| I-867 | i-Pr | H | CF3 | H | CN | H | Cl | 0 | |
| I-868 | i-Pr | H | CF3 | H | CN | H | Cl | 1 | |
| I-869 | CH2CH3 | CH2CH3 | CF3 | H | CN | H | Cl | 0 | |
| I-870 | CH2CH3 | CH2CH3 | CF3 | H | CN | H | Cl | 1 | |
| I-871 | -CH2CH=CH2 | H | CF3 | H | CN | H | Cl | 0 | |
| I-872 | -CH2CH=CH2 | H | CF3 | H | CN | H | Cl | 1 | |
| I-873 | -CH2C≡CH | H | CF3 | H | CN | H | Cl | 0 | |
| I-874 | -CH2C≡CH | H | CF3 | H | CN | H | Cl | 1 | |
| I-875 | CH3 | CH₃ | H | H | CN | H | Cl | 0 | |
| I-876 | CH3 | CH3 | H | H | CN | H | Cl | 1 | |
| I-877 | CH3 | CH3 | CH3 | H | CN | H | Cl | 0 | |
| I-878 | CH3 | CH3 | CH3 | H | CN | H | Cl | 1 | |
| I-879 | CH3 | CH3 | Ph | H | CN | H | Cl | 0 | |
| I-880 | CH3 | CH3 | Ph | H | CN | H | Cl | 1 | |
| I-881 | -C2H4- | CH3 | -C2H4- | H | CN | H | Cl | 0 | |
| I-882 | -C2H4- | CH3 | -C2H4- | H | CN | H | Cl | 1 | |
| I-883 | -CO- | CH3 | -C2H4- | H | CN | H | CI | 0 | |
| I-884 | -CO- | CH3 | -C2H4- | H | CN | H | Cl | 1 | |
| I-885 | H | H | CF3 | H | CN | H | Br | 0 | |
| I-886 | H | H | CF3 | H | CN | H | Br | 1 | |
| I-887 | CH3 | H | CF3 | H | CN | H | Br | 0 | |
| I-888 | CH3 | H | CF3 | H | CN | H | Br | 1 | |
| I-889 | CH3 | CH3 | CF3 | H | CN | H | Br | 0 | |
| I-890 | CH3 | CH3 | CF3 | H | CN | H | Br | 1 | |
| I-891 | CH2CF3 | H | CF3 | H | CN | H | Br | 0 | |
| I-892 | CH2CF3 | H | CF3 | H | CN | H | Br | 1 | |
| I-893 | i-Pr | H | CF3 | H | CN | H | Br | 0 | |
| I-894 | i-Pr | H | CF3 | H | CN | H | Br | 1 | |
| I-895 | CH2CH3 | CH2CH3 | CF3 | H | CN | H | Br | 0 | |
| I-896 | CH2CH3 | CH2CH3 | CF3 | H | CN | H | Br | 1 | |
| I-897 | -CH2CH=CH2 | H | CF3 | H | CN | H | Br | 0 | |
| I-898 | -CH2CH=CH2 | H | CF3 | H | CN | H | Br | 1 | |
| I-899 | -CH2C≡CH | H | CF3 | H | CN | H | Br | 0 | |
| I-900 | -CH2C≡CH | H | CF3 | H | CN | H | Br | 1 | |
| I-901 | CH3 | CH3 | H | H | CN | H | Br | 0 | |
| I-902 | CH3 | CH3 | H | H | CN | H | Br | 1 | |
| I-903 | CH3 | CH3 | CH3 | H | CN | H | Br | 0 | |
| I-904 | CH3 | CH3 | CH3 | H | CN | H | Br | 1 | |
| I-905 | CH3 | CH3 | Ph | H | CN | H | Br | 0 | |
| I-906 | CH3 | CH3 | Ph | H | CN | H | Br | 1 | |
| I-907 | -C2H4- | CH3 | -C2H4- | H | CN | H | Br | 0 | |
| I-908 | -C2H4- | CH3 | -C2H4- | H | CN | H | Br | 1 | |
| I-909 | -CO- | CH3 | -C2H4- | H | CN | H | Br | 0 | |
| I-910 | -CO- | CH3 | -C2H4- | H | CN | H | Br | 1 | |
| I-911 | H | H | CF3 | H | CN | H | I | 0 | |
| I-912 | H | H | CF3 | H | CN | H | I | 1 | |
| I-913 | CH3 | H | CF3 | H | CN | H | I | 0 | |
| I-914 | CH3 | H | CF3 | H | CN | H | I | 1 | |
| I-915 | CH3 | CH3 | CF3 | H | CN | H | I | 0 | |
| I-916 | CH3 | CH3 | CF3 | H | CN | H | I | 1 | |
| I-917 | CH2CF3 | H | CF3 | H | CN | H | I | 0 | |
| I-918 | CH2CF3 | H | CF3 | H | CN | H | I | 1 | |
| I-919 | i-Pr | H | CF3 | H | CN | H | I | 0 | |
| I-920 | i-Pr | H | CF3 | H | CN | H | I | 1 | |
| I-921 | CH2CH3 | CH2CH3 | CF3 | H | CN | H | I | 0 | |
| I-922 | CH2CH3 | CH2CH3 | CF3 | H | CN | H | I | 1 | |
| I-923 | -CH2CH=CH2 | H | CF3 | H | CN | H | I | 0 | |
| I-924 | -CH2CH=CH2 | H | CF3 | H | CN | H | I | 1 | |
| I-925 | -CH2C≡CH | H | CF3 | H | CN | H | I | 0 | |
| I-926 | -CH2C≡CH | H | CF3 | H | CN | H | I | 1 | |
| I-927 | CH3 | CH3 | H | H | CN | H | I | 0 | |
| I-928 | CH3 | CH3 | H | H | CN | H | I | 1 | |
| I-929 | CH3 | CH3 | CH3 | H | CN | H | I | 0 | |
| I-930 | CH3 | CH3 | CH3 | H | CN | H | I | 1 | |
| I-931 | CH3 | CH3 | Ph | H | CN | H | I | 0 | |
| I-932 | CH3 | CH3 | Ph | H | CN | H | I | 1 | |
| I-933 | -C2H4- | CH3 | -C2H4- | H | CN | H | I | 0 | |
| I-934 | -C2H4- | CH3 | -C2H4- | H | CN | H | I | 1 | |
| I-935 | -CO- | CH3 | -C2H4- | H | CN | H | I | 0 | |
| I-936 | -CO- | CH3 | -C2H4- | H | CN | H | I | 1 | |
| I-937 | H | H | CF3 | Cl | CN | H | H | 0 | |
| I-938 | H | H | CF3 | Cl | CN | H | H | 1 | |
| I-939 | CH3 | H | CF3 | Cl | CN | H | H | 0 | |
| I-940 | CH3 | H | CF3 | Cl | CN | H | H | 1 | |
| I-941 | CH3 | CH3 | CF3 | Cl | CN | H | H | 0 | |
| I-942 | CH3 | CH3 | CF3 | Cl | CN | H | H | 1 | |
| I-943 | CH2CF3 | H | CF3 | Cl | CN | H | H | 0 | |
| I-944 | CH2CF3 | H | CF3 | Cl | CN | H | H | 1 | |
| I-945 | i-Pr | H | CF3 | Cl | CN | H | H | 0 | |
| I-946 | i-Pr | H | CF3 | Cl | CN | H | H | 1 | |
| I-947 | CH2CH3 | CH2CH3 | CF3 | Cl | CN | H | H | 0 | |
| I-948 | CH2CH3 | CH2CH3 | CF3 | Cl | CN | H | H | 1 | |
| I-949 | -CH2CH=CH2 | H | CF3 | Cl | CN | H | H | 0 | |
| I-950 | -CH2CH=CH2 | H | CF3 | Cl | CN | H | H | 1 | |
| I-951 | -CH2C≡CH | H | CF3 | Cl | CN | H | H | 0 | |
| I-952 | -CH2C≡CH | H | CF3 | Cl | CN | H | H | 1 | |
| I-953 | CH3 | CH3 | H | Cl | CN | H | H | 0 | |
| I-954 | CH3 | CH3 | H | Cl | CN | H | H | 1 | |
| I-955 | CH3 | CH3 | CH3 | Cl | CN | H | H | 0 | |
| I-956 | CH3 | CH3 | CH3 | Cl | CN | H | H | 1 | |
| I-957 | CH3 | CH3 | Ph | Cl | CN | H | H | 0 | |
| I-958 | CH3 | CH3 | Ph | Cl | CN | H | H | 1 | |
| I-959 | -C2H4- | CH3 | -C2H4- | Cl | CN | H | H | 0 | |
| I-960 | -C2H4- | CH3 | -C2H4- | Cl | CN | H | H | 1 | |
| I-961 | -CO- | CH3 | -C2H4- | Cl | CN | H | H | 0 | |
| I-962 | -CO- | CH3 | -C2H4- | Cl | CN | H | H | 1 | |
| I-963 | H | H | CF3 | H | CN | H | CH3 | 0 | |
| I-964 | H | H | CF3 | H | CN | H | CH3 | 1 | |
| I-965 | CH3 | H | CF3 | H | CN | H | CH3 | 0 | |
| I-966 | CH3 | H | CF3 | H | CN | H | CH3 | 1 | |
| I-967 | CH3 | CH3 | CF3 | H | CN | H | CH3 | 0 | |
| I-968 | CH3 | CH3 | CF3 | H | CN | H | CH3 | 1 | |
| I-969 | CH2CF3 | H | CF3 | H | CN | H | CH3 | 0 | |
| I-970 | CH2CF3 | H | CF3 | H | CN | H | CH3 | 1 | |
| I-971 | i-Pr | H | CF3 | H | CN | H | CH3 | 0 | |
| I-972 | i-Pr | H | CF3 | H | CN | H | CH3 | 1 | |
| I-973 | CH2CH3 | CH2CH3 | CF3 | H | CN | H | CH3 | 0 | |
| I-974 | CH2CH3 | CH2CH3 | CF3 | H | CN | H | CH3 | 1 | |
| I-975 | -CH2CH=CH2 | H | CF3 | H | CN | H | CH3 | 0 | |
| I-976 | -CH2CH=CH2 | H | CF3 | H | CN | H | CH3 | 1 | |
| I-977 | -CH2C≡CH | H | CF3 | H | CN | H | CH3 | 0 | |
| I-978 | -CH2C≡CH | H | CF3 | H | CN | H | CH3 | 1 | |
| I-979 | CH3 | CH3 | H | H | CN | H | CH3 | 0 | |
| I-980 | CH3 | CH3 | H | H | CN | H | CH3 | 1 | |
| I-981 | CH3 | CH3 | CH3 | H | CN | H | CH3 | 0 | 1.5463 |
| I-982 | CH3 | CH3 | CH3 | H | CN | H | CH3 | 1 | 1.5472 |
| I-983 | CH3 | CH3 | Ph | H | CN | H | CH3 | 0 | |
| I-984 | CH3 | CH3 | Ph | H | CN | H | CH3 | 1 | |
| I-985 | -C2H4- | CH3 | -C2H4- | H | CN | H | CH3 | 0 | |
| I-986 | -C2H4- | CH3 | -C2H4- | H | CN | H | CH3 | 1 | |
| I-987 | -CO- | CH3 | -C2H4- | H | CN | H | CH3 | 0 | |
| I-988 | -CO- | CH3 | -C2H4- | H | CN | H | CH3 | 1 | |
| I-989 | H | H | CF3 | H | CN | H | CH3 | 0 | |
| I-990 | H | H | CF3 | H | CN | H | CH3 | 1 | |
| I-991 | CH3 | H | CF3 | H | CN | H | CH3 | 0 | 1.517 |
| I-992 | CH3 | H | CF3 | H | CN | H | CH3 | 1 | |
| I-993 | CH3 | CH3 | CF3 | H | CN | H | CH3 | 0 | |
| I-994 | CH3 | CH3 | CF3 | H | CN | H | CH3 | 1 | |
| I-995 | CH2CF3 | H | CF3 | H | CN | H | CH3 | 0 | |
| I-996 | CH2CF3 | H | CF3 | H | CN | H | CH3 | 1 | |
| I-997 | i-Pr | H | CF3 | H | CN | H | CH3 | 0 | |
| I-998 | i-Pr | H | CF3 | H | CN | H | CH3 | 1 | |
| I-999 | CH2CH3 | CH2CH3 | CF3 | H | CN | H | CH3 | 0 | |
| I-1000 | CH2CH3 | CH2CH3 | CF3 | H | CN | H | CH3 | 1 | |
| I-1001 | -CH2CH=CH2 | H | CF3 | H | CN | H | CH3 | 0 | |
| I-1002 | -CH2CH=CH2 | H | CF3 | H | CN | H | CH3 | 1 | |
| I-1003 | -CH2C≡CH | H | CF3 | H | CN | H | CH3 | 0 | |
| I-1004 | -CH2C≡CH | H | CF3 | H | CN | H | CH3 | 1 | |
| I-1005 | CH3 | CH3 | H | H | CN | H | CH3 | 0 | |
| I-1006 | CH3 | CH3 | H | H | CN | H | CH3 | 1 | |
| I-1007 | CH3 | CH3 | CH3 | H | CN | H | CH3 | 0 | |
| I-1008 | CH3 | CH3 | CH3 | H | CN | H | CH3 | 1 | |
| I-1009 | CH3 | CH3 | Ph | H | CN | H | CH3 | 0 | |
| I-1010 | CH3 | CH3 | Ph | H | CN | H | CH3 | 1 | |
| I-1011 | -C2H4- | CH3 | -C2H4- | H | CN | H | CH3 | 0 | |
| I-1012 | -C2H4- | CH3 | -C2H4- | H | CN | H | CH3 | 1 | |
| I-1013 | -CO- | CH3 | -C2H4- | H | CN | H | CH3 | 0 | |
| I-1014 | -CO- | CH3 | -C2H4- | H | CN | H | CH3 | 1 | |
| I-1015 | H | H | CF3 | H | CN | H | CH3 | 0 | |
| I-1016 | H | H | CF3 | H | CN | H | CH3 | 1 | |
| I-1017 | CH3 | H | CF3 | H | CN | H | CH3 | 0 | |
| I-1018 | CH3 | H | CF3 | H | CN | H | CH3 | 1 | |
| I-1019 | CH3 | CH3 | CF3 | H | CN | H | CH3 | 0 | |
| I-1020 | CH3 | CH3 | CF3 | H | CN | H | CH3 | 1 | |
| I-1021 | CH2CF3 | H | CF3 | H | CN | H | CH3 | 0 | |
| I-1022 | CH2CF3 | H | CF3 | H | CN | H | CH3 | 1 | |
| I-1023 | i-Pr | H | CF3 | H | CN | H | CH3 | 0 | |
| I-1024 | i-Pr | H | CF3 | H | CN | H | CH3 | 1 | |
| I-1025 | CH2CH3 | CH2CH3 | CF3 | H | CN | H | CH3 | 0 | |
| I-1026 | CH2CH3 | CH2CH3 | CF3 | H | CN | H | CH3 | 1 | |
| I-1027 | -CH2CH=CH2 | H | CF3 | H | CN | H | CH3 | 0 | |
| I-1028 | -CH2CH=CH2 | H | CF3 | H | CN | H | CH3 | 1 | |
| I-1029 | -CH2C≡CH | H | CF3 | H | CN | H | CH3 | 0 | |
| I-1030 | -CH2C≡CH | H | CF3 | H | CN | H | CH3 | 1 | |
| I-1031 | CH3 | CH3 | H | H | CN | H | CH3 | 0 | |
| I-1032 | CH3 | CH3 | H | H | CN | H | CH3 | 1 | |
| I-1033 | CH3 | CH3 | CH3 | H | CN | H | CH3 | 0 | |
| I-1034 | CH3 | CH3 | CH3 | H | CN | H | CH3 | 1 | |
| I-1035 | CH3 | CH3 | Ph | H | CN | H | CH3 | 0 | |
| I-1036 | CH3 | CH3 | Ph | H | CN | H | CH3 | 1 | |
| I-1037 | -C2H4- | CH3 | -C2H4- | H | CN | H | CH3 | 0 | |
| I-1038 | -C2H4- | CH3 | -C2H4- | H | CN | H | CH3 | 1 | |
| I-1039 | -CO- | CH3 | -C2H4- | H | CN | H | CH3 | 0 | |
| I-1040 | -CO- | CH3 | -C2H4- | H | CN | H | CH3 | 1 | |
| I-1041 | H | H | CF3 | H | CN | H | CH3 | 0 | |
| I-1042 | H | H | CF3 | H | CN | H | CH3 | 1 | |
| I-1043 | CH3 | H | CF3 | H | CN | H | CH3 | 0 | |
| I-1044 | CH3 | H | CF3 | H | CN | H | CH3 | 1 | |
| I-1045 | CH3 | CH3 | CF3 | H | CN | H | CH3 | 0 | |
| I-1046 | CH3 | CH3 | CF3 | H | CN | H | CH3 | 1 | |
| I-1047 | CH2CF3 | H | CF3 | H | CN | H | CH3 | 0 | |
| I-1048 | CH2CF3 | H | CF3 | H | CN | H | CH3 | 1 | |
| I-1049 | i-Pr | H | CF3 | H | CN | H | CH3 | 0 | |
| I-1050 | i-Pr | H | CF3 | H | CN | H | CH3 | 1 | |
| I-1051 | CH2CH3 | CH2CH3 | CF3 | H | CN | H | CH3 | 0 | |
| I-1052 | CH2CH3 | CH2CH3 | CF3 | H | CN | H | CH3 | 1 | |
| I-1053 | -CH2CH=CH2 | H | CF3 | H | CN | H | CH3 | 0 | |
| I-1054 | -CH2CH=CH2 | H | CF3 | H | CN | H | CH3 | 1 | |
| I-1055 | -CH2C≡CH | H | CF3 | H | CN | H | CH3 | 0 | |
| I-1056 | -CH2C≡CH | H | CF3 | H | CN | H | CH3 | 1 | |
| I-1057 | CH3 | CH3 | H | H | CN | H | CH3 | 0 | |
| I-1058 | CH3 | CH3 | H | H | CN | H | CH3 | 1 | |
| I-1059 | CH3 | CH3 | CH3 | H | CN | H | CH3 | 0 | |
| I-1060 | CH3 | CH3 | CH3 | H | CN | H | CH3 | 1 | |
| I-1061 | CH3 | CH3 | Ph | H | CN | H | CH3 | 0 | |
| I-1062 | CH3 | CH3 | Ph | H | CN | H | CH3 | 1 | |
| I-1063 | -C2H4- | CH3 | -C2H4- | H | CN | H | CH3 | 0 | |
| I-1064 | -C2H4- | CH3 | -C2H4- | H | CN | H | CH3 | 1 | |
| I-1065 | -CO- | CH3 | -C2H4- | H | CN | H | CH3 | 0 | |
| I-1066 | -CO- | CH3 | -C2H4- | H | CN | H | CH3 | 1 | |
| I-1067 | H | H | CF3 | Cl | CN | H | CH3 | 0 | |
| I-1068 | H | H | CF3 | Cl | CN | H | CH3 | 1 | |
| I-1069 | CH3 | H | CF3 | Cl | CN | H | CH3 | 0 | |
| I-1070 | CH3 | H | CF3 | Cl | CN | H | CH3 | 1 | |
| I-1071 | CH3 | CH3 | CF3 | Cl | CN | H | CH3 | 0 | |
| I-1072 | CH3 | CH3 | CF3 | Cl | CN | H | CH3 | 1 | |
| I-1073 | CH2CF3 | H | CF3 | Cl | CN | H | CH3 | 0 | |
| I-1074 | CH2CF3 | H | CF3 | Cl | CN | H | CH3 | 1 | |
| I-1075 | i-Pr | H | CF3 | Cl | CN | H | CH3 | 0 | |
| I-1076 | i-Pr | H | CF3 | Cl | CN | H | CH3 | 1 | |
| I-1077 | CH2CH3 | CH2CH3 | CF3 | Cl | CN | H | CH3 | 0 | |
| I-1078 | CH2CH3 | CH2CH3 | CF3 | Cl | CN | H | CH3 | 1 | |
| I-1079 | -CH2CH=CH2 | H | CF3 | Cl | CN | H | CH3 | 0 | |
| I-1080 | -CH2CH=CH2 | H | CF3 | Cl | CN | H | CH3 | 1 | |
| I-1081 | -CH2C≡CH | H | CF3 | Cl | CN | H | CH3 | 0 | |
| I-1082 | -CH2C≡CH | H | CF3 | Cl | CN | H | CH3 | 1 | |
| I-1083 | CH3 | CH3 | H | Cl | CN | H | CH3 | 0 | |
| I-1084 | CH3 | CH3 | H | Cl | CN | H | CH3 | 1 | |
| I-1085 | CH3 | CH3 | CH3 | Cl | CN | H | CH3 | 0 | 1.5516 |
| I-1086 | CH3 | CH3 | CH3 | Cl | CN | H | CH3 | 1 | |
| I-1087 | CH3 | CH3 | Ph | Cl | CN | H | CH3 | 0 | |
| I-1088 | CH3 | CH3 | Ph | Cl | CN | H | CH3 | 1 | |
| I-1089 | -C2H4- | CH3 | -C2H4- | Cl | CN | H | CH3 | 0 | |
| I-1090 | -C2H4- | CH3 | -C2H4- | Cl | CN | H | CH3 | 1 | |
| I-1091 | -CO- | CH3 | -C2H4- | Cl | CN | H | CH3 | 0 | |
| I-1092 | -CO- | CH3 | -C2H4- | Cl | CN | H | CH3 | 1 | |
| I-1093 | H | H | CF3 | H | CN | H | H | 0 | |
| I-1094 | H | H | CF3 | H | CN | H | H | 1 | |
| I-1095 | CH3 | H | CF3 | H | CN | H | H | 0 | |
| I-1096 | CH3 | H | CF3 | H | CN | H | H | 1 | |
| I-1097 | CH3 | CH3 | CF3 | H | CN | H | H | 0 | |
| I-1098 | CH3 | CH3 | CF3 | H | CN | H | H | 1 | |
| I-1099 | CH2CF3 | H | CF3 | H | CN | H | H | 0 | |
| I-1100 | CH2CF3 | H | CF3 | H | CN | H | H | 1 | |
| I-1101 | i-Pr | H | CF3 | H | CN | H | H | 0 | |
| I-1102 | i-Pr | H | CF3 | H | CN | H | H | 1 | |
| I-1103 | CH2CH3 | CH2CH3 | CF3 | H | CN | H | H | 0 | |
| I-1104 | CH2CH3 | CH2CH3 | CF3 | H | CN | H | H | 1 | |
| I-1105 | -CH2CH=CH2 | H | CF3 | H | CN | H | H | 0 | |
| I-1106 | -CH2CH=CH2 | H | CF3 | H | CN | H | H | 1 | |
| I-1107 | -CH2C≡CH | H | CF3 | H | CN | H | H | 0 | |
| I-1108 | -CH2C≡CH | H | CF3 | H | CN | H | H | 1 | |
| 1-1109 | CH3 | CH3 | H | H | CN | H | H | 0 | |
| I-1110 | CH3 | CH3 | H | H | CN | H | H | 1 | |
| I-1111 | CH3 | CH3 | CH3 | H | CN | H | H | 0 | |
| I-1112 | CH3 | CH3 | CH3 | H | CN | H | H | 1 | |
| I-1113 | CH3 | CH3 | Ph | H | CN | H | H | 0 | |
| I-1114 | CH3 | CH3 | Ph | H | CN | H | H | 1 | |
| I-1115 | -C2H4- | CH3 | -C2H4- | H | CN | H | H | 0 | |
| I-1116 | -C2H4- | CH3 | -C2H4- | H | CN | H | H | 1 | |
| I-1117 | -CO- | CH3 | -C2H4- | H | CN | H | H | 0 | |
| I-1118 | -CO- | CH3 | -C2H4- | H | CN | H | H | 1 | |
| I-1119 | H | H | CF3 | H | CN | H | Cl | 0 | |
| I-1120 | H | H | CF3 | H | CN | H | Cl | 1 | |
| I-1121 | CH3 | H | CF3 | H | CN | H | Cl | 0 | |
| I-1122 | CH3 | H | CF3 | H | CN | H | Cl | 1 | |
| I-1123 | CH3 | CH3 | CF3 | H | CN | H | Cl | 0 | |
| I-1124 | CH3 | CH3 | CF3 | H | CN | H | Cl | 1 | |
| I-1125 | CH2CF3 | H | CF3 | H | CN | H | Cl | 0 | |
| I-1126 | CH2CF3 | H | CF3 | H | CN | H | Cl | 1 | |
| I-1127 | i-Pr | H | CF3 | H | CN | H | Cl | 0 | |
| I-1128 | i-Pr | H | CF3 | H | CN | H | Cl | 1 | |
| I-1129 | CH2CH3 | CH2CH3 | CF3 | H | CN | H | Cl | 0 | |
| I-1130 | CH2CH3 | CH2CH3 | CF3 | H | CN | H | Cl | 1 | |
| I-1131 | -CH2CH=CH2 | H | CF3 | H | CN | H | Cl | 0 | |
| 1-1132 | -CH2CH=CH2 | H | CF3 | H | CN | H | Cl | 1 | |
| I-1133 | -CH2C≡CH | H | CF3 | H | CN | H | Cl | 0 | |
| I-1134 | -CH2C≡CH | H | CF3 | H | CN | H | Cl | 1 | |
| I-1135 | CH3 | CH3 | H | H | CN | H | Cl | 0 | |
| I-1136 | CH3 | CH3 | H | H | CN | H | Cl | 1 | |
| I-1137 | CH3 | CH3 | CH3 | H | CN | H | Cl | 0 | |
| I-1138 | CH3 | CH3 | CH3 | H | CN | H | Cl | 1 | |
| I-1139 | CH3 | CH3 | Ph | H | CN | H | Cl | 0 | |
| I-1140 | CH3 | CH3 | Ph | H | CN | H | Cl | 1 | |
| I-1141 | -C2H4- | CH3 | -C2H4- | H | CN | H | Cl | 0 | |
| I-1142 | -C2H4- | CH3 | -C2H4- | H | CN | H | Cl | 1 | |
| I-1143 | -CO- | CH3 | -C2H4- | H | CN | H | Cl | 0 | |
| I-1144 | -CO- | CH3 | -C2H4- | H | CN | H | Cl | 1 | |
| I-1145 | H | H | CF3 | H | CN | H | Br | 0 | |
| I-1146 | H | H | CF3 | H | CN | H | Br | 1 | |
| I-1147 | CH3 | H | CF3 | H | CN | H | Br | 0 | |
| I-1148 | CH3 | H | CF3 | H | CN | H | Br | 1 | |
| I-1149 | CH3 | CH3 | CF3 | H | CN | H | Br | 0 | |
| I-1150 | CH3 | CH₃ | CF3 | H | CN | H | Br | 1 | |
| I-1151 | CH2CF3 | H | CF3 | H | CN | H | Br | 0 | |
| I-1152 | CH2CF3 | H | CF3 | H | CN | H | Br | 1 | |
| I-1153 | i-Pr | H | CF3 | H | CN | H | Br | 0 | |
| I-1154 | i-Pr | H | CF3 | H | CN | H | Br | 1 | |
| I-1155 | CH2CH3 | CH2CH3 | CF3 | H | CN | H | Br | 0 | |
| I-1156 | CH2CH3 | CH2CH3 | CF3 | H | CN | H | Br | 1 | |
| I-1157 | -CH2CH=CH2 | H | CF3 | H | CN | H | Br | 0 | |
| I-1158 | -CH2CH=CH2 | H | CF3 | H | CN | H | Br | 1 | |
| I-1159 | -CH2C≡CH | H | CF3 | H | CN | H | Br | 0 | |
| I-1160 | -CH2C≡CH | H | CF3 | H | CN | H | Br | 1 | |
| 1-1161 | CH3 | CH3 | H | H | CN | H | Br | 0 | |
| I-1162 | CH3 | CH3 | H | H | CN | H | Br | 1 | |
| I-1163 | CH3 | CH3 | CH3 | H | CN | H | Br | 0 | |
| I-1164 | CH3 | CH3 | CH3 | H | CN | H | Br | 1 | |
| I-1165 | CH3 | CH3 | Ph | H | CN | H | Br | 0 | |
| I-1166 | CH3 | CH3 | Ph | H | CN | H | Br | 1 | |
| I-1167 | -C2H4- | CH3 | -C2H4- | H | CN | H | Br | 0 | |
| I-1168 | -C2H4- | CH3 | -C2H4- | H | CN | H | Br | 1 | |
| 1-1169 | -CO- | CH3 | -C2H4- | H | CN | H | Br | 0 | |
| I-1170 | -CO- | CH3 | -C2H4- | H | CN | H | Br | 1 | |
| I-1171 | H | H | CF3 | H | CN | H | I | 0 | |
| 1-1172 | H | H | CF3 | H | CN | H | I | 1 | |
| I-1173 | CH3 | H | CF3 | H | CN | H | I | 0 | |
| I-1174 | CH3 | H | CF3 | H | CN | H | I | 1 | |
| I-1175 | CH3 | CH3 | CF3 | H | CN | H | I | 0 | |
| I-1176 | CH3 | CH3 | CF3 | H | CN | H | I | 1 | |
| I-1177 | CH2CF3 | H | CF3 | H | CN | H | I | 0 | |
| I-1178 | CH2CF3 | H | CF3 | H | CN | H | I | 1 | |
| I-1179 | i-Pr | H | CF3 | H | CN | H | I | 0 | |
| I-1180 | i-Pr | H | CF3 | H | CN | H | I | 1 | |
| I-1181 | CH2CH3 | CH2CH3 | CF3 | H | CN | H | I | 0 | |
| I-1182 | CH2CH3 | CH2CH3 | CF3 | H | CN | H | I | 1 | |
| I-1183 | -CH2CH=CH2 | H | CF3 | H | CN | H | I | 0 | |
| I-1184 | -CH2CH=CH2 | H | CF3 | H | CN | H | I | 1 | |
| I-1185 | -CH2C≡CH | H | CF3 | H | CN | H | I | 0 | |
| I-1186 | -CH2C≡CH | H | CF3 | H | CN | H | I | 1 | |
| I-1187 | CH3 | CH3 | H | H | CN | H | I | 0 | |
| I-1188 | CH3 | CH3 | H | H | CN | H | I | 1 | |
| I-1189 | CH3 | CH3 | CH3 | H | CN | H | I | 0 | |
| I-1190 | CH3 | CH3 | CH3 | H | CN | H | I | 1 | |
| I-1191 | CH3 | CH3 | Ph | H | CN | H | I | 0 | |
| I-1192 | CH3 | CH3 | Ph | H | CN | H | I | 1 | |
| I-1193 | -C2H4- | CH3 | -C2H4- | H | CN | H | I | 0 | |
| I-1194 | -C2H4- | CH3 | -C2H4- | H | CN | H | I | 1 | |
| I-1195 | -CO- | CH3 | -C2H4- | H | CN | H | I | 0 | |
| I-1196 | -CO- | CH3 | -C2H4- | H | CN | H | I | 1 | |
| I-1197 | H | H | CF3 | Cl | CN | H | H | 0 | |
| I-1198 | H | H | CF3 | Cl | CN | H | H | 1 | |
| I-1199 | CH3 | H | CF3 | Cl | CN | H | H | 0 | |
| I-1200 | CH3 | H | CF3 | Cl | CN | H | H | 1 | |
| I-1201 | CH3 | CH3 | CF3 | Cl | CN | H | H | 0 | |
| I-1202 | CH3 | CH3 | CF3 | Cl | CN | H | H | 1 | |
| I-1203 | CH2CF3 | H | CF3 | Cl | CN | H | H | 0 | |
| I-1204 | CH2CF3 | H | CF3 | Cl | CN | H | H | 1 | |
| I-1205 | i-Pr | H | CF3 | Cl | CN | H | H | 0 | |
| I-1206 | i-Pr | H | CF3 | Cl | CN | H | H | 1 | |
| I-1207 | CH2CH3 | CH2CH3 | CF3 | Cl | CN | H | H | 0 | |
| I-1208 | CH2CH3 | CH2CH3 | CF3 | Cl | CN | H | H | 1 | |
| I-1209 | -CH2CH=CH2 | H | CF3 | Cl | CN | H | H | 0 | |
| I-1210 | -CH2CH=CH2 | H | CF3 | Cl | CN | H | H | 1 | |
| I-1211 | -CH2C≡CH | H | CF3 | Cl | CN | H | H | 0 | |
| I-1212 | -CH2C≡CH | H | CF3 | Cl | CN | H | H | 1 | |
| I-1213 | CH3 | CH3 | H | Cl | CN | H | H | 0 | |
| I-1214 | CH3 | CH3 | H | Cl | CN | H | H | 1 | |
| I-1215 | CH3 | CH3 | CH3 | Cl | CN | H | H | 0 | |
| I-1216 | CH3 | CH3 | CH3 | Cl | CN | H | H | 1 | |
| I-1217 | CH3 | CH3 | Ph | Cl | CN | H | H | 0 | |
| I-1218 | CH3 | CH3 | Ph | Cl | CN | H | H | 1 | |
| I-1219 | -C2H4- | CH3 | -C2H4- | Cl | CN | H | H | 0 | |
| I-1220 | -C2H4- | CH3 | -C2H4- | Cl | CN | H | H | 1 | |
| I-1221 | -CO- | CH3 | -C2H4- | Cl | CN | H | H | 0 | |
| I-1222 | -CO- | CH3 | -C2H4- | Cl | CN | H | H | 1 | |
| I-1223 | H | H | CF3 | H | CN | H | NO2 | 0 | |
| I-1224 | H | H | CF3 | H | CN | H | NO2 | 1 | |
| I-1225 | CH3 | H | CF3 | H | CN | H | NO2 | 0 | |
| I-1226 | CH3 | H | CF3 | H | CN | H | NO2 | 1 | |
| I-1227 | CH3 | CH3 | CF3 | H | CN | H | NO2 | 0 | |
| I-1228 | CH3 | CH3 | CF3 | H | CN | H | NO2 | 1 | |
| I-1229 | CH2CF3 | H | CF3 | H | CN | H | NO2 | 0 | |
| I-1230 | CH2CF3 | H | CF3 | H | CN | H | NO2 | 1 | |
| I-1231 | i-Pr | H | CF3 | H | CN | H | NO2 | 0 | |
| I-1232 | i-Pr | H | CF3 | H | CN | H | NO2 | 1 | |
| I-1233 | CH2CH3 | CH2CH3 | CF3 | H | CN | H | NO2 | 0 | 1.5743 |
| I-1234 | CH2CH3 | CH2CH3 | CF3 | H | CN | H | NO2 | 1 | 112-114 |
| I-1235 | -CH2CH=CH2 | H | CF3 | H | CN | H | NO2 | 0 | |
| I-1236 | -CH2CH=CH2 | H | CF3 | H | CN | H | NO2 | 1 | |
| I-1237 | -CH2C≡CH | H | CF3 | H | CN | H | NO2 | 0 | |
| I-1238 | -CH2C≡CH | H | CF3 | H | CN | H | NO2 | 1 | |
| I-1239 | CH3 | CH3 | CN | H | CN | H | NO2 | 0 | |
| I-1240 | CH3 | CH3 | CN | H | CN | H | NO2 | 1 | |
| I-1241 | CH3 | CH3 | H | H | CN | H | NO2 | 0 | |
| I-1242 | CH3 | CH3 | H | H | CN | H | NO2 | 1 | |
| I-1243 | CH3 | CH3 | CH3 | H | CN | H | NO2 | 0 | |
| I-1244 | CH3 | CH3 | CH3 | H | CN | H | NO2 | 1 | |
| I-1245 | CH3 | CH3 | Ph | H | CN | H | NO2 | 0 | |
| I-1246 | CH3 | CH3 | Ph | H | CN | H | NO2 | 1 | |
| I-1247 | -C2H4- | CH3 | -C2H4- | H | CN | H | NO2 | 0 | |
| I-1248 | -C2H4- | CH3 | -C2H4- | H | CN | H | NO2 | 1 | |
| I-1249 | -CO- | CH3 | -C2H4- | H | CN | H | NO2 | 0 | |
| I-1250 | -CO- | CH3 | -C2H4- | H | CN | H | NO2 | 1 | |
| I-1251 | H | H | CF3 | H | CN | H | NO2 | 0 | |
| I-1252 | H | H | CF3 | H | CN | H | NO2 | 1 | |
| I-1253 | CH3 | H | CF3 | H | CN | H | NO2 | 0 | |
| I-1254 | CH3 | H | CF3 | H | CN | H | NO2 | 1 | |
| I-1255 | CH3 | CH3 | CF3 | H | CN | H | NO2 | 0 | |
| I-1256 | CH3 | CH3 | CF3 | H | CN | H | NO2 | 1 | |
| I-1257 | CH2CF3 | H | CF3 | H | CN | H | NO2 | 0 | |
| I-1258 | CH2CF3 | H | CF3 | H | CN | H | NO2 | 1 | |
| I-1259 | i-Pr | H | CF3 | H | CN | H | NO2 | 0 | |
| I-1260 | i-Pr | H | CF3 | H | CN | H | NO2 | 1 | |
| I-1261 | CH2CH3 | CH2CH3 | CF3 | H | CN | H | NO2 | 0 | |
| I-1262 | CH2CH3 | CH2CH3 | CF3 | H | CN | H | NO2 | 1 | |
| I-1263 | -CH2CH=CH2 | H | CF3 | H | CN | H | NO2 | 0 | |
| I-1264 | -CH2CH=CH2 | H | CF3 | H | CN | H | NO2 | 1 | |
| I-1265 | -CH2C≡CH | H | CF3 | H | CN | H | NO2 | 0 | |
| I-1266 | -CH2C≡CH | H | CF3 | H | CN | H | NO2 | 1 | |
| I-1267 | CH3 | CH3 | CN | H | CN | H | NO2 | 0 | |
| I-1268 | CH3 | CH3 | CN | H | CN | H | NO2 | 1 | |
| I-1269 | CH3 | CH3 | H | H | CN | H | NO2 | 0 | |
| I-1270 | CH3 | CH3 | H | H | CN | H | NO2 | 1 | |
| I-1271 | CH3 | CH3 | CH3 | H | CN | H | NO2 | 0 | |
| I-1272 | CH3 | CH3 | CH3 | H | CN | H | NO2 | 1 | |
| I-1273 | CH3 | CH3 | Ph | H | CN | H | NO2 | 0 | |
| I-1274 | CH3 | CH3 | Ph | H | CN | H | NO2 | 1 | |
| I-1275 | -C2H4- | CH3 | -C2H4- | H | CN | H | NO2 | 0 | |
| I-1276 | -C2H4- | CH3 | -C2H4- | H | CN | H | NO2 | 1 | |
| I-1277 | -CO- | CH3 | -C2H4- | H | CN | H | NO2 | 0 | |
| I-1278 | -CO- | CH3 | -C2H4- | H | CN | H | NO2 | 1 | |
| I-1279 | H | H | CF3 | H | CN | H | CF3 | 0 | |
| I-1280 | H | H | CF3 | H | CN | H | CF3 | 1 | |
| I-1281 | CH3 | H | CF3 | H | CN | H | CF3 | 0 | |
| I-1282 | CH3 | H | CF3 | H | CN | H | CF3 | 1 | |
| I-1283 | CH3 | CH3 | CF3 | H | CN | H | CF3 | 0 | |
| I-1284 | CH3 | CH3 | CF3 | H | CN | H | CF3 | 1 | |
| I-1285 | CH2CF3 | H | CF3 | H | CN | H | CF3 | 0 | |
| I-1286 | CH2CF3 | H | CF3 | H | CN | H | CF3 | 1 | |
| I-1287 | i-Pr | H | CF3 | H | CN | H | CF3 | 0 | |
| I-1288 | i-Pr | H | CF3 | H | CN | H | CF3 | 1 | |
| I-1289 | CH2CH3 | CH2CH3 | CF3 | H | CN | H | CF3 | 0 | |
| I-1290 | CH2CH3 | CH2CH3 | CF3 | H | CN | H | CF3 | 1 | |
| I-1291 | -CH2CH=CH2 | H | CF3 | H | CN | H | CF3 | 0 | |
| I-1292 | -CH2CH=CH2 | H | CF3 | H | CN | H | CF3 | 1 | |
| I-1293 | -CH2C≡CH | H | CF3 | H | CN | H | CF3 | 0 | |
| I-1294 | -CH2C≡CH | H | CF3 | H | CN | H | CF3 | 1 | |
| I-1295 | CH3 | CH3 | CN | H | CN | H | CF3 | 0 | |
| I-1296 | CH3 | CH3 | CN | H | CN | H | CF3 | 1 | |
| I-1297 | CH3 | CH3 | H | H | CN | H | CF3 | 0 | |
| I-1298 | CH3 | CH3 | H | H | CN | H | CF3 | 1 | |
| I-1299 | CH3 | CH3 | CH3 | H | CN | H | CF3 | 0 | |
| I-1300 | CH3 | CH3 | CH3 | H | CN | H | CF3 | 1 | |
| I-1301 | CH3 | CH3 | Ph | H | CN | H | CF3 | 0 | |
| I-1302 | CH3 | CH3 | Ph | H | CN | H | CF3 | 1 | |
| I-1303 | -C2H4- | CH3 | -C2H4- | H | CN | H | CF3 | 0 | |
| I-1304 | -C2H4- | CH3 | -C2H4- | H | CN | H | CF3 | 1 | |
| I-1305 | -CO- | CH3 | -C2H4- | H | CN | H | CF3 | 0 | |
| I-1306 | -CO- | CH3 | -C2H4- | H | CN | H | CF3 | 1 | |
| I-1307 | H | H | CF3 | H | CN | H | Cl | 0 | 93-100 |
| I-1308 | H | H | CF3 | H | CN | H | Cl | 1 | |
| I-1309 | CH3 | H | CF3 | H | CN | H | Cl | 0 | 1.5282 |
| I-1310 | CH3 | H | CF3 | H | CN | H | Cl | 1 | |
| I-1311 | CH3 | CH3 | CF3 | H | CN | H | Cl | 0 | |
| I-1312 | CH3 | CH3 | CF3 | H | CN | H | Cl | 1 | |
| I-1313 | CH2CF3 | H | CF3 | H | CN | H | Cl | 0 | |
| I-1314 | CH2CF3 | H | CF3 | H | CN | H | Cl | 1 | |
| I-1315 | i-Pr | H | CF3 | H | CN | H | Cl | 0 | |
| I-1316 | i-Pr | H | CF3 | H | CN | H | Cl | 1 | |
| I-1317 | CH2CH3 | CH2CH3 | CF3 | H | CN | H | Cl | 0 | |
| I-1318 | CH2CH3 | CH2CH3 | CF3 | H | CN | H | Cl | 1 | |
| I-1319 | -CH2CH=CH2 | H | CF3 | H | CN | H | Cl | 0 | |
| I-1320 | -CH2CH=CH2 | H | CF3 | H | CN | H | Cl | 1 | |
| I-1321 | -CH2C≡CH | H | CF3 | H | CN | H | Cl | 0 | |
| I-1322 | -CH2C≡CH | H | CF3 | H | CN | H | Cl | 1 | |
| I-1323 | CH3 | CH3 | H | H | CN | H | Cl | 0 | |
| I-1324 | CH3 | CH3 | H | H | CN | H | Cl | 1 | |
| I-1325 | CH3 | CH3 | CH3 | H | CN | H | Cl | 0 | |
| I-1326 | CH3 | CH3 | CH3 | H | CN | H | Cl | 1 | |
| I-1327 | CH3 | CH3 | Ph | H | CN | H | Cl | 0 | |
| I-1328 | CH3 | CH3 | Ph | H | CN | H | Cl | 1 | |
| I-1329 | -C2H4- | CH3 | -C2H4- | H | CN | H | Cl | 0 | |
| I-1330 | -C2H4- | CH3 | -C2H4- | H | CN | H | Cl | 1 | |
| I-1331 | -CO- | CH3 | -C2H4- | H | CN | H | Cl | 0 | |
| I-1332 | -CO- | CH3 | -C2H4- | H | CN | H | Cl | 1 | |
| I-1333 | H | H | CF3 | H | CN | H | Cl | 0 | |
| I-1334 | H | H | CF3 | H | CN | H | Cl | 1 | |
| I-1335 | CH3 | H | CF3 | H | CN | H | Cl | 0 | |
| I-1336 | CH3 | H | CF3 | H | CN | H | Cl | 1 | |
| I-1337 | CH3 | CH3 | CF3 | H | CN | H | Cl | 0 | |
| I-1338 | CH3 | CH3 | CF3 | H | CN | H | Cl | 1 | |
| I-1339 | CH2CF3 | H | CF3 | H | CN | H | Cl | 0 | |
| I-1340 | CH2CF3 | H | CF3 | H | CN | H | Cl | 1 | |
| I-1341 | i-Pr | H | CF3 | H | CN | H | Cl | 0 | |
| I-1342 | i-Pr | H | CF3 | H | CN | H | Cl | 1 | |
| I-1343 | CH2CH3 | CH2CH3 | CF3 | H | CN | H | Cl | 0 | |
| I-1344 | CH2CH3 | CH2CH3 | CF3 | H | CN | H | Cl | 1 | |
| I-1345 | -CH2CH=CH2 | H | CF3 | H | CN | H | Cl | 0 | |
| I-1346 | -CH2CH=CH2 | H | CF3 | H | CN | H | Cl | 1 | |
| I-1347 | -CH2C≡CH | H | CF3 | H | CN | H | Cl | 0 | |
| I-1348 | -CH2C≡CH | H | CF3 | H | CN | H | Cl | 1 | |
| I-1349 | CH3 | CH3 | H | H | CN | H | Cl | 0 | |
| I-1350 | CH3 | CH3 | H | H | CN | H | Cl | 1 | |
| I-1351 | CH3 | CH3 | CH3 | H | CN | H | Cl | 0 | |
| I-1352 | CH3 | CH3 | CH3 | H | CN | H | Cl | 1 | |
| I-1353 | CH3 | CH3 | Ph | H | CN | H | Cl | 0 | |
| I-1354 | CH3 | CH3 | Ph | H | CN | H | Cl | 1 | |
| I-1355 | -C2H4- | CH3 | -C2H4- | H | CN | H | Cl | 0 | |
| I-1356 | -C2H4- | CH3 | -C2H4- | H | CN | H | Cl | 1 | |
| I-1357 | -CO- | CH3 | -C2H4- | H | CN | H | Cl | 0 | |
| I-1358 | -CO- | CH3 | -C2H4- | H | CN | H | Cl | 1 | |
| I-1359 | H | H | CF3 | H | CN | H | Cl | 0 | |
| I-1360 | H | H | CF3 | H | CN | H | Cl | 1 | |
| I-1361 | CH3 | H | CF3 | H | CN | H | Cl | 0 | |
| I-1362 | CH3 | H | CF3 | H | CN | H | Cl | 1 | |
| I-1363 | CH3 | CH3 | CF3 | H | CN | H | Cl | 0 | |
| I-1364 | CH3 | CH3 | CF3 | H | CN | H | Cl | 1 | |
| I-1365 | CH2CF3 | H | CF3 | H | CN | H | Cl | 0 | |
| I-1366 | CH2CF3 | H | CF3 | H | CN | H | Cl | 1 | |
| I-1367 | i-Pr | H | CF3 | H | CN | H | Cl | 0 | |
| I-1368 | i-Pr | H | CF3 | H | CN | H | Cl | 1 | |
| I-1369 | CH2CH3 | CH2CH3 | CF3 | H | CN | H | Cl | 0 | |
| I-1370 | CH2CH3 | CH2CH3 | CF3 | H | CN | H | Cl | 1 | |
| I-1371 | -CH2CH=CH2 | H | CF3 | H | CN | H | Cl | 0 | |
| I-1372 | -CH2CH=CH2 | H | CF3 | H | CN | H | Cl | 1 | |
| I-1373 | -CH2C≡CH | H | CF3 | H | CN | H | Cl | 0 | |
| I-1374 | -CH2C≡CH | H | CF3 | H | CN | H | Cl | 1 | |
| I-1375 | CH3 | CH3 | H | H | CN | H | Cl | 0 | |
| I-1376 | CH3 | CH3 | H | H | CN | H | Cl | 1 | |
| I-1377 | CH3 | CH3 | CH3 | H | CN | H | Cl | 0 | |
| I-1378 | CH3 | CH3 | CH3 | H | CN | H | Cl | 1 | |
| I-1379 | CH3 | CH3 | Ph | H | CN | H | Cl | 0 | |
| I-1380 | CH3 | CH3 | Ph | H | CN | H | Cl | 1 | |
| I-1381 | -C2H4- | CH3 | -C2H4- | H | CN | H | Cl | 0 | |
| I-1382 | -C2H4- | CH3 | -C2H4- | H | CN | H | Cl | 1 | |
| I-1383 | -CO- | CH3 | -C2H4- | H | CN | H | Cl | 0 | |
| I-1384 | -CO- | CH3 | -C2H4- | H | CN | H | Cl | 1 | |
| I-1385 | H | H | CF3 | H | CN | H | Br | 0 | |
| I-1386 | H | H | CF3 | H | CN | H | Br | 1 | |
| I-1387 | CH3 | H | CF3 | H | CN | H | Br | 0 | |
| I-1388 | CH3 | H | CF3 | H | CN | H | Br | 1 | |
| I-1389 | CH3 | CH3 | CF3 | H | CN | H | Br | 0 | |
| I-1390 | CH3 | CH3 | CF3 | H | CN | H | Br | 1 | |
| I-1391 | CH2CF3 | H | CF3 | H | CN | H | Br | 0 | |
| I-1392 | CH2CF3 | H | CF3 | H | CN | H | Br | 1 | |
| I-1393 | i-Pr | H | CF3 | H | CN | H | Br | 0 | |
| I-1394 | i-Pr | H | CF3 | H | CN | H | Br | 1 | |
| I-1395 | CH2CH3 | CH2CH3 | CF3 | H | CN | H | Br | 0 | |
| I-1396 | CH2CH3 | CH2CH3 | CF3 | H | CN | H | Br | 1 | |
| I-1397 | -CH2CH=CH2 | H | CF3 | H | CN | H | Br | 0 | |
| I-1398 | -CH2CH=CH2 | H | CF3 | H | CN | H | Br | 1 | |
| I-1399 | -CH2C≡CH | H | CF3 | H | CN | H | Br | 0 | |
| I-1400 | -CH2C≡CH | H | CF3 | H | CN | H | Br | 1 | |
| I-1401 | CH3 | CH3 | H | H | CN | H | Br | 0 | |
| I-1402 | CH3 | CH3 | H | H | CN | H | Br | 1 | |
| I-1403 | CH3 | CH3 | CH3 | H | CN | H | Br | 0 | |
| I-1404 | CH3 | CH3 | CH3 | H | CN | H | Br | 1 | |
| I-1405 | CH3 | CH3 | Ph | H | CN | H | Br | 0 | |
| I-1406 | CH3 | CH3 | Ph | H | CN | H | Br | 1 | |
| I-1407 | -C2H4- | CH3 | -C2H4- | H | CN | H | Br | 0 | |
| I-1408 | -C2H4- | CH3 | -C2H4- | H | CN | H | Br | 1 | |
| I-1409 | -CO- | CH3 | -C2H4- | H | CN | H | Br | 0 | |
| I-1410 | -CO- | CH3 | -C2H4- | H | CN | H | Br | 1 | |

**[TABLE 2](Not according to the invention)**

| | | | | | | | | | | |
|---|---|---|---|---|---|---|---|---|---|---|
| Table 2 | 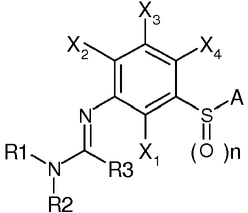 | | | | | | | | | |

| Comp. No. | R1 | R2 | R3 | X1 | X2 | X3 | X4 | A | n | nD20 or m.p. |
|---|---|---|---|---|---|---|---|---|---|---|
| II-1 | CH3 | H | CF3 | H | H | H | CN | CH3 | 0 | |
| II-2 | CH3 | H | CF3 | H | H | H | CN | CH3 | 1 | |
| II-3 | CH3 | CH3 | CF3 | H | H | H | CN | CH3 | 0 | 1.597 |
| II-4 | CH3 | CH3 | CF3 | H | H | H | CN | CH3 | 1 | |
| II-5 | CH3 | H | CF3 | H | F | H | CN | CH3 | 0 | |
| II-6 | CH3 | H | CF3 | H | F | H | CN | CH3 | 1 | |
| II-7 | CH3 | CH3 | CF3 | H | F | H | CN | CH3 | 0 | |
| II-8 | CH3 | CH3 | CF3 | H | F | H | CN | CH3 | 1 | |
| II-9 | CH3 | H | CF3 | H | Cl | H | CN | CH3 | 0 | |
| II-10 | CH3 | H | CF3 | H | Cl | H | CN | CH3 | 1 | |
| II-11 | CH3 | CH3 | CF3 | H | Cl | H | CN | CH3 | 0 | |
| II-12 | CH3 | CH3 | CF3 | H | Cl | H | CN | CH3 | 1 | |
| II-13 | CH3 | H | CF3 | H | CN | H | H | CH3 | 0 | |
| II-14 | CH3 | H | CF3 | H | CN | H | H | CH3 | 1 | |
| II-15 | CH3 | CH3 | CF3 | H | CN | H | H | CH3 | 0 | |
| II-16 | CH3 | CH3 | CF3 | H | CN | H | H | CH3 | 1 | |
| II-17 | CH3 | H | CF3 | H | CN | H | Cl | CH3 | 0 | |
| II-18 | CH3 | H | CF3 | H | CN | H | Cl | CH3 | 1 | |
| II-19 | CH3 | CH3 | CF3 | H | CN | H | Cl | CH3 | 0 | |
| II-20 | CH3 | CH3 | CF3 | H | CN | H | Cl | CH3 | 1 | |
| II-21 | CH3 | H | CF3 | H | H | H | CF3 | CH3 | 0 | |
| II-22 | CH3 | H | CF3 | H | H | H | CF3 | CH3 | 1 | |
| II-23 | CH3 | CH3 | CF3 | H | H | H | CF3 | CH3 | 0 | |
| II-24 | CH3 | CH3 | CF3 | H | H | H | CF3 | CH3 | 1 | |
| II-25 | CH3 | H | CF3 | H | Cl | H | CF3 | CH3 | 0 | |
| II-26 | CH3 | H | CF3 | H | Cl | H | CF3 | CH3 | 1 | |
| II-27 | CH3 | CH3 | CF3 | H | Cl | H | CF3 | CH3 | 0 | |
| II-28 | CH3 | CH3 | CF3 | H | Cl | H | CF3 | CH3 | 1 | |
| II-29 | CH3 | H | CF3 | H | CF3 | H | H | CH3 | 0 | |
| II-30 | CH3 | H | CF3 | H | CF3 | H | H | CH3 | 1 | |
| II-31 | CH3 | CH3 | CF3 | H | CF3 | H | H | CH3 | 0 | |
| II-32 | CH3 | CH3 | CF3 | H | CF3 | H | H | CH3 | 1 | |
| II-33 | CH3 | H | CF3 | H | CF3 | H | Cl | CH3 | 0 | |
| II-34 | CH3 | H | CF3 | H | CF3 | H | Cl | CH3 | 1 | |
| II-35 | CH3 | CH3 | CF3 | H | CF3 | H | Cl | CH3 | 0 | |
| II-36 | CH3 | CH3 | CF3 | H | CF3 | H | Cl | CH3 | 1 | |
| II-37 | CH3 | H | CF3 | H | H | H | CHF2 | CH3 | 0 | |
| II-38 | CH3 | H | CF3 | H | H | H | CHF2 | CH3 | 1 | |
| II-39 | CH3 | CH3 | CF3 | H | H | H | CHF2 | CH3 | 0 | |
| II-40 | CH3 | CH3 | CF3 | H | H | H | CHF2 | CH3 | 1 | |
| II-41 | CH3 | H | CF3 | H | Cl | H | CHF2 | CH3 | 0 | |
| II-42 | CH3 | H | CF3 | H | Cl | H | CHF2 | CH3 | 1 | |
| II-43 | CH3 | CH3 | CF3 | H | Cl | H | CHF2 | CH3 | 0 | |
| II-44 | CH3 | CH3 | CF3 | H | Cl | H | CHF2 | CH3 | 1 | |
| II-45 | CH3 | H | CF3 | H | CHF2 | H | H | CH3 | 0 | |
| II-46 | CH3 | H | CF3 | H | CHF2 | H | H | CH3 | 1 | |
| II-47 | CH3 | CH3 | CF3 | H | CHF2 | H | H | CH3 | 0 | |
| II-48 | CH3 | CH3 | CF3 | H | CHF2 | H | H | CH3 | 1 | |
| II-49 | CH3 | H | CF3 | H | CHF2 | H | Cl | CH3 | 0 | |
| II-50 | CH3 | H | CF3 | H | CHF2 | H | Cl | CH3 | 1 | |
| II-51 | CH3 | CH3 | CF3 | H | CHF2 | H | Cl | CH3 | 0 | |
| II-52 | CH3 | CH3 | CF3 | H | CHF2 | H | Cl | CH3 | 1 | |
| II-53 | CH3 | H | CF3 | H | H | H | CH3 | CH3 | 0 | |
| II-54 | CH3 | H | CF3 | H | H | H | CH3 | CH3 | 1 | |
| II-55 | CH3 | CH3 | CF3 | H | H | H | CH3 | CH3 | 0 | |
| II-56 | CH3 | CH3 | CF3 | H | H | H | CH3 | CH3 | 1 | |
| II-57 | CH3 | H | CF3 | H | Cl | H | CH3 | CH3 | 0 | |
| II-58 | CH3 | H | CF3 | H | Cl | H | CH3 | CH3 | 1 | |
| II-59 | CH3 | CH3 | CF3 | H | Cl | H | CH3 | CH3 | 0 | |
| II-60 | CH3 | CH3 | CF3 | H | Cl | H | CH3 | CH3 | 1 | |
| II-61 | CH3 | H | CF3 | H | CH3 | H | H | CH3 | 0 | |
| II-62 | CH3 | H | CF3 | H | CH3 | H | H | CH3 | 1 | |
| II-63 | CH3 | CH3 | CF3 | H | CH3 | H | H | CH3 | 0 | |
| II-64 | CH3 | CH3 | CF3 | H | CH3 | H | H | CH3 | 1 | |
| II-65 | CH3 | H | CF3 | H | CH3 | H | Cl | CH3 | 0 | |
| II-66 | CH3 | H | CF3 | H | CH3 | H | Cl | CH3 | 1 | |
| II-67 | CH3 | CH3 | CF3 | H | CH3 | H | Cl | CH3 | 0 | |
| II-68 | CH3 | CH3 | CF3 | H | CH3 | H | Cl | CH3 | 1 | |
| II-69 | CH3 | H | CF3 | H | Cl | H | NO2 | CH3 | 0 | |
| II-70 | CH3 | H | CF3 | H | Cl | H | NO2 | CH3 | 1 | |
| II-71 | CH3 | CH3 | CF3 | H | Cl | H | NO2 | CH3 | 0 | |
| II-72 | CH3 | CH3 | CF3 | H | Cl | H | NO2 | CH3 | 1 | |
| II-73 | CH3 | H | CF3 | H | NO2 | H | NO2 | CH3 | 0 | |
| II-74 | CH3 | H | CF3 | H | NO2 | H | NO2 | CH3 | 1 | |
| II-75 | CH3 | CH3 | CF3 | H | NO2 | H | NO2 | CH3 | 0 | |
| II-76 | CH3 | CH3 | CF3 | H | NO2 | H | NO2 | CH3 | 1 | |
| II-77 | CH3 | H | CF3 | H | NO2 | H | CF3 | CH3 | 0 | |
| II-78 | CH3 | H | CF3 | H | NO2 | H | CF3 | CH3 | 1 | |
| II-79 | CH3 | CH3 | CF3 | H | NO2 | H | CF3 | CH3 | 0 | |
| II-80 | CH3 | CH3 | CF3 | H | NO2 | H | CF3 | CH3 | 1 | |
| II-81 | CH3 | H | CF3 | H | Cl | H | Cl | CH3 | 0 | |
| II-82 | CH3 | H | CF3 | H | Cl | H | Cl | CH3 | 1 | |
| II-83 | CH3 | CH3 | CF3 | H | Cl | H | Cl | CH3 | 0 | |
| II-84 | CH3 | CH3 | CF3 | H | Cl | H | Cl | CH3 | 1 | |
| II-85 | CH3 | H | CF3 | H | Br | H | Cl | CH3 | 0 | |
| II-86 | CH3 | H | CF3 | H | Br | H | Cl | CH3 | 1 | |
| II-87 | CH3 | CH3 | CF3 | H | Br | H | Cl | CH3 | 0 | |
| II-88 | CH3 | CH3 | CF3 | H | Br | H | Cl | CH3 | 1 | |
| II-89 | CH3 | H | CF3 | H | I | H | Cl | CH3 | 0 | |
| II-90 | CH3 | H | CF3 | H | I | H | Cl | CH3 | 1 | |
| II-91 | CH3 | CH3 | CF3 | H | I | H | Cl | CH3 | 0 | |
| II-92 | CH3 | CH3 | CF3 | H | I | H | Cl | CH3 | 1 | |
| II-93 | CH3 | H | CF3 | H | Br | H | Br | CH3 | 0 | |
| II-94 | CH3 | H | CF3 | H | Br | H | Br | CH3 | 1 | |
| II-95 | CH3 | CH3 | CF3 | H | Br | H | Br | CH3 | 0 | |
| II-96 | CH3 | CH3 | CF3 | H | Br | H | Br | CH3 | 1 | |
| II-97 | CH3 | H | CF3 | H | H | H | CN | CH2CH3 | 0 | |
| II-98 | CH3 | H | CF3 | H | H | H | CN | CH2CH3 | 1 | |
| II-99 | CH3 | CH3 | CF3 | H | H | H | CN | CH2CH3 | 0 | |
| II-100 | CH3 | CH3 | CF3 | H | H | H | CN | CH2CH3 | 1 | |
| II-101 | CH3 | H | CF3 | H | F | H | CN | CH2CH3 | 0 | |
| II-102 | CH3 | H | CF3 | H | F | H | CN | CH2CH3 | 1 | |
| II-103 | CH3 | CH3 | CF3 | H | F | H | CN | CH2CH3 | 0 | |
| II-104 | CH3 | CH3 | CF3 | H | F | H | CN | CH2CH3 | 1 | |
| II-105 | CH3 | H | CF3 | H | Cl | H | CN | CH2CH3 | 0 | |
| II-106 | CH3 | H | CF3 | H | Cl | H | CN | CH2CH3 | 1 | |
| II-107 | CH3 | CH3 | CF3 | H | Cl | H | CN | CH2CH3 | 0 | |
| II-108 | CH3 | CH3 | CF3 | H | Cl | H | CN | CH2CH3 | 1 | |
| II-109 | CH3 | H | CF3 | H | CN | H | H | CH2CH3 | 0 | |
| II-110 | CH3 | H | CF3 | H | CN | H | H | CH2CH3 | 1 | |
| II-111 | CH3 | CH3 | CF3 | H | CN | H | H | CH2CH3 | 0 | |
| II-112 | CH3 | CH3 | CF3 | H | CN | H | H | CH2CH3 | 1 | |
| II-113 | CH3 | H | CF3 | H | CN | H | Cl | CH2CH3 | 0 | |
| II-114 | CH3 | H | CF3 | H | CN | H | Cl | CH2CH3 | 1 | |
| II-115 | CH3 | CH3 | CF3 | H | CN | H | Cl | CH2CH3 | 0 | |
| II-116 | CH3 | CH3 | CF3 | H | CN | H | Cl | CH2CH3 | 1 | |
| II-117 | CH3 | H | CF3 | H | H | H | CF3 | CH2CH3 | 0 | |
| II-118 | CH3 | H | CF3 | H | H | H | CF3 | CH2CH3 | 1 | |
| II-119 | CH3 | CH3 | CF3 | H | H | H | CF3 | CH2CH3 | 0 | |
| II-120 | CH3 | CH3 | CF3 | H | H | H | CF3 | CH2CH3 | 1 | |
| II-121 | CH3 | H | CF3 | H | Cl | H | CF3 | CH2CH3 | 0 | |
| II-122 | CH3 | H | CF3 | H | Cl | H | CF3 | CH2CH3 | 1 | |
| II-123 | CH3 | CH3 | CF3 | H | Cl | H | CF3 | CH2CH3 | 0 | |
| II-124 | CH3 | CH3 | CF3 | H | Cl | H | CF3 | CH2CH3 | 1 | |
| II-125 | CH3 | H | CF3 | H | CF3 | H | H | CH2CH3 | 0 | |
| II-126 | CH3 | H | CF3 | H | CF3 | H | H | CH2CH3 | 1 | |
| II-127 | CH3 | CH3 | CF3 | H | CF3 | H | H | CH2CH3 | 0 | |
| II-128 | CH3 | CH3 | CF3 | H | CF3 | H | H | CH2CH3 | 1 | |
| II-129 | CH3 | H | CF3 | H | CF3 | H | Cl | CH2CH3 | 0 | |
| II-130 | CH3 | H | CF3 | H | CF3 | H | Cl | CH2CH3 | 1 | |
| II-131 | CH3 | CH3 | CF3 | H | CF3 | H | Cl | CH2CH3 | 0 | |
| II-132 | CH3 | CH3 | CF3 | H | CF3 | H | Cl | CH2CH3 | 1 | |
| II-133 | CH3 | H | CF3 | H | H | H | CHF2 | CH2CH3 | 0 | |
| II-134 | CH3 | H | CF3 | H | H | H | CHF2 | CH2CH3 | 1 | |
| II-135 | CH3 | CH3 | CF3 | H | H | H | CHF2 | CH2CH3 | 0 | |
| II-136 | CH3 | CH3 | CF3 | H | H | H | CHF2 | CH2CH3 | 1 | |
| II-137 | CH3 | H | CF3 | H | Cl | H | CHF2 | CH2CH3 | 0 | |
| II-138 | CH3 | H | CF3 | H | Cl | H | CHF2 | CH2CH3 | 1 | |
| II-139 | CH3 | CH3 | CF3 | H | Cl | H | CHF2 | CH2CH3 | 0 | |
| II-140 | CH3 | CH3 | CF3 | H | Cl | H | CHF2 | CH2CH3 | 1 | |
| II-141 | CH3 | H | CF3 | H | CHF2 | H | H | CH2CH3 | 0 | |
| II-142 | CH3 | H | CF3 | H | CHF2 | H | H | CH2CH3 | 1 | |
| II-143 | CH3 | CH3 | CF3 | H | CHF2 | H | H | CH2CH3 | 0 | |
| II-144 | CH3 | CH3 | CF3 | H | CHF2 | H | H | CH2CH3 | 1 | |
| II-145 | CH3 | H | CF3 | H | CHF2 | H | Cl | CH2CH3 | 0 | |
| II-146 | CH3 | H | CF3 | H | CHF2 | H | Cl | CH2CH3 | 1 | |
| II-147 | CH3 | CH3 | CF3 | H | CHF2 | H | Cl | CH2CH3 | 0 | |
| II-148 | CH3 | CH3 | CF3 | H | CHF2 | H | Cl | CH2CH3 | 1 | |
| II-149 | CH3 | H | CF3 | H | H | H | CH3 | CH2CH3 | 0 | |
| II-150 | CH3 | H | CF3 | H | H | H | CH3 | CH2CH3 | 1 | |
| II-151 | CH3 | CH3 | CF3 | H | H | H | CH3 | CH2CH3 | 0 | |
| II-152 | CH3 | CH3 | CF3 | H | H | H | CH3 | CH2CH3 | 1 | |
| II-153 | CH3 | H | CF3 | H | Cl | H | CH3 | CH2CH3 | 0 | |
| II-154 | CH3 | H | CF3 | H | Cl | H | CH3 | CH2CH3 | 1 | |
| II-155 | CH3 | CH3 | CF3 | H | Cl | H | CH3 | CH2CH3 | 0 | |
| II-156 | CH3 | CH3 | CF3 | H | Cl | H | CH3 | CH2CH3 | 1 | |
| II-157 | CH3 | H | CF3 | H | CH3 | H | H | CH2CH3 | 0 | |
| II-158 | CH3 | H | CF3 | H | CH3 | H | H | CH2CH3 | 1 | |
| II-159 | CH3 | CH3 | CF3 | H | CH3 | H | H | CH2CH3 | 0 | |
| II-160 | CH3 | CH3 | CF3 | H | CH3 | H | H | CH2CH3 | 1 | |
| II-161 | CH3 | H | CF3 | H | CH3 | H | Cl | CH2CH3 | 0 | |
| II-162 | CH3 | H | CF3 | H | CH3 | H | Cl | CH2CH3 | 1 | |
| II-163 | CH3 | CH3 | CF3 | H | CH3 | H | Cl | CH2CH3 | 0 | |
| II-164 | CH3 | CH3 | CF3 | H | CH3 | H | Cl | CH2CH3 | 1 | |
| II-165 | CH3 | H | CF3 | H | Cl | H | NO2 | CH2CH3 | 0 | |
| II-166 | CH3 | H | CF3 | H | Cl | H | NO2 | CH2CH3 | 1 | |
| II-167 | CH3 | CH3 | CF3 | H | Cl | H | NO2 | CH2CH3 | 0 | |
| II-168 | CH3 | CH3 | CF3 | H | Cl | H | NO2 | CH2CH3 | 1 | |
| II-169 | CH3 | H | CF3 | H | NO2 | H | NO2 | CH2CH3 | 0 | |
| II-170 | CH3 | H | CF3 | H | NO2 | H | NO2 | CH2CH3 | 1 | |
| II-171 | CH3 | CH3 | CF3 | H | NO2 | H | NO2 | CH2CH3 | 0 | |
| II-172 | CH3 | CH3 | CF3 | H | NO2 | H | NO2 | CH2CH3 | 1 | |
| II-173 | CH3 | H | CF3 | H | NO2 | H | CF3 | CH2CH3 | 0 | |
| II-174 | CH3 | H | CF3 | H | NO2 | H | CF3 | CH2CH3 | 1 | |
| II-175 | CH3 | CH3 | CF3 | H | NO2 | H | CF3 | CH2CH3 | 0 | |
| II-176 | CH3 | CH3 | CF3 | H | NO2 | H | CF3 | CH2CH3 | 1 | |
| II-177 | CH3 | H | CF3 | H | Cl | H | Cl | CH2CH3 | 0 | |
| II-178 | CH3 | H | CF3 | H | Cl | H | Cl | CH2CH3 | 1 | |
| II-179 | CH3 | CH3 | CF3 | H | Cl | H | Cl | CH2CH3 | 0 | |
| II-180 | CH3 | CH3 | CF3 | H | Cl | H | Cl | CH2CH3 | 1 | |
| II-181 | CH3 | H | CF3 | H | Br | H | Cl | CH2CH3 | 0 | |
| II-182 | CH3 | H | CF3 | H | Br | H | Cl | CH2CH3 | 1 | |
| II-183 | CH3 | CH3 | CF3 | H | Br | H | Cl | CH2CH3 | 0 | |
| II-184 | CH3 | CH3 | CF3 | H | Br | H | Cl | CH2CH3 | 1 | |
| II-185 | CH3 | H | CF3 | H | I | H | Cl | CH2CH3 | 0 | |
| II-186 | CH3 | H | CF3 | H | I | H | Cl | CH2CH3 | 1 | |
| II-187 | CH3 | CH3 | CF3 | H | I | H | Cl | CH2CH3 | 0 | |
| II-188 | CH3 | CH3 | CF3 | H | I | H | Cl | CH2CH3 | 1 | |
| II-189 | CH3 | H | CF3 | H | Br | H | Br | CH2CH3 | 0 | |
| II-190 | CH3 | H | CF3 | H | Br | H | Br | CH2CH3 | 1 | |
| II-191 | CH3 | CH3 | CF3 | H | Br | H | Br | CH2CH3 | 0 | |
| II-192 | CH3 | CH3 | CF3 | H | Br | H | Br | CH2CH3 | 1 | |
| II-193 | CH3 | H | CF3 | H | H | H | CN | CH2CH2CH3 | 0 | |
| II-194 | CH3 | H | CF3 | H | H | H | CN | CH2CH2CH3 | 1 | |
| II-195 | CH3 | CH3 | CF3 | H | H | H | CN | CH2CH2CH3 | 0 | |
| II-196 | CH3 | CH3 | CF3 | H | H | H | CN | CH2CH2CH3 | 1 | |
| II-197 | CH3 | H | CF3 | H | F | H | CN | CH2CH2CH3 | 0 | |
| II-198 | CH3 | H | CF3 | H | F | H | CN | CH2CH2CH3 | 1 | |
| II-199 | CH3 | CH3 | CF3 | H | F | H | CN | CH2CH2CH3 | 0 | 94-90 |
| II-200 | CH3 | CH3 | CF3 | H | F | H | CN | CH2CH2CH3 | 1 | |
| II-201 | CH3 | H | CF3 | H | Cl | H | CN | CH2CH2CH3 | 0 | |
| II-202 | CH3 | H | CF3 | H | Cl | H | CN | CH2CH2CH3 | 1 | |
| II-203 | CH3 | CH3 | CF3 | H | Cl | H | CN | CH2CH2CH3 | 0 | |
| II-204 | CH3 | CH3 | CF3 | H | Cl | H | CN | CH2CH2CH3 | 1 | |
| II-205 | CH3 | H | CF3 | H | CN | H | H | CH2CH2CH3 | 0 | |
| II-206 | CH3 | H | CF3 | H | CN | H | H | CH2CH2CH3 | 1 | |
| II-207 | CH3 | CH3 | CF3 | H | CN | H | H | CH2CH2CH3 | 0 | |
| II-208 | CH3 | CH3 | CF3 | H | CN | H | H | CH2CH2CH3 | 1 | |
| II-209 | CH3 | H | CF3 | H | CN | H | Cl | CH2CH2CH3 | 0 | |
| II-210 | CH3 | H | CF3 | H | CN | H | Cl | CH2CH2CH3 | 1 | |
| II-211 | CH3 | CH3 | CF3 | H | CN | H | Cl | CH2CH2CH3 | 0 | |
| II-212 | CH3 | CH3 | CF3 | H | CN | H | Cl | CH2CH2CH3 | 1 | |
| II-213 | CH3 | H | CF3 | H | H | H | CF3 | CH2CH2CH3 | 0 | |
| II-214 | CH3 | H | CF3 | H | H | H | CF3 | CH2CH2CH3 | 1 | |
| II-215 | CH3 | CH3 | CF3 | H | H | H | CF3 | CH2CH2CH3 | 0 | |
| II-216 | CH3 | CH3 | CF3 | H | H | H | CF3 | CH2CH2CH3 | 1 | |
| II-217 | CH3 | H | CF3 | H | Cl | H | CF3 | CH2CH2CH3 | 0 | |
| II-218 | CH3 | H | CF3 | H | Cl | H | CF3 | CH2CH2CH3 | 1 | |
| II-219 | CH3 | CH3 | CF3 | H | Cl | H | CF3 | CH2CH2CH3 | 0 | |
| II-220 | CH3 | CH3 | CF3 | H | Cl | H | CF3 | CH2CH2CH3 | 1 | |
| II-221 | CH3 | H | CF3 | H | CF3 | H | H | CH2CH2CH3 | 0 | |
| II-222 | CH3 | H | CF3 | H | CF3 | H | H | CH2CH2CH3 | 1 | |
| II-223 | CH3 | CH3 | CF3 | H | CF3 | H | H | CH2CH2CH3 | 0 | |
| II-224 | CH3 | CH3 | CF3 | H | CF3 | H | H | CH2CH2CH3 | 1 | |
| II-225 | CH3 | H | CF3 | H | CF3 | H | Cl | CH2CH2CH3 | 0 | |
| II-226 | CH3 | H | CF3 | H | CF3 | H | Cl | CH2CH2CH3 | 1 | |
| II-227 | CH3 | CH3 | CF3 | H | CF3 | H | Cl | CH2CH2CH3 | 0 | |
| II-228 | CH3 | CH3 | CF3 | H | CF3 | H | Cl | CH2CH2CH3 | 1 | |
| II-229 | CH3 | H | CF3 | H | H | H | CHF2 | CH2CH2CH3 | 0 | |
| II-230 | CH3 | H | CF3 | H | H | H | CHF2 | CH2CH2CH3 | 1 | |
| II-231 | CH3 | CH3 | CF3 | H | H | H | CHF2 | CH2CH2CH3 | 0 | |
| II-232 | CH3 | CH3 | CF3 | H | H | H | CHF2 | CH2CH2CH3 | 1 | |
| II-233 | CH3 | H | CF3 | H | Cl | H | CHF2 | CH2CH2CH3 | 0 | |
| II-234 | CH3 | H | CF3 | H | Cl | H | CHF2 | CH2CH2CH3 | 1 | |
| II-235 | CH3 | CH3 | CF3 | H | Cl | H | CHF2 | CH2CH2CH3 | 0 | |
| II-236 | CH3 | CH3 | CF3 | H | Cl | H | CHF2 | CH2CH2CH3 | 1 | |
| II-237 | CH3 | H | CF3 | H | CHF2 | H | H | CH2CH2CH3 | 0 | |
| II-238 | CH3 | H | CF3 | H | CHF2 | H | H | CH2CH2CH3 | 1 | |
| II-239 | CH3 | CH3 | CF3 | H | CHF2 | H | H | CH2CH2CH3 | 0 | |
| II-240 | CH3 | CH3 | CF3 | H | CHF2 | H | H | CH2CH2CH3 | 1 | |
| II-241 | CH3 | H | CF3 | H | CHF2 | H | Cl | CH2CH2CH3 | 0 | |
| II-242 | CH3 | H | CF3 | H | CHF2 | H | Cl | CH2CH2CH3 | 1 | |
| II-243 | CH3 | CH3 | CF3 | H | CHF2 | H | Cl | CH2CH2CH3 | 0 | |
| II-244 | CH3 | CH3 | CF3 | H | CHF2 | H | Cl | CH2CH2CH3 | 1 | |
| II-245 | CH3 | H | CF3 | H | H | H | CH3 | CH2CH2CH3 | 0 | |
| II-246 | CH3 | H | CF3 | H | H | H | CH3 | CH2CH2CH3 | 1 | |
| II-247 | CH3 | CH3 | CF3 | H | H | H | CH3 | CH2CH2CH3 | 0 | |
| II-248 | CH3 | CH3 | CF3 | H | H | H | CH3 | CH2CH2CH3 | 1 | |
| II-249 | CH3 | H | CF3 | H | Cl | H | CH3 | CH2CH2CH3 | 0 | |
| II-250 | CH3 | H | CF3 | H | Cl | H | CH3 | CH2CH2CH3 | 1 | |
| II-251 | CH3 | CH3 | CF3 | H | Cl | H | CH3 | CH2CH2CH3 | 0 | |
| II-252 | CH3 | CH3 | CF3 | H | Cl | H | CH3 | CH2CH2CH3 | 1 | |
| II-253 | CH3 | H | CF3 | H | CH3 | H | H | CH2CH2CH3 | 0 | |
| II-254 | CH3 | H | CF3 | H | CH3 | H | H | CH2CH2CH3 | 1 | |
| II-255 | CH3 | CH3 | CF3 | H | CH3 | H | H | CH2CH2CH3 | 0 | |
| II-256 | CH3 | CH3 | CF3 | H | CH3 | H | H | CH2CH2CH3 | 1 | |
| II-257 | CH3 | H | CF3 | H | CH3 | H | Cl | CH2CH2CH3 | 0 | |
| II-258 | CH3 | H | CF3 | H | CH3 | H | Cl | CH2CH2CH3 | 1 | |
| II-259 | CH3 | CH3 | CF3 | H | CH3 | H | Cl | CH2CH2CH3 | 0 | |
| II-260 | CH3 | CH3 | CF3 | H | CH3 | H | Cl | CH2CH2CH3 | 1 | |
| II-261 | CH3 | H | CF3 | H | Cl | H | NO2 | CH2CH2CH3 | 0 | |
| II-262 | CH3 | H | CF3 | H | Cl | H | NO2 | CH2CH2CH3 | 1 | |
| II-263 | CH3 | CH3 | CF3 | H | Cl | H | NO2 | CH2CH2CH3 | 0 | |
| II-264 | CH3 | CH3 | CF3 | H | Cl | H | NO2 | CH2CH2CH3 | 1 | |
| II-265 | CH3 | H | CF3 | H | NO2 | H | NO2 | CH2CH2CH3 | 0 | |
| II-266 | CH3 | H | CF3 | H | NO2 | H | NO2 | CH2CH2CH3 | 1 | |
| II-267 | CH3 | CH3 | CF3 | H | NO2 | H | NO2 | CH2CH2CH3 | 0 | |
| II-268 | CH3 | CH3 | CF3 | H | NO2 | H | NO2 | CH2CH2CH3 | 1 | |
| II-269 | CH3 | H | CF3 | H | NO2 | H | CF3 | CH2CH2CH3 | 0 | |
| II-270 | CH3 | H | CF3 | H | NO2 | H | CF3 | CH2CH2CH3 | 1 | |
| II-271 | CH3 | CH3 | CF3 | H | NO2 | H | CF3 | CH2CH2CH3 | 0 | |
| II-272 | CH3 | CH3 | CF3 | H | NO2 | H | CF3 | CH2CH2CH3 | 1 | |
| II-273 | CH3 | H | CF3 | H | Cl | H | Cl | CH2CH2CH3 | 0 | |
| II-274 | CH3 | H | CF3 | H | Cl | H | Cl | CH2CH2CH3 | 1 | |
| II-275 | CH3 | CH3 | CF3 | H | Cl | H | Cl | CH2CH2CH3 | 0 | |
| II-276 | CH3 | CH3 | CF3 | H | Cl | H | Cl | CH2CH2CH3 | 1 | |
| II-277 | CH3 | H | CF3 | H | Br | H | Cl | CH2CH2CH3 | 0 | |
| II-278 | CH3 | H | CF3 | H | Br | H | Cl | CH2CH2CH3 | 1 | |
| II-279 | CH3 | CH3 | CF3 | H | Br | H | Cl | CH2CH2CH3 | 0 | |
| II-280 | CH3 | CH3 | CF3 | H | Br | H | Cl | CH2CH2CH3 | 1 | |
| II-281 | CH3 | H | CF3 | H | I | H | Cl | CH2CH2CH3 | 0 | |
| II-282 | CH3 | H | CF3 | H | I | H | Cl | CH2CH2CH3 | 1 | |
| II-283 | CH3 | CH3 | CF3 | H | I | H | Cl | CH2CH2CH3 | 0 | |
| II-284 | CH3 | CH3 | CF3 | H | I | H | Cl | CH2CH2CH3 | 1 | |
| II-285 | CH3 | H | CF3 | H | Br | H | Br | CH2CH2CH3 | 0 | |
| II-286 | CH3 | H | CF3 | H | Br | H | Br | CH2CH2CH3 | 1 | |
| II-287 | CH3 | CH3 | CF3 | H | Br | H | Br | CH2CH2CH3 | 0 | |
| II-288 | CH3 | CH3 | CF3 | H | Br | H | Br | CH2CH2CH3 | 1 | |
| II-289 | CH3 | H | CF3 | H | H | H | CN | -CH(CH3)2 | 0 | |
| II-290 | CH3 | H | CF3 | H | H | H | CN | -CH(CH3)2 | 1 | |
| II-291 | CH3 | CH3 | CF3 | H | H | H | CN | -CH(CH3)2 | 0 | |
| II-292 | CH3 | CH3 | CF3 | H | H | H | CN | -CH(CH3)2 | 1 | |
| II-293 | CH3 | H | CF3 | H | F | H | CN | -CH(CH3)2 | 0 | |
| II-294 | CH3 | H | CF3 | H | F | H | CN | -CH(CH3)2 | 1 | |
| II-295 | CH3 | CH3 | CF3 | H | F | H | CN | -CH(CH3)2 | 0 | |
| II-296 | CH3 | CH3 | CF3 | H | F | H | CN | -CH(CH3)2 | 1 | |
| II-297 | CH3 | H | CF3 | H | Cl | H | CN | -CH(CH3)2 | 0 | |
| II-298 | CH3 | H | CF3 | H | Cl | H | CN | -CH(CH3)2 | 1 | |
| II-299 | CH3 | CH3 | CF3 | H | Cl | H | CN | -CH(CH3)2 | 0 | |
| II-300 | CH3 | CH3 | CF3 | H | Cl | H | CN | -CH(CH3)2 | 1 | |
| II-301 | CH3 | H | CF3 | H | CN | H | H | -CH(CH3)2 | 0 | |
| II-302 | CH3 | H | CF3 | H | CN | H | H | -CH(CH3)2 | 1 | |
| II-303 | CH3 | CH3 | CF3 | H | CN | H | H | -CH(CH3)2 | 0 | |
| II-304 | CH3 | CH3 | CF3 | H | CN | H | H | -CH(CH3)2 | 1 | |
| II-305 | CH3 | H | CF3 | H | CN | H | Cl | -CH(CH3)2 | 0 | |
| II-306 | CH3 | H | CF3 | H | CN | H | Cl | -CH(CH3)2 | 1 | |
| II-307 | CH3 | CH3 | CF3 | H | CN | H | Cl | -CH(CH3)2 | 0 | |
| II-308 | CH3 | CH3 | CF3 | H | CN | H | Cl | -CH(CH3)2 | 1 | |
| II-309 | CH3 | H | CF3 | H | H | H | CF3 | -CH(CH3)2 | 0 | |
| II-310 | CH3 | H | CF3 | H | H | H | CF3 | -CH(CH3)2 | 1 | |
| II-311 | CH3 | CH3 | CF3 | H | H | H | CF3 | -CH(CH3)2 | 0 | |
| II-312 | CH3 | CH3 | CF3 | H | H | H | CF3 | -CH(CH3)2 | 1 | |
| II-313 | CH3 | H | CF3 | H | Cl | H | CF3 | -CH(CH3)2 | 0 | |
| II-314 | CH3 | H | CF3 | H | Cl | H | CF3 | -CH(CH3)2 | 1 | |
| II-315 | CH3 | CH3 | CF3 | H | Cl | H | CF3 | -CH(CH3)2 | 0 | |
| II-316 | CH3 | CH3 | CF3 | H | Cl | H | CF3 | -CH(CH3)2 | 1 | |
| II-317 | CH3 | H | CF3 | H | CF3 | H | H | -CH(CH3)2 | 0 | |
| II-318 | CH3 | H | CF3 | H | CF3 | H | H | -CH(CH3)2 | 1 | |
| II-319 | CH3 | CH3 | CF3 | H | CF3 | H | H | -CH(CH3)2 | 0 | |
| II-320 | CH3 | CH3 | CF3 | H | CF3 | H | H | -CH(CH3)2 | 1 | |
| II-321 | CH3 | H | CF3 | H | CF3 | H | Cl | -CH(CH3)2 | 0 | |
| II-322 | CH3 | H | CF3 | H | CF3 | H | Cl | -CH(CH3)2 | 1 | |
| II-323 | CH3 | CH3 | CF3 | H | CF3 | H | Cl | -CH(CH3)2 | 0 | |
| II-324 | CH3 | CH3 | CF3 | H | CF3 | H | Cl | -CH(CH3)2 | 1 | |
| II-325 | CH3 | H | CF3 | H | H | H | CHF2 | -CH(CH3)2 | 0 | |
| II-326 | CH3 | H | CF3 | H | H | H | CHF2 | -CH(CH3)2 | 1 | |
| II-327 | CH3 | CH3 | CF3 | H | H | H | CHF2 | -CH(CH3)2 | 0 | |
| II-328 | CH3 | CH3 | CF3 | H | H | H | CHF2 | -CH(CH3)2 | 1 | |
| II-329 | CH3 | H | CF3 | H | Cl | H | CHF2 | -CH(CH3)2 | 0 | |
| II-330 | CH3 | H | CF3 | H | Cl | H | CHF2 | -CH(CH3)2 | 1 | |
| II-331 | CH3 | CH3 | CF3 | H | Cl | H | CHF2 | -CH(CH3)2 | 0 | |
| II-332 | CH3 | CH3 | CF3 | H | Cl | H | CHF2 | -CH(CH3)2 | 1 | |
| II-333 | CH3 | H | CF3 | H | CHF2 | H | H | -CH(CH3)2 | 0 | |
| II-334 | CH3 | H | CF3 | H | CHF2 | H | H | -CH(CH3)2 | 1 | |
| II-335 | CH3 | CH3 | CF3 | H | CHF2 | H | H | -CH(CH3)2 | 0 | |
| II-336 | CH3 | CH3 | CF3 | H | CHF2 | H | H | -CH(CH3)2 | 1 | |
| II-337 | CH3 | H | CF3 | H | CHF2 | H | Cl | -CH(CH3)2 | 0 | |
| II-338 | CH3 | H | CF3 | H | CHF2 | H | Cl | -CH(CH3)2 | 1 | |
| II-339 | CH3 | CH3 | CF3 | H | CHF2 | H | Cl | -CH(CH3)2 | 0 | |
| II-340 | CH3 | CH3 | CF3 | H | CHF2 | H | Cl | -CH(CH3)2 | 1 | |
| II-341 | CH3 | H | CF3 | H | H | H | CH3 | -CH(CH3)2 | 0 | |
| II-342 | CH3 | H | CF3 | H | H | H | CH3 | -CH(CH3)2 | 1 | |
| II-343 | CH3 | CH3 | CF3 | H | H | H | CH3 | -CH(CH3)2 | 0 | |
| II-344 | CH3 | CH3 | CF3 | H | H | H | CH3 | -CH(CH3)2 | 1 | |
| II-345 | CH3 | H | CF3 | H | Cl | H | CH3 | -CH(CH3)2 | 0 | |
| II-346 | CH3 | H | CF3 | H | Cl | H | CH3 | -CH(CH3)2 | 1 | |
| II-347 | CH3 | CH3 | CF3 | H | Cl | H | CH3 | -CH(CH3)2 | 0 | |
| II-348 | CH3 | CH3 | CF3 | H | Cl | H | CH3 | -CH(CH3)2 | 1 | |
| II-349 | CH3 | H | CF3 | H | CH3 | H | H | -CH(CH3)2 | 0 | |
| II-350 | CH3 | H | CF3 | H | CH3 | H | H | -CH(CH3)2 | 1 | |
| II-351 | CH3 | CH3 | CF3 | H | CH3 | H | H | -CH(CH3)2 | 0 | |
| II-352 | CH3 | CH3 | CF3 | H | CH3 | H | H | -CH(CH3)2 | 1 | |
| II-353 | CH3 | H | CF3 | H | CH3 | H | Cl | -CH(CH3)2 | 0 | |
| II-354 | CH3 | H | CF3 | H | CH3 | H | Cl | -CH(CH3)2 | 1 | |
| II-355 | CH3 | CH3 | CF3 | H | CH3 | H | Cl | -CH(CH3)2 | 0 | |
| II-356 | CH3 | CH3 | CF3 | H | CH3 | H | Cl | -CH(CH3)2 | 1 | |
| II-357 | CH3 | H | CF3 | H | Cl | H | NO2 | -CH(CH3)2 | 0 | |
| II-358 | CH3 | H | CF3 | H | Cl | H | NO2 | -CH(CH3)2 | 1 | |
| II-359 | CH3 | CH3 | CF3 | H | Cl | H | NO2 | -CH(CH3)2 | 0 | |
| II-360 | CH3 | CH3 | CF3 | H | Cl | H | NO2 | -CH(CH3)2 | 1 | |
| II-361 | CH3 | H | CF3 | H | NO2 | H | NO2 | -CH(CH3)2 | 0 | |
| II-362 | CH3 | H | CF3 | H | NO2 | H | NO2 | -CH(CH3)2 | 1 | |
| II-363 | CH3 | CH3 | CF3 | H | NO2 | H | NO2 | -CH(CH3)2 | 0 | |
| II-364 | CH3 | CH3 | CF3 | H | NO2 | H | NO2 | -CH(CH3)2 | 1 | |
| II-365 | CH3 | H | CF3 | H | NO2 | H | CF3 | -CH(CH3)2 | 0 | |
| II-366 | CH3 | H | CF3 | H | NO2 | H | CF3 | -CH(CH3)2 | 1 | |
| II-367 | CH3 | CH3 | CF3 | H | NO2 | H | CF3 | -CH(CH3)2 | 0 | |
| II-368 | CH3 | CH3 | CF3 | H | NO2 | H | CF3 | -CH(CH3)2 | 1 | |
| II-369 | CH3 | H | CF3 | H | Cl | H | Cl | -CH(CH3)2 | 0 | |
| II-370 | CH3 | H | CF3 | H | Cl | H | Cl | -CH(CH3)2 | 1 | |
| II-371 | CH3 | CH3 | CF3 | H | Cl | H | Cl | -CH(CH3)2 | 0 | |
| II-372 | CH3 | CH3 | CF3 | H | Cl | H | Cl | -CH(CH3)2 | 1 | |
| II-373 | CH3 | H | CF3 | H | Br | H | Cl | -CH(CH3)2 | 0 | |
| II-374 | CH3 | H | CF3 | H | Br | H | Cl | -CH(CH3)2 | 1 | |
| II-375 | CH3 | CH3 | CF3 | H | Br | H | Cl | -CH(CH3)2 | 0 | |
| II-376 | CH3 | CH3 | CF3 | H | Br | H | Cl | -CH(CH3)2 | 1 | |
| II-377 | CH3 | H | CF3 | H | I | H | Cl | -CH(CH3)2 | 0 | |
| II-378 | CH3 | H | CF3 | H | I | H | Cl | -CH(CH3)2 | 1 | |
| II-379 | CH3 | CH3 | CF3 | H | I | H | Cl | -CH(CH3)2 | 0 | |
| II-380 | CH3 | CH3 | CF3 | H | I | H | Cl | -CH(CH3)2 | 1 | |
| II-381 | CH3 | H | CF3 | H | Br | H | Br | -CH(CH3)2 | 0 | |
| II-382 | CH3 | H | CF3 | H | Br | H | Br | -CH(CH3)2 | 1 | |
| II-383 | CH3 | CH3 | CF3 | H | Br | H | Br | -CH(CH3)2 | 0 | |
| II-384 | CH3 | CH3 | CF3 | H | Br | H | Br | -CH(CH3)2 | 1 | |
| II-385 | CH3 | H | CF3 | H | H | H | CN | -CH2CH2CH2CH3 | 0 | |
| II-386 | CH3 | H | CF3 | H | H | H | CN | -CH2CH2CH2CH3 | 1 | |
| II-387 | CH3 | CH3 | CF3 | H | H | H | CN | -CH2CH2CH2CH3 | 0 | |
| II-388 | CH3 | CH3 | CF3 | H | H | H | CN | -CH2CH2CH2CH3 | 1 | |
| II-389 | CH3 | H | CF3 | H | F | H | CN | -CH2CH2CH2CH3 | 0 | |
| II-390 | CH3 | H | CF3 | H | F | H | CN | -CH2CH2CH2CH3 | 1 | |
| II-391 | CH3 | CH3 | CF3 | H | F | H | CN | -CH2CH2CH2CH3 | 0 | |
| II-392 | CH3 | CH3 | CF3 | H | F | H | CN | -CH2CH2CH2CH3 | 1 | |
| II-393 | CH3 | H | CF3 | H | Cl | H | CN | -CH2CH2CH2CH3 | 0 | |
| II-394 | CH3 | H | CF3 | H | Cl | H | CN | -CH2CH2CH2CH3 | 1 | |
| II-395 | CH3 | CH3 | CF3 | H | Cl | H | CN | -CH2CH2CH2CH3 | 0 | |
| II-396 | CH3 | CH3 | CF3 | H | Cl | H | CN | -CH2CH2CH2CH3 | 1 | |
| II-397 | CH3 | H | CF3 | H | CN | H | H | -CH2CH2CH2CH3 | 0 | |
| II-398 | CH3 | H | CF3 | H | CN | H | H | -CH2CH2CH2CH3 | 1 | |
| II-399 | CH3 | CH3 | CF3 | H | CN | H | H | -CH2CH2CH2CH3 | 0 | |
| II-400 | CH3 | CH3 | CF3 | H | CN | H | H | -CH2CH2CH2CH3 | 1 | |
| II-401 | CH3 | H | CF3 | H | CN | H | Cl | -CH2CH2CH2CH3 | 0 | |
| II-402 | CH3 | H | CF3 | H | CN | H | Cl | -CH2CH2CH2CH3 | 1 | |
| II-403 | CH3 | CH3 | CF3 | H | CN | H | Cl | -CH2CH2CH2CH3 | 0 | |
| II-404 | CH3 | CH3 | CF3 | H | CN | H | Cl | -CH2CH2CH2CH3 | 1 | |
| II-405 | CH3 | H | CF3 | H | H | H | CF3 | -CH2CH2CH2CH3 | 0 | |
| II-406 | CH3 | H | CF3 | H | H | H | CF3 | -CH2CH2CH2CH3 | 1 | |
| II-407 | CH3 | CH3 | CF3 | H | H | H | CF3 | -CH2CH2CH2CH3 | 0 | |
| II-408 | CH3 | CH3 | CF3 | H | H | H | CF3 | -CH2CH2CH2CH3 | 1 | |
| II-409 | CH3 | H | CF3 | H | Cl | H | CF3 | -CH2CH2CH2CH3 | 0 | |
| II-410 | CH3 | H | CF3 | H | Cl | H | CF3 | -CH2CH2CH2CH3 | 1 | |
| II-411 | CH3 | CH3 | CF3 | H | Cl | H | CF3 | -CH2CH2CH2CH3 | 0 | |
| II-412 | CH3 | CH3 | CF3 | H | Cl | H | CF3 | -CH2CH2CH2CH3 | 1 | |
| II-413 | CH3 | H | CF3 | H | CF3 | H | H | -CH2CH2CH2CH3 | 0 | |
| II-414 | CH3 | H | CF3 | H | CF3 | H | H | -CH2CH2CH2CH3 | 1 | |
| II-415 | CH3 | CH3 | CF3 | H | CF3 | H | H | -CH2CH2CH2CH3 | 0 | |
| II-416 | CH3 | CH3 | CF3 | H | CF3 | H | H | -CH2CH2CH2CH3 | 1 | |
| II-417 | CH3 | H | CF3 | H | CF3 | H | Cl | -CH2CH2CH2CH3 | 0 | |
| II-418 | CH3 | H | CF3 | H | CF3 | H | Cl | -CH2CH2CH2CH3 | 1 | |
| II-419 | CH3 | CH3 | CF3 | H | CF3 | H | Cl | -CH2CH2CH2CH3 | 0 | |
| II-420 | CH3 | CH3 | CF3 | H | CF3 | H | Cl | -CH2CH2CH2CH3 | 1 | |
| II-421 | CH3 | H | CF3 | H | H | H | CHF2 | -CH2CH2CH2CH3 | 0 | |
| II-422 | CH3 | H | CF3 | H | H | H | CHF2 | -CH2CH2CH2CH3 | 1 | |
| II-423 | CH3 | CH3 | CF3 | H | H | H | CHF2 | -CH2CH2CH2CH3 | 0 | |
| II-424 | CH3 | CH3 | CF3 | H | H | H | CHF2 | -CH2CH2CH2CH3 | 1 | |
| II-425 | CH3 | H | CF3 | H | Cl | H | CHF2 | -CH2CH2CH2CH3 | 0 | |
| II-426 | CH3 | H | CF3 | H | Cl | H | CHF2 | -CH2CH2CH2CH3 | 1 | |
| II-427 | CH3 | CH3 | CF3 | H | Cl | H | CHF2 | -CH2CH2CH2CH3 | 0 | |
| II-428 | CH3 | CH3 | CF3 | H | Cl | H | CHF2 | -CH2CH2CH2CH3 | 1 | |
| II-429 | CH3 | H | CF3 | H | CHF2 | H | H | -CH2CH2CH2CH3 | 0 | |
| II-430 | CH3 | H | CF3 | H | CHF2 | H | H | -CH2CH2CH2CH3 | 1 | |
| II-431 | CH3 | CH3 | CF3 | H | CHF2 | H | H | -CH2CH2CH2CH3 | 0 | |
| II-432 | CH3 | CH3 | CF3 | H | CHF2 | H | H | -CH2CH2CH2CH3 | 1 | |
| II-433 | CH3 | H | CF3 | H | CHF2 | H | Cl | -CH2CH2CH2CH3 | 0 | |
| II-434 | CH3 | H | CF3 | H | CHF2 | H | Cl | -CH2CH2CH2CH3 | 1 | |
| II-435 | CH3 | CH3 | CF3 | H | CHF2 | H | Cl | -CH2CH2CH2CH3 | 0 | |
| II-436 | CH3 | CH3 | CF3 | H | CHF2 | H | Cl | -CH2CH2CH2CH3 | 1 | |
| II-437 | CH3 | H | CF3 | H | H | H | CH3 | -CH2CH2CH2CH3 | 0 | |
| II-438 | CH3 | H | CF3 | H | H | H | CH3 | -CH2CH2CH2CH3 | 1 | |
| II-439 | CH3 | CH3 | CF3 | H | H | H | CH3 | -CH2CH2CH2CH3 | 0 | |
| II-440 | CH3 | CH3 | CF3 | H | H | H | CH3 | -CH2CH2CH2CH3 | 1 | |
| II-441 | CH3 | H | CF3 | H | Cl | H | CH3 | -CH2CH2CH2CH3 | 0 | |
| II-442 | CH3 | H | CF3 | H | Cl | H | CH3 | -CH2CH2CH2CH3 | 1 | |
| II-443 | CH3 | CH3 | CF3 | H | Cl | H | CH3 | -CH2CH2CH2CH3 | 0 | |
| II-444 | CH3 | CH3 | CF3 | H | Cl | H | CH3 | -CH2CH2CH2CH3 | 1 | |
| II-445 | CH3 | H | CF3 | H | CH3 | H | H | -CH2CH2CH2CH3 | 0 | |
| II-446 | CH3 | H | CF3 | H | CH3 | H | H | -CH2CH2CH2CH3 | 1 | |
| II-447 | CH3 | CH3 | CF3 | H | CH3 | H | H | -CH2CH2CH2CH3 | 0 | |
| II-448 | CH3 | CH3 | CF3 | H | CH3 | H | H | -CH2CH2CH2CH3 | 1 | |
| II-449 | CH3 | H | CF3 | H | CH3 | H | Cl | -CH2CH2CH2CH3 | 0 | |
| II-450 | CH3 | H | CF3 | H | CH3 | H | Cl | -CH2CH2CH2CH3 | 1 | |
| II-451 | CH3 | CH3 | CF3 | H | CH3 | H | Cl | -CH2CH2CH2CH3 | 0 | |
| II-452 | CH3 | CH3 | CF3 | H | CH3 | H | Cl | -CH2CH2CH2CH3 | 1 | |
| II-453 | CH3 | H | CF3 | H | Cl | H | NO2 | -CH2CH2CH2CH3 | 0 | |
| II-454 | CH3 | H | CF3 | H | Cl | H | NO2 | -CH2CH2CH2CH3 | 1 | |
| II-455 | CH3 | CH3 | CF3 | H | Cl | H | NO2 | -CH2CH2CH2CH3 | 0 | |
| II-456 | CH3 | CH3 | CF3 | H | Cl | H | NO2 | -CH2CH2CH2CH3 | 1 | |
| II-457 | CH3 | H | CF3 | H | NO2 | H | NO2 | -CH2CH2CH2CH3 | 0 | |
| II-458 | CH3 | H | CF3 | H | NO2 | H | NO2 | -CH2CH2CH2CH3 | 1 | |
| II-459 | CH3 | CH3 | CF3 | H | NO2 | H | NO2 | -CH2CH2CH2CH3 | 0 | |
| II-460 | CH3 | CH3 | CF3 | H | NO2 | H | NO2 | -CH2CH2CH2CH3 | 1 | |
| II-461 | CH3 | H | CF3 | H | NO2 | H | CF3 | -CH2CH2CH2CH3 | 0 | |
| II-462 | CH3 | H | CF3 | H | NO2 | H | CF3 | -CH2CH2CH2CH3 | 1 | |
| II-463 | CH3 | CH3 | CF3 | H | NO2 | H | CF3 | -CH2CH2CH2CH3 | 0 | |
| II-464 | CH3 | CH3 | CF3 | H | NO2 | H | CF3 | -CH2CH2CH2CH3 | 1 | |
| II-465 | CH3 | H | CF3 | H | Cl | H | Cl | -CH2CH2CH2CH3 | 0 | |
| II-466 | CH3 | H | CF3 | H | Cl | H | Cl | -CH2CH2CH2CH3 | 1 | |
| II-467 | CH3 | CH3 | CF3 | H | Cl | H | Cl | -CH2CH2CH2CH3 | 0 | |
| II-468 | CH3 | CH3 | CF3 | H | Cl | H | Cl | -CH2CH2CH2CH3 | 1 | |
| II-469 | CH3 | H | CF3 | H | Br | H | Cl | -CH2CH2CH2CH3 | 0 | |
| II-470 | CH3 | H | CF3 | H | Br | H | Cl | -CH2CH2CH2CH3 | 1 | |
| II-471 | CH3 | CH3 | CF3 | H | Br | H | Cl | -CH2CH2CH2CH3 | 0 | |
| II-472 | CH3 | CH3 | CF3 | H | Br | H | Cl | -CH2CH2CH2CH3 | 1 | |
| II-473 | CH3 | H | CF3 | H | I | H | Cl | -CH2CH2CH2CH3 | 0 | |
| II-474 | CH3 | H | CF3 | H | I | H | Cl | -CH2CH2CH2CH3 | 1 | |
| II-475 | CH3 | CH3 | CF3 | H | I | H | Cl | -CH2CH2CH2CH3 | 0 | |
| II-476 | CH3 | CH3 | CF3 | H | I | H | Cl | -CH2CH2CH2CH3 | 1 | |
| II-477 | CH3 | H | CF3 | H | Br | H | Br | -CH2CH2CH2CH3 | 0 | |
| II-478 | CH3 | H | CF3 | H | Br | H | Br | -CH2CH2CH2CH3 | 1 | |
| II-479 | CH3 | CH3 | CF3 | H | Br | H | Br | -CH2CH2CH2CH3 | 0 | |
| II-480 | CH3 | CH3 | CF3 | H | Br | H | Br | -CH2CH2CH2CH3 | 1 | |
| II-481 | CH3 | H | CF3 | H | H | H | CN | -CH2CH(CH3)2 | 0 | |
| II-482 | CH3 | H | CF3 | H | H | H | CN | -CH2CH(CH3)2 | 1 | |
| II-483 | CH3 | CH3 | CF3 | H | H | H | CN | -CH2CH(CH3)2 | 0 | |
| II-484 | CH3 | CH3 | CF3 | H | H | H | CN | -CH2CH(CH3)2 | 1 | |
| II-485 | CH3 | H | CF3 | H | F | H | CN | -CH2CH(CH3)2 | 0 | |
| II-486 | CH3 | H | CF3 | H | F | H | CN | -CH2CH(CH3)2 | 1 | |
| II-487 | CH3 | CH3 | CF3 | H | F | H | CN | -CH2CH(CH3)2 | 0 | |
| II-488 | CH3 | CH3 | CF3 | H | F | H | CN | -CH2CH(CH3)2 | 1 | |
| II-489 | CH3 | H | CF3 | H | Cl | H | CN | -CH2CH(CH3)2 | 0 | |
| II-490 | CH3 | H | CF3 | H | Cl | H | CN | -CH2CH(CH3)2 | 1 | |
| II-491 | CH3 | CH3 | CF3 | H | Cl | H | CN | -CH2CH(CH3)2 | 0 | |
| II-492 | CH3 | CH3 | CF3 | H | Cl | H | CN | -CH2CH(CH3)2 | 1 | |
| II-493 | CH3 | H | CF3 | H | CN | H | H | -CH2CH(CH3)2 | 0 | |
| II-494 | CH3 | H | CF3 | H | CN | H | H | -CH2CH(CH3)2 | 1 | |
| II-495 | CH3 | CH3 | CF3 | H | CN | H | H | -CH2CH(CH3)2 | 0 | |
| II-496 | CH3 | CH3 | CF3 | H | CN | H | H | -CH2CH(CH3)2 | 1 | |
| II-497 | CH3 | H | CF3 | H | CN | H | Cl | -CH2CH(CH3)2 | 0 | |
| II-498 | CH3 | H | CF3 | H | CN | H | Cl | -CH2CH(CH3)2 | 1 | |
| II-499 | CH3 | CH3 | CF3 | H | CN | H | Cl | -CH2CH(CH3)2 | 0 | |
| II-500 | CH3 | CH3 | CF3 | H | CN | H | Cl | -CH2CH(CH3)2 | 1 | |
| II-501 | CH3 | H | CF3 | H | H | H | CF3 | -CH2CH(CH3)2 | 0 | |
| II-502 | CH3 | H | CF3 | H | H | H | CF3 | -CH2CH(CH3)2 | 1 | |
| II-503 | CH3 | CH3 | CF3 | H | H | H | CF3 | -CH2CH(CH3)2 | 0 | |
| II-504 | CH3 | CH3 | CF3 | H | H | H | CF3 | -CH2CH(CH3)2 | 1 | |
| II-505 | CH3 | H | CF3 | H | Cl | H | CF3 | -CH2CH(CH3)2 | 0 | |
| II-506 | CH3 | H | CF3 | H | Cl | H | CF3 | -CH2CH(CH3)2 | 1 | |
| II-507 | CH3 | CH3 | CF3 | H | Cl | H | CF3 | -CH2CH(CH3)2 | 0 | |
| II-508 | CH3 | CH3 | CF3 | H | Cl | H | CF3 | -CH2CH(CH3)2 | 1 | |
| II-509 | CH3 | H | CF3 | H | CF3 | H | H | -CH2CH(CH3)2 | 0 | |
| II-510 | CH3 | H | CF3 | H | CF3 | H | H | -CH2CH(CH3)2 | 1 | |
| II-511 | CH3 | CH3 | CF3 | H | CF3 | H | H | -CH2CH(CH3)2 | 0 | |
| II-512 | CH3 | CH3 | CF3 | H | CF3 | H | H | -CH2CH(CH3)2 | 1 | |
| II-513 | CH3 | H | CF3 | H | CF3 | H | Cl | -CH2CH(CH3)2 | 0 | |
| II-514 | CH3 | H | CF3 | H | CF3 | H | Cl | -CH2CH(CH3)2 | 1 | |
| II-515 | CH3 | CH3 | CF3 | H | CF3 | H | Cl | -CH2CH(CH3)2 | 0 | |
| II-516 | CH3 | CH3 | CF3 | H | CF3 | H | Cl | -CH2CH(CH3)2 | 1 | |
| II-517 | CH3 | H | CF3 | H | H | H | CHF2 | -CH2CH(CH3)2 | 0 | |
| II-518 | CH3 | H | CF3 | H | H | H | CHF2 | -CH2CH(CH3)2 | 1 | |
| II-519 | CH3 | CH3 | CF3 | H | H | H | CHF2 | -CH2CH(CH3)2 | 0 | |
| II-520 | CH3 | CH3 | CF3 | H | H | H | CHF2 | -CH2CH(CH3)2 | 1 | |
| II-521 | CH3 | H | CF3 | H | Cl | H | CHF2 | -CH2CH(CH3)2 | 0 | |
| II-522 | CH3 | H | CF3 | H | Cl | H | CHF2 | -CH2CH(CH3)2 | 1 | |
| II-523 | CH3 | CH3 | CF3 | H | Cl | H | CHF2 | -CH2CH(CH3)2 | 0 | |
| II-524 | CH3 | CH3 | CF3 | H | Cl | H | CHF2 | -CH2CH(CH3)2 | 1 | |
| II-525 | CH3 | H | CF3 | H | CHF2 | H | H | -CH2CH(CH3)2 | 0 | |
| II-526 | CH3 | H | CF3 | H | CHF2 | H | H | -CH2CH(CH3)2 | 1 | |
| II-527 | CH3 | CH3 | CF3 | H | CHF2 | H | H | -CH2CH(CH3)2 | 0 | |
| II-528 | CH3 | CH3 | CF3 | H | CHF2 | H | H | -CH2CH(CH3)2 | 1 | |
| II-529 | CH3 | H | CF3 | H | CHF2 | H | Cl | -CH2CH(CH3)2 | 0 | |
| II-530 | CH3 | H | CF3 | H | CHF2 | H | Cl | -CH2CH(CH3)2 | 1 | |
| II-531 | CH3 | CH3 | CF3 | H | CHF2 | H | Cl | -CH2CH(CH3)2 | 0 | |
| II-532 | CH3 | CH3 | CF3 | H | CHF2 | H | Cl | -CH2CH(CH3)2 | 1 | |
| II-533 | CH3 | H | CF3 | H | H | H | CH3 | -CH2CH(CH3)2 | 0 | |
| II-534 | CH3 | H | CF3 | H | H | H | CH3 | -CH2CH(CH3)2 | 1 | |
| II-535 | CH3 | CH3 | CF3 | H | H | H | CH3 | -CH2CH(CH3)2 | 0 | |
| II-536 | CH3 | CH3 | CF3 | H | H | H | CH3 | -CH2CH(CH3)2 | 1 | |
| II-537 | CH3 | H | CF3 | H | Cl | H | CH3 | -CH2CH(CH3)2 | 0 | |
| II-538 | CH3 | H | CF3 | H | Cl | H | CH3 | -CH2CH(CH3)2 | 1 | |
| II-539 | CH3 | CH3 | CF3 | H | Cl | H | CH3 | -CH2CH(CH3)2 | 0 | |
| II-540 | CH3 | CH3 | CF3 | H | Cl | H | CH3 | -CH2CH(CH3)2 | 1 | |
| II-541 | CH3 | H | CF3 | H | CH3 | H | H | -CH2CH(CH3)2 | 0 | |
| II-542 | CH3 | H | CF3 | H | CH3 | H | H | -CH2CH(CH3)2 | 1 | |
| II-543 | CH3 | CH3 | CF3 | H | CH3 | H | H | -CH2CH(CH3)2 | 0 | |
| II-544 | CH3 | CH3 | CF3 | H | CH3 | H | H | -CH2CH(CH3)2 | 1 | |
| II-545 | CH3 | H | CF3 | H | CH3 | H | Cl | -CH2CH(CH3)2 | 0 | |
| II-546 | CH3 | H | CF3 | H | CH3 | H | Cl | -CH2CH(CH3)2 | 1 | |
| II-547 | CH3 | CH3 | CF3 | H | CH3 | H | Cl | -CH2CH(CH3)2 | 0 | |
| II-548 | CH3 | CH3 | CF3 | H | CH3 | H | Cl | -CH2CH(CH3)2 | 1 | |
| II-549 | CH3 | H | CF3 | H | Cl | H | NO2 | -CH2CH(CH3)2 | 0 | |
| II-550 | CH3 | H | CF3 | H | Cl | H | NO2 | -CH2CH(CH3)2 | 1 | |
| II-551 | CH3 | CH3 | CF3 | H | Cl | H | NO2 | -CH2CH(CH3)2 | 0 | |
| II-552 | CH3 | CH3 | CF3 | H | Cl | H | NO2 | -CH2CH(CH3)2 | 1 | |
| II-553 | CH3 | H | CF3 | H | NO2 | H | NO2 | -CH2CH(CH3)2 | 0 | |
| II-554 | CH3 | H | CF3 | H | NO2 | H | NO2 | -CH2CH(CH3)2 | 1 | |
| II-555 | CH3 | CH3 | CF3 | H | NO2 | H | NO2 | -CH2CH(CH3)2 | 0 | |
| II-556 | CH3 | CH3 | CF3 | H | NO2 | H | NO2 | -CH2CH(CH3)2 | 1 | |
| II-557 | CH3 | H | CF3 | H | NO2 | H | CF3 | -CH2CH(CH3)2 | 0 | |
| II-558 | CH3 | H | CF3 | H | NO2 | H | CF3 | -CH2CH(CH3)2 | 1 | |
| II-559 | CH3 | CH3 | CF3 | H | NO2 | H | CF3 | -CH2CH(CH3)2 | 0 | |
| II-560 | CH3 | CH3 | CF3 | H | NO2 | H | CF3 | -CH2CH(CH3)2 | 1 | |
| II-561 | CH3 | H | CF3 | H | Cl | H | Cl | -CH2CH(CH3)2 | 0 | |
| II-562 | CH3 | H | CF3 | H | Cl | H | Cl | -CH2CH(CH3)2 | 1 | |
| II-563 | CH3 | CH3 | CF3 | H | Cl | H | Cl | -CH2CH(CH3)2 | 0 | |
| II-564 | CH3 | CH3 | CF3 | H | Cl | H | Cl | -CH2CH(CH3)2 | 1 | |
| II-565 | CH3 | H | CF3 | H | Br | H | Cl | -CH2CH(CH3)2 | 0 | |
| II-566 | CH3 | H | CF3 | H | Br | H | Cl | -CH2CH(CH3)2 | 1 | |
| II-567 | CH3 | CH3 | CF3 | H | Br | H | Cl | -CH2CH(CH3)2 | 0 | |
| II-568 | CH3 | CH3 | CF3 | H | Br | H | Cl | -CH2CH(CH3)2 | 1 | |
| II-569 | CH3 | H | CF3 | H | I | H | Cl | -CH2CH(CH3)2 | 0 | |
| II-570 | CH3 | H | CF3 | H | I | H | Cl | -CH2CH(CH3)2 | 1 | |
| II-571 | CH3 | CH3 | CF3 | H | I | H | Cl | -CH2CH(CH3)2 | 0 | |
| II-572 | CH3 | CH3 | CF3 | H | I | H | Cl | -CH2CH(CH3)2 | 1 | |
| II-573 | CH3 | H | CF3 | H | Br | H | Br | -CH2CH(CH3)2 | 0 | |
| II-574 | CH3 | H | CF3 | H | Br | H | Br | -CH2CH(CH3)2 | 1 | |
| II-575 | CH3 | CH3 | CF3 | H | Br | H | Br | -CH2CH(CH3)2 | 0 | |
| II-576 | CH3 | CH3 | CF3 | H | Br | H | Br | -CH2CH(CH3)2 | 1 | |
| II-577 | CH3 | H | CF3 | H | H | H | CN | -CH2-cyc-C3H5 | 0 | |
| II-578 | CH3 | H | CF3 | H | H | H | CN | -CH2-cyc-C3H5 | 1 | |
| II-579 | CH3 | CH3 | CF3 | H | H | H | CN | -CH2-cyc-C3H5 | 0 | |
| II-580 | CH3 | CH3 | CF3 | H | H | H | CN | -CH2-cyc-C3H5 | 1 | |
| II-581 | CH3 | H | CF3 | H | F | H | CN | -CH2-cyc-C3H5 | 0 | |
| II-582 | CH3 | H | CF3 | H | F | H | CN | -CH2-cyc-C3H5 | 1 | |
| II-583 | CH3 | CH3 | CF3 | H | F | H | CN | -CH2-cyc-C3H5 | 0 | |
| II-584 | CH3 | CH3 | CF3 | H | F | H | CN | -CH2-cyc-C3H5 | 1 | |
| II-585 | CH3 | H | CF3 | H | Cl | H | CN | -CH2-cyc-C3H5 | 0 | |
| II-586 | CH3 | H | CF3 | H | Cl | H | CN | -CH2-cyc-C3H5 | 1 | |
| II-587 | CH3 | CH3 | CF3 | H | Cl | H | CN | -CH2-cyc-C3H5 | 0 | |
| II-588 | CH3 | CH3 | CF3 | H | Cl | H | CN | -CH2-cyc-C3H5 | 1 | |
| II-589 | CH3 | H | CF3 | H | CN | H | H | -CH2-cyc-C3H5 | 0 | |
| II-590 | CH3 | H | CF3 | H | CN | H | H | -CH2-cyc-C3H5 | 1 | |
| II-591 | CH3 | CH3 | CF3 | H | CN | H | H | -CH2-cyc-C3H5 | 0 | |
| II-592 | CH3 | CH3 | CF3 | H | CN | H | H | -CH2-cyc-C3H5 | 1 | |
| II-593 | CH3 | H | CF3 | H | CN | H | Cl | -CH2-cyc-C3H5 | 0 | |
| II-594 | CH3 | H | CF3 | H | CN | H | Cl | -CH2-cyc-C3H5 | 1 | |
| II-595 | CH3 | CH3 | CF3 | H | CN | H | Cl | -CH2-cyc-C3H5 | 0 | |
| II-596 | CH3 | CH3 | CF3 | H | CN | H | Cl | -CH2-cyc-C3H5 | 1 | |
| II-597 | CH3 | H | CF3 | H | H | H | CF3 | -CH2-cyc-C3H5 | 0 | |
| II-598 | CH3 | H | CF3 | H | H | H | CF3 | -CH2-cyc-C3H5 | 1 | |
| II-599 | CH3 | CH3 | CF3 | H | H | H | CF3 | -CH2-cyc-C3H5 | 0 | |
| II-600 | CH3 | CH3 | CF3 | H | H | H | CF3 | -CH2-cyc-C3H5 | 1 | |
| II-601 | CH3 | H | CF3 | H | Cl | H | CF3 | -CH2-cyc-C3H5 | 0 | |
| II-602 | CH3 | H | CF3 | H | Cl | H | CF3 | -CH2-cyc-C3H5 | 1 | |
| II-603 | CH3 | CH3 | CF3 | H | Cl | H | CF3 | -CH2-cyc-C3H5 | 0 | |
| II-604 | CH3 | CH3 | CF3 | H | Cl | H | CF3 | -CH2-cyc-C3H5 | 1 | |
| II-605 | CH3 | H | CF3 | H | CF3 | H | H | -CH2-cyc-C3H5 | 0 | |
| II-606 | CH3 | H | CF3 | H | CF3 | H | H | -CH2-cyc-C3H5 | 1 | |
| II-607 | CH3 | CH3 | CF3 | H | CF3 | H | H | -CH2-cyc-C3H5 | 0 | |
| II-608 | CH3 | CH3 | CF3 | H | CF3 | H | H | -CH2-cyc-C3H5 | 1 | |
| II-609 | CH3 | H | CF3 | H | CF3 | H | Cl | -CH2-cyc-C3H5 | 0 | |
| II-610 | CH3 | H | CF3 | H | CF3 | H | Cl | -CH2-cyc-C3H5 | 1 | |
| II-611 | CH3 | CH3 | CF3 | H | CF3 | H | Cl | -CH2-cyc-C3H5 | 0 | |
| II-612 | CH3 | CH3 | CF3 | H | CF3 | H | Cl | -CH2-cyc-C3H5 | 1 | |
| II-613 | CH3 | H | CF3 | H | H | H | CHF2 | -CH2-cyc-C3H5 | 0 | |
| II-614 | CH3 | H | CF3 | H | H | H | CHF2 | -CH2-cyc-C3H5 | 1 | |
| II-615 | CH3 | CH3 | CF3 | H | H | H | CHF2 | -CH2-cyc-C3H5 | 0 | |
| II-616 | CH3 | CH3 | CF3 | H | H | H | CHF2 | -CH2-cyc-C3H5 | 1 | |
| II-617 | CH3 | H | CF3 | H | Cl | H | CHF2 | -CH2-cyc-C3H5 | 0 | |
| II-618 | CH3 | H | CF3 | H | Cl | H | CHF2 | -CH2-cyc-C3H5 | 1 | |
| II-619 | CH3 | CH3 | CF3 | H | Cl | H | CHF2 | -CH2-cyc-C3H5 | 0 | |
| II-620 | CH3 | CH3 | CF3 | H | Cl | H | CHF2 | -CH2-cyc-C3H5 | 1 | |
| II-621 | CH3 | H | CF3 | H | CHF2 | H | H | -CH2-cyc-C3H5 | 0 | |
| II-622 | CH3 | H | CF3 | H | CHF2 | H | H | -CH2-cyc-C3H5 | 1 | |
| II-623 | CH3 | CH3 | CF3 | H | CHF2 | H | H | -CH2-cyc-C3H5 | 0 | |
| II-624 | CH3 | CH3 | CF3 | H | CHF2 | H | H | -CH2-cyc-C3H5 | 1 | |
| II-625 | CH3 | H | CF3 | H | CHF2 | H | Cl | -CH2-cyc-C3H5 | 0 | |
| II-626 | CH3 | H | CF3 | H | CHF2 | H | Cl | -CH2-cyc-C3H5 | 1 | |
| II-627 | CH3 | CH3 | CF3 | H | CHF2 | H | Cl | -CH2-cyc-C3H5 | 0 | |
| II-628 | CH3 | CH3 | CF3 | H | CHF2 | H | Cl | -CH2-cyc-C3H5 | 1 | |
| II-629 | CH3 | H | CF3 | H | H | H | CH3 | -CH2-cyc-C3H5 | 0 | |
| II-630 | CH3 | H | CF3 | H | H | H | CH3 | -CH2-cyc-C3H5 | 1 | |
| II-631 | CH3 | CH3 | CF3 | H | H | H | CH3 | -CH2-cyc-C3H5 | 0 | |
| II-632 | CH3 | CH3 | CF3 | H | H | H | CH3 | -CH2-cyc-C3H5 | 1 | |
| II-633 | CH3 | H | CF3 | H | Cl | H | CH3 | -CH2-cyc-C3H5 | 0 | |
| II-634 | CH3 | H | CF3 | H | Cl | H | CH3 | -CH2-cyc-C3H5 | 1 | |
| II-635 | CH3 | CH3 | CF3 | H | Cl | H | CH3 | -CH2-cyc-C3H5 | 0 | |
| II-636 | CH3 | CH3 | CF3 | H | Cl | H | CH3 | -CH2-cyc-C3H5 | 1 | |
| II-637 | CH3 | H | CF3 | H | CH3 | H | H | -CH2-cyc-C3H5 | 0 | |
| II-638 | CH3 | H | CF3 | H | CH3 | H | H | -CH2-cyc-C3H5 | 1 | |
| II-639 | CH3 | CH3 | CF3 | H | CH3 | H | H | -CH2-cyc-C3H5 | 0 | |
| II-640 | CH3 | CH3 | CF3 | H | CH3 | H | H | -CH2-cyc-C3H5 | 1 | |
| II-641 | CH3 | H | CF3 | H | CH3 | H | Cl | -CH2-cyc-C3H5 | 0 | |
| II-642 | CH3 | H | CF3 | H | CH3 | H | Cl | -CH2-cyc-C3H5 | 1 | |
| II-643 | CH3 | CH3 | CF3 | H | CH3 | H | Cl | -CH2-cyc-C3H5 | 0 | |
| II-644 | CH3 | CH3 | CF3 | H | CH3 | H | Cl | -CH2-cyc-C3H5 | 1 | |
| II-645 | CH3 | H | CF3 | H | Cl | H | NO2 | -CH2-cyc-C3H5 | 0 | |
| II-646 | CH3 | H | CF3 | H | Cl | H | NO2 | -CH2-cyc-C3H5 | 1 | |
| II-647 | CH3 | CH3 | CF3 | H | Cl | H | NO2 | -CH2-cyc-C3H5 | 0 | |
| II-648 | CH3 | CH3 | CF3 | H | Cl | H | NO2 | -CH2-cyc-C3H5 | 1 | |
| II-649 | CH3 | H | CF3 | H | NO2 | H | NO2 | -CH2-cyc-C3H5 | 0 | |
| II-650 | CH3 | H | CF3 | H | NO2 | H | NO2 | -CH2-cyc-C3H5 | 1 | |
| II-651 | CH3 | CH3 | CF3 | H | NO2 | H | NO2 | -CH2-cyc-C3H5 | 0 | |
| II-652 | CH3 | CH3 | CF3 | H | NO2 | H | NO2 | -CH2-cyc-C3H5 | 1 | |
| II-653 | CH3 | H | CF3 | H | NO2 | H | CF3 | -CH2-cyc-C3H5 | 0 | |
| II-654 | CH3 | H | CF3 | H | NO2 | H | CF3 | -CH2-cyc-C3H5 | 1 | |
| II-655 | CH3 | CH3 | CF3 | H | NO2 | H | CF3 | -CH2-cyc-C3H5 | 0 | |
| II-656 | CH3 | CH3 | CF3 | H | NO2 | H | CF3 | -CH2-cyc-C3H5 | 1 | |
| II-657 | CH3 | H | CF3 | H | Cl | H | Cl | -CH2-cyc-C3H5 | 0 | |
| II-658 | CH3 | H | CF3 | H | Cl | H | Cl | -CH2-cyc-C3H5 | 1 | |
| II-659 | CH3 | CH3 | CF3 | H | Cl | H | Cl | -CH2-cyc-C3H5 | 0 | |
| II-660 | CH3 | CH3 | CF3 | H | Cl | H | Cl | -CH2-cyc-C3H5 | 1 | |
| II-661 | CH3 | H | CF3 | H | Br | H | Cl | -CH2-cyc-C3H5 | 0 | |
| II-662 | CH3 | H | CF3 | H | Br | H | Cl | -CH2-cyc-C3H5 | 1 | |
| II-663 | CH3 | CH3 | CF3 | H | Br | H | Cl | -CH2-cyc-C3H5 | 0 | |
| II-664 | CH3 | CH3 | CF3 | H | Br | H | Cl | -CH2-cyc-C3H5 | 1 | |
| II-665 | CH3 | H | CF3 | H | I | H | Cl | -CH2-cyc-C3H5 | 0 | |
| II-666 | CH3 | H | CF3 | H | I | H | Cl | -CH2-cyc-C3H5 | 1 | |
| II-667 | CH3 | CH3 | CF3 | H | I | H | Cl | -CH2-cyc-C3H5 | 0 | |
| II-668 | CH3 | CH3 | CF3 | H | I | H | Cl | -CH2-cyc-C3H5 | 1 | |
| II-669 | CH3 | H | CF3 | H | Br | H | Br | -CH2-cyc-C3H5 | 0 | |
| II-670 | CH3 | H | CF3 | H | Br | H | Br | -CH2-cyc-C3H5 | 1 | |
| II-671 | CH3 | CH3 | CF3 | H | Br | H | Br | -CH2-cyc-C3H5 | 0 | |
| II-672 | CH3 | CH3 | CF3 | H | Br | H | Br | -CH2-cyc-C3H5 | 1 | |
| II-673 | CH3 | H | CF3 | H | H | H | CN | -CH2COOCH3 | 0 | |
| II-674 | CH3 | H | CF3 | H | H | H | CN | -CH2COOCH3 | 1 | |
| II-675 | CH3 | CH3 | CF3 | H | H | H | CN | -CH2COOCH3 | 0 | |
| II-676 | CH3 | CH3 | CF3 | H | H | H | CN | -CH2COOCH3 | 1 | |
| II-677 | CH3 | H | CF3 | H | F | H | CN | -CH2COOCH3 | 0 | |
| II-678 | CH3 | H | CF3 | H | F | H | CN | -CH2COOCH3 | 1 | |
| II-679 | CH3 | CH3 | CF3 | H | F | H | CN | -CH2COOCH3 | 0 | |
| II-680 | CH3 | CH3 | CF3 | H | F | H | CN | -CH2COOCH3 | 1 | 1.5824 |
| II-681 | CH3 | H | CF3 | H | Cl | H | CN | -CH2COOCH3 | 0 | |
| II-682 | CH3 | H | CF3 | H | Cl | H | CN | -CH2COOCH3 | 1 | |
| II-683 | CH3 | CH3 | CF3 | H | Cl | H | CN | -CH2COOCH3 | 0 | |
| II-684 | CH3 | CH3 | CF3 | H | Cl | H | CN | -CH2COOCH3 | 1 | |
| II-685 | CH3 | H | CF3 | H | CN | H | H | -CH2COOCH3 | 0 | |
| II-686 | CH3 | H | CF3 | H | CN | H | H | -CH2COOCH3 | 1 | |
| II-687 | CH3 | CH3 | CF3 | H | CN | H | H | -CH2COOCH3 | 0 | |
| II-688 | CH3 | CH3 | CF3 | H | CN | H | H | -CH2COOCH3 | 1 | |
| II-689 | CH3 | H | CF3 | H | CN | H | Cl | -CH2COOCH3 | 0 | |
| II-690 | CH3 | H | CF3 | H | CN | H | Cl | -CH2COOCH3 | 1 | |
| II-691 | CH3 | CH3 | CF3 | H | CN | H | Cl | -CH2COOCH3 | 0 | |
| II-692 | CH3 | CH3 | CF3 | H | CN | H | Cl | -CH2COOCH3 | 1 | |
| II-693 | CH3 | H | CF3 | H | H | H | CF3 | -CH2COOCH3 | 0 | |
| II-694 | CH3 | H | CF3 | H | H | H | CF3 | -CH2COOCH3 | 1 | |
| II-695 | CH3 | CH3 | CF3 | H | H | H | CF3 | -CH2COOCH3 | 0 | |
| II-696 | CH3 | CH3 | CF3 | H | H | H | CF3 | -CH2COOCH3 | 1 | |
| II-697 | CH3 | H | CF3 | H | Cl | H | CF3 | -CH2COOCH3 | 0 | |
| II-698 | CH3 | H | CF3 | H | Cl | H | CF3 | -CH2COOCH3 | 1 | |
| II-699 | CH3 | CH3 | CF3 | H | Cl | H | CF3 | -CH2COOCH3 | 0 | |
| II-700 | CH3 | CH3 | CF3 | H | Cl | H | CF3 | -CH2COOCH3 | 1 | |
| II-701 | CH3 | H | CF3 | H | CF3 | H | H | -CH2COOCH3 | 0 | |
| II-702 | CH3 | H | CF3 | H | CF3 | H | H | -CH2COOCH3 | 1 | |
| II-703 | CH3 | CH3 | CF3 | H | CF3 | H | H | -CH2COOCH3 | 0 | |
| II-704 | CH3 | CH3 | CF3 | H | CF3 | H | H | -CH2COOCH3 | 1 | |
| II-705 | CH3 | H | CF3 | H | CF3 | H | Cl | -CH2COOCH3 | 0 | |
| II-706 | CH3 | H | CF3 | H | CF3 | H | Cl | -CH2COOCH3 | 1 | |
| II-707 | CH3 | CH3 | CF3 | H | CF3 | H | Cl | -CH2COOCH3 | 0 | |
| II-708 | CH3 | CH3 | CF3 | H | CF3 | H | Cl | -CH2COOCH3 | 1 | |
| II-709 | CH3 | H | CF3 | H | H | H | CHF2 | -CH2COOCH3 | 0 | |
| II-710 | CH3 | H | CF3 | H | H | H | CHF2 | -CH2COOCH3 | 1 | |
| II-711 | CH3 | CH3 | CF3 | H | H | H | CHF2 | -CH2COOCH3 | 0 | |
| II-712 | CH3 | CH3 | CF3 | H | H | H | CHF2 | -CH2COOCH3 | 1 | |
| II-713 | CH3 | H | CF3 | H | Cl | H | CHF2 | -CH2COOCH3 | 0 | |
| II-714 | CH3 | H | CF3 | H | Cl | H | CHF2 | -CH2COOCH3 | 1 | |
| II-715 | CH3 | CH3 | CF3 | H | Cl | H | CHF2 | -CH2COOCH3 | 0 | |
| II-716 | CH3 | CH3 | CF3 | H | Cl | H | CHF2 | -CH2COOCH3 | 1 | |
| II-717 | CH3 | H | CF3 | H | CHF2 | H | H | -CH2COOCH3 | 0 | |
| II-718 | CH3 | H | CF3 | H | CHF2 | H | H | -CH2COOCH3 | 1 | |
| II-719 | CH3 | CH3 | CF3 | H | CHF2 | H | H | -CH2COOCH3 | 0 | |
| II-720 | CH3 | CH3 | CF3 | H | CHF2 | H | H | -CH2COOCH3 | 1 | |
| II-721 | CH3 | H | CF3 | H | CHF2 | H | Cl | -CH2COOCH3 | 0 | |
| II-722 | CH3 | H | CF3 | H | CHF2 | H | Cl | -CH2COOCH3 | 1 | |
| II-723 | CH3 | CH3 | CF3 | H | CHF2 | H | Cl | -CH2COOCH3 | 0 | |
| II-724 | CH3 | CH3 | CF3 | H | CHF2 | H | Cl | -CH2COOCH3 | 1 | |
| II-725 | CH3 | H | CF3 | H | H | H | CH3 | -CH2COOCH3 | 0 | |
| II-726 | CH3 | H | CF3 | H | H | H | CH3 | -CH2COOCH3 | 1 | |
| II-727 | CH3 | CH3 | CF3 | H | H | H | CH3 | -CH2COOCH3 | 0 | |
| II-728 | CH3 | CH3 | CF3 | H | H | H | CH3 | -CH2COOCH3 | 1 | |
| II-729 | CH3 | H | CF3 | H | Cl | H | CH3 | -CH2COOCH3 | 0 | |
| II-730 | CH3 | H | CF3 | H | Cl | H | CH3 | -CH2COOCH3 | 1 | |
| II-731 | CH3 | CH3 | CF3 | H | Cl | H | CH3 | -CH2COOCH3 | 0 | |
| II-732 | CH3 | CH3 | CF3 | H | Cl | H | CH3 | -CH2COOCH3 | 1 | |
| II-733 | CH3 | H | CF3 | H | CH3 | H | H | -CH2COOCH3 | 0 | |
| II-734 | CH3 | H | CF3 | H | CH3 | H | H | -CH2COOCH3 | 1 | |
| II-735 | CH3 | CH3 | CF3 | H | CH3 | H | H | -CH2COOCH3 | 0 | |
| II-736 | CH3 | CH3 | CF3 | H | CH3 | H | H | -CH2COOCH3 | 1 | |
| II-737 | CH3 | H | CF3 | H | CH3 | H | Cl | -CH2COOCH3 | 0 | |
| II-738 | CH3 | H | CF3 | H | CH3 | H | Cl | -CH2COOCH3 | 1 | |
| II-739 | CH3 | CH3 | CF3 | H | CH3 | H | Cl | -CH2COOCH3 | 0 | |
| II-740 | CH3 | CH3 | CF3 | H | CH3 | H | Cl | -CH2COOCH3 | 1 | |
| II-741 | CH3 | H | CF3 | H | Cl | H | NO2 | -CH2COOCH3 | 0 | |
| II-742 | CH3 | H | CF3 | H | Cl | H | NO2 | -CH2COOCH3 | 1 | |
| II-743 | CH3 | CH3 | CF3 | H | Cl | H | NO2 | -CH2COOCH3 | 0 | |
| II-744 | CH3 | CH3 | CF3 | H | Cl | H | NO2 | -CH2COOCH3 | 1 | |
| II-745 | CH3 | H | CF3 | H | NO2 | H | NO2 | -CH2COOCH3 | 0 | |
| II-746 | CH3 | H | CF3 | H | NO2 | H | NO2 | -CH2COOCH3 | 1 | |
| II-747 | CH3 | CH3 | CF3 | H | NO2 | H | NO2 | -CH2COOCH3 | 0 | |
| II-748 | CH3 | CH3 | CF3 | H | NO2 | H | NO2 | -CH2COOCH3 | 1 | |
| II-749 | CH3 | H | CF3 | H | NO2 | H | CF3 | -CH2COOCH3 | 0 | |
| II-750 | CH3 | H | CF3 | H | NO2 | H | CF3 | -CH2COOCH3 | 1 | |
| II-751 | CH3 | CH3 | CF3 | H | NO2 | H | CF3 | -CH2COOCH3 | 0 | |
| II-752 | CH3 | CH3 | CF3 | H | NO2 | H | CF3 | -CH2COOCH3 | 1 | |
| II-753 | CH3 | H | CF3 | H | Cl | H | Cl | -CH2COOCH3 | 0 | |
| II-754 | CH3 | H | CF3 | H | Cl | H | Cl | -CH2COOCH3 | 1 | |
| II-755 | CH3 | CH3 | CF3 | H | Cl | H | Cl | -CH2COOCH3 | 0 | |
| II-756 | CH3 | CH3 | CF3 | H | Cl | H | Cl | -CH2COOCH3 | 1 | |
| II-757 | CH3 | H | CF3 | H | Br | H | Cl | -CH2COOCH3 | 0 | |
| II-758 | CH3 | H | CF3 | H | Br | H | Cl | -CH2COOCH3 | 1 | |
| II-759 | CH3 | CH3 | CF3 | H | Br | H | Cl | -CH2COOCH3 | 0 | |
| II-760 | CH3 | CH3 | CF3 | H | Br | H | Cl | -CH2COOCH3 | 1 | |
| II-761 | CH3 | H | CF3 | H | I | H | Cl | -CH2COOCH3 | 0 | |
| II-762 | CH3 | H | CF3 | H | I | H | Cl | -CH2COOCH3 | 1 | |
| II-763 | CH3 | CH3 | CF3 | H | I | H | Cl | -CH2COOCH3 | 0 | |
| II-764 | CH3 | CH3 | CF3 | H | I | H | Cl | -CH2COOCH3 | 1 | |
| II-765 | CH3 | H | CF3 | H | Br | H | Br | -CH2COOCH3 | 0 | |
| II-766 | CH3 | H | CF3 | H | Br | H | Br | -CH2COOCH3 | 1 | |
| II-767 | CH3 | CH3 | CF3 | H | Br | H | Br | -CH2COOCH3 | 0 | |
| II-768 | CH3 | CH3 | CF3 | H | Br | H | Br | -CH2COOCH3 | 1 | |
| II-769 | CH3 | H | CF3 | H | H | H | CN | -CH2Ph | 0 | |
| II-770 | CH3 | H | CF3 | H | H | H | CN | -CH2Ph | 1 | |
| II-771 | CH3 | CH3 | CF3 | H | H | H | CN | -CH2Ph | 0 | |
| II-772 | CH3 | CH3 | CF3 | H | H | H | CN | -CH2Ph | 1 | |
| II-773 | CH3 | H | CF3 | H | F | H | CN | -CH2Ph | 0 | |
| II-774 | CH3 | H | CF3 | H | F | H | CN | -CH2Ph | 1 | |
| II-775 | CH3 | CH3 | CF3 | H | F | H | CN | -CH2Ph | 0 | |
| II-776 | CH3 | CH3 | CF3 | H | F | H | CN | -CH2Ph | 1 | |
| II-777 | CH3 | H | CF3 | H | Cl | H | CN | -CH2Ph | 0 | |
| II-778 | CH3 | H | CF3 | H | Cl | H | CN | -CH2Ph | 1 | |
| II-779 | CH3 | CH3 | CF3 | H | Cl | H | CN | -CH2Ph | 0 | |
| II-780 | CH3 | CH3 | CF3 | H | Cl | H | CN | -CH2Ph | 1 | |
| II-781 | CH3 | H | CF3 | H | CN | H | H | -CH2Ph | 0 | |
| II-782 | CH3 | H | CF3 | H | CN | H | H | -CH2Ph | 1 | |
| II-783 | CH3 | CH3 | CF3 | H | CN | H | H | -CH2Ph | 0 | |
| II-784 | CH3 | CH3 | CF3 | H | CN | H | H | -CH2Ph | 1 | |
| II-785 | CH3 | H | CF3 | H | CN | H | Cl | -CH2Ph | 0 | |
| II-786 | CH3 | H | CF3 | H | CN | H | Cl | -CH2Ph | 1 | |
| II-787 | CH3 | CH3 | CF3 | H | CN | H | Cl | -CH2Ph | 0 | |
| II-788 | CH3 | CH3 | CF3 | H | CN | H | Cl | -CH2Ph | 1 | |
| II-789 | CH3 | H | CF3 | H | H | H | CF3 | -CH2Ph | 0 | |
| II-790 | CH3 | H | CF3 | H | H | H | CF3 | -CH2Ph | 1 | |
| II-791 | CH3 | CH3 | CF3 | H | H | H | CF3 | -CH2Ph | 0 | |
| II-792 | CH3 | CH3 | CF3 | H | H | H | CF3 | -CH2Ph | 1 | |
| II-793 | CH3 | H | CF3 | H | Cl | H | CF3 | -CH2Ph | 0 | |
| II-794 | CH3 | H | CF3 | H | Cl | H | CF3 | -CH2Ph | 1 | |
| II-795 | CH3 | CH3 | CF3 | H | Cl | H | CF3 | -CH2Ph | 0 | |
| II-796 | CH3 | CH3 | CF3 | H | Cl | H | CF3 | -CH2Ph | 1 | |
| II-797 | CH3 | H | CF3 | H | CF3 | H | H | -CH2Ph | 0 | |
| II-798 | CH3 | H | CF3 | H | CF3 | H | H | -CH2Ph | 1 | |
| II-799 | CH3 | CH3 | CF3 | H | CF3 | H | H | -CH2Ph | 0 | |
| II-800 | CH3 | CH3 | CF3 | H | CF3 | H | H | -CH2Ph | 1 | |
| II-801 | CH3 | H | CF3 | H | CF3 | H | Cl | -CH2Ph | 0 | |
| II-802 | CH3 | H | CF3 | H | CF3 | H | Cl | -CH2Ph | 1 | |
| II-803 | CH3 | CH3 | CF3 | H | CF3 | H | Cl | -CH2Ph | 0 | |
| II-804 | CH3 | CH3 | CF3 | H | CF3 | H | Cl | -CH2Ph | 1 | |
| II-805 | CH3 | H | CF3 | H | H | H | CHF2 | -CH2Ph | 0 | |
| II-806 | CH3 | H | CF3 | H | H | H | CHF2 | -CH2Ph | 1 | |
| II-807 | CH3 | CH3 | CF3 | H | H | H | CHF2 | -CH2Ph | 0 | |
| II-808 | CH3 | CH3 | CF3 | H | H | H | CHF2 | -CH2Ph | 1 | |
| II-809 | CH3 | H | CF3 | H | Cl | H | CHF2 | -CH2Ph | 0 | |
| II-810 | CH3 | H | CF3 | H | C | H | CHF2 | -CH2Ph | 1 | |
| II-811 | CH3 | CH3 | CF3 | H | Cl | H | CHF2 | -CH2Ph | 0 | |
| II-812 | CH3 | CH3 | CF3 | H | Cl | H | CHF2 | -CH2Ph | 1 | |
| II-813 | CH3 | H | CF3 | H | CHF2 | H | H | -CH2Ph | 0 | |
| II-814 | CH3 | H | CF3 | H | CHF2 | H | H | -CH2Ph | 1 | |
| II-815 | CH3 | CH3 | CF3 | H | CHF2 | H | H | -CH2Ph | 0 | |
| II-816 | CH3 | CH3 | CF3 | H | CHF2 | H | H | -CH2Ph | 1 | |
| II-817 | CH3 | H | CF3 | H | CHF2 | H | Cl | -CH2Ph | 0 | |
| II-818 | CH3 | H | CF3 | H | CHF2 | H | Cl | -CH2Ph | 1 | |
| II-819 | CH3 | CH3 | CF3 | H | CHF2 | H | Cl | -CH2Ph | 0 | |
| II-820 | CH3 | CH3 | CF3 | H | CHF2 | H | Cl | -CH2Ph | 1 | |
| II-821 | CH3 | H | CF3 | H | H | H | CH3 | -CH2Ph | 0 | |
| II-822 | CH3 | H | CF3 | H | H | H | CH3 | -CH2Ph | 1 | |
| II-823 | CH3 | CH3 | CF3 | H | H | H | CH3 | -CH2Ph | 0 | |
| II-824 | CH3 | CH3 | CF3 | H | H | H | CH3 | -CH2Ph | 1 | |
| II-825 | CH3 | H | CF3 | H | Cl | H | CH3 | -CH2Ph | 0 | |
| II-826 | CH3 | H | CF3 | H | Cl | H | CH3 | -CH2Ph | 1 | |
| II-827 | CH3 | CH3 | CF3 | H | Cl | H | CH3 | -CH2Ph | 0 | |
| II-828 | CH3 | CH3 | CF3 | H | Cl | H | CH3 | -CH2Ph | 1 | |
| II-829 | CH3 | H | CF3 | H | CH3 | H | H | -CH2Ph | 0 | |
| II-830 | CH3 | H | CF3 | H | CH3 | H | H | -CH2Ph | 1 | |
| II-831 | CH3 | CH3 | CF3 | H | CH3 | H | H | -CH2Ph | 0 | |
| II-832 | CH3 | CH3 | CF3 | H | CH3 | H | H | -CH2Ph | 1 | |
| II-833 | CH3 | H | CF3 | H | CH3 | H | Cl | -CH2Ph | 0 | |
| II-834 | CH3 | H | CF3 | H | CH3 | H | Cl | -CH2Ph | 1 | |
| II-835 | CH3 | CH3 | CF3 | H | CH3 | H | Cl | -CH2Ph | 0 | |
| II-836 | CH3 | CH3 | CF3 | H | CH3 | H | Cl | -CH2Ph | 1 | |
| II-837 | CH3 | H | CF3 | H | Cl | H | NO2 | -CH2Ph | 0 | |
| II-838 | CH3 | H | CF3 | H | Cl | H | NO2 | -CH2Ph | 1 | |
| II-839 | CH3 | CH3 | CF3 | H | Cl | H | NO2 | -CH2Ph | 0 | |
| II-840 | CH3 | CH3 | CF3 | H | Cl | H | NO2 | -CH2Ph | 1 | |
| II-841 | CH3 | H | CF3 | H | NO2 | H | NO2 | -CH2Ph | 0 | |
| II-842 | CH3 | H | CF3 | H | NO2 | H | NO2 | -CH2Ph | 1 | |
| II-843 | CH3 | CH3 | CF3 | H | NO2 | H | NO2 | -CH2Ph | 0 | |
| II-844 | CH3 | CH3 | CF3 | H | NO2 | H | NO2 | -CH2Ph | 1 | |
| II-845 | CH3 | H | CF3 | H | NO2 | H | CF3 | -CH2Ph | 0 | |
| II-846 | CH3 | H | CF3 | H | NO2 | H | CF3 | -CH2Ph | 1 | |
| II-847 | CH3 | CH3 | CF3 | H | NO2 | H | CF3 | -CH2Ph | 0 | |
| II-848 | CH3 | CH3 | CF3 | H | NO2 | H | CF3 | -CH2Ph | 1 | |
| II-849 | CH3 | H | CF3 | H | Cl | H | Cl | -CH2Ph | 0 | |
| II-850 | CH3 | H | CF3 | H | Cl | H | Cl | -CH2Ph | 1 | |
| II-851 | CH3 | CH3 | CF3 | H | Cl | H | Cl | -CH2Ph | 0 | |
| II-852 | CH3 | CH3 | CF3 | H | Cl | H | Cl | -CH2Ph | 1 | |
| II-853 | CH3 | H | CF3 | H | Br | H | Cl | -CH2Ph | 0 | |
| II-854 | CH3 | H | CF3 | H | Br | H | Cl | -CH2Ph | 1 | |
| II-855 | CH3 | CH3 | CF3 | H | Br | H | Cl | -CH2Ph | 0 | |
| II-856 | CH3 | CH3 | CF3 | H | Br | H | Cl | -CH2Ph | 1 | |
| II-857 | CH3 | H | CF3 | H | I | H | Cl | -CH2Ph | 0 | |
| II-858 | CH3 | H | CF3 | H | I | H | Cl | -CH2Ph | 1 | |
| II-859 | CH3 | CH3 | CF3 | H | I | H | Cl | -CH2Ph | 0 | |
| II-860 | CH3 | CH3 | CF3 | H | I | H | Cl | -CH2Ph | 1 | |
| II-861 | CH3 | H | CF3 | H | Br | H | Br | -CH2Ph | 0 | |
| II-862 | CH3 | H | CF3 | H | Br | H | Br | -CH2Ph | 1 | |
| II-863 | CH3 | CH3 | CF3 | H | Br | H | Br | -CH2Ph | 0 | |
| II-864 | CH3 | CH3 | CF3 | H | Br | H | Br | -CH2Ph | 1 | |
| II-865 | CH3 | H | CH3 | H | F | H | CN | CH2CH2CH3 | 0 | 73-76 |
| II-866 | CH3 | H | CH3 | H | F | H | CN | CH2CH2CH3 | 1 | 105-112 |
| II-867 | CH3 | CH3 | CH3 | H | F | H | CN | CH2CH2CH3 | 0 | 1.6053 |
| II-868 | CH3 | CH3 | CH3 | H | F | H | CN | CH2CH2CH3 | 1 | 1.5943 |

**[TABLE 3]**

| Table 3 | | | | | |
|---|---|---|---|---|---|
| 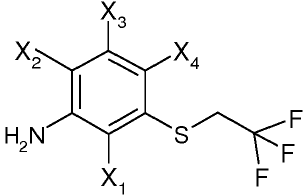 | | | | | |

| Comp. No. | X1 | X2 | X3 | X4 | m.p., NMR |
|---|---|---|---|---|---|
| III-1 | H | H | H | CN | |
| III-2 | H | F | H | CN | 105-109 |
| III-3 | H | Cl | H | CN | |
| III-4 | H | Br | H | CN | |
| III-5 | H | I | H | CN | |
| III-6 | Cl | H | H | CN | |
| III-7 | H | CN | H | H | |
| III-8 | H | CN | H | Cl | |
| III-9 | H | CN | H | Br | |
| III-10 | H | CN | H | I | |
| III-11 | Cl | CN | H | H | |
| III-12 | H | H | H | CF3 | |
| III-13 | H | Cl | H | CF3 | |
| III-14 | H | Br | H | CF3 | |
| III-15 | H | I | H | CF3 | |
| III-16 | Cl | H | H | CF3 | |
| III-17 | H | CF3 | H | H | |
| III-18 | H | CF3 | H | Cl | |
| III-19 | H | CF3 | H | Br | |
| III-20 | H | CF3 | H | I | |
| III-21 | Cl | CF3 | H | H | |
| III-22 | H | H | H | CHF2 | |
| III-23 | H | Cl | H | CHF2 | |
| III-24 | H | Br | H | CHF2 | |
| III-25 | H | I | H | CHF2 | |
| III-26 | Cl | H | H | CHF2 | |
| III-27 | H | CHF2 | H | H | |
| III-28 | H | CHF2 | H | Cl | |
| III-29 | H | CHF2 | H | Br | |
| III-30 | H | CHF2 | H | I | |
| III-31 | Cl | CHF2 | H | H | |
| III-32 | H | H | H | CH3 | |
| III-33 | H | Cl | H | CH3 | |
| III-34 | H | Br | H | CH3 | |
| III-35 | H | I | H | CH3 | |
| III-36 | Cl | H | H | CH3 | |
| III-37 | H | CH3 | H | H | |
| III-38 | H | CH3 | H | Cl | |
| III-39 | H | CH3 | H | Br | |
| III-40 | H | CH3 | H | I | |
| III-41 | Cl | CH3 | H | H | |
| III-42 | H | Cl | H | Cl | 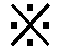 |
| III-43 | H | Br | H | Cl | |
| III-44 | H | I | H | Cl | |
| III-45 | H | Br | H | Br | |
| III-46 | H | Cl | H | NO2 | |
| III-47 | H | NO2 | H | NO2 | |
| III-48 | H | CF3 | H | NO2 | |

| | | | | | |
|---|---|---|---|---|---|
| 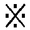 = 1H-NMR(CDCl3, δ) 3.43(2H, ddd, J = 9.6 Hz) 4.2(2H, m) 6.98(1 H, s) 7.32(1 H, s). | | | | | |

Biological Test Example 1: test on SPODOPTERA LITURA larva Preparation of sample chemical solution
Solvent: dimethylformamide 3 parts by weight
Emulsifier: polyoxyethylene alkylphenyl ether 1 part by weight

For preparing a suitable blend of active compounds, 1 part by weight of active compounds was mixed with a solvent of the above-mentioned amount containing an emulsifier of the above-mentioned amount, and the mixture was diluted to given concentration with water.

### Test Method

Leaves of sweet potato were immersed in a sample chemical solution diluted to a given concentration, the chemical solution was air-dried, then, placed in a petri dish having a diameter of 9 cm, ten 3 day-age SPODOPTERA LITURA larvae were released, the petri dish was placed in a constant temperature room at 25°C, and leaves of sweet potato were added 2 days and 4 days after, and the number of dead insects was checked 7 days after and the insecticidal ratio was calculated.

In this test, the results of two petri dishes in one district were averaged.

### Test result

In the above-mentioned Biological Test Example 1, typically, the above-mentioned compound No. I-62 manifested a control effect of an insecticidal ratio of 100% at an active ingredient concentration of 500 ppm.

### Biological Test Example 2: test on two-spotted spider mite (Tetranychus urticae) (spray test)

### Test method

On leaves of marrow bean in the two true leaves developing period cultivated in a pot having a diameter of 6 cm, 50 to 100 adult two-spotted spider mite (Tetranychus urticae) insects were released, and one day after, a water-diluted solution of the active compounds prepared above of a given concentration was sprayed in sufficient amount using a spray gun. After spraying, the leaves were placed in a green house and 7 days after, the miticidal ratio was calculated.

### Test results

The above-mentioned compounds Nos. I-3, I-4, I-7, I-9, I-10, I-15, I-16, I-29, I-30, I-31, I-32, I-33, I-34, I-37, I-38, I-39, I-40, I-41, I-42, I-43, I-44, I-49, I-50, I-51, I-52, I-53, I-54, I-55, I-65, I-68, I-69, I-70, I-71, I-75, I-81, I-82, I-91, I-92, I-93, I-94, I-101, I-102, I-105, I-115, I-116, I-117, I-118, I-147, I-148, I-155, I-156, I-157, II-229, II-230 manifested a control effect of a miticidal ratio of 98% or more at an active ingredient concentration of 100 ppm, as typical examples.

### Biological Test Example 3: test on cucurbit leaf beetles (AULACOPHORA FEMORALIS) (spray test)

### Test method

Cucumber leaves were immersed in a water-diluted solution of active compounds prepared above of a given concentration, and the chemical solution was air-dried, then, placed in a plastic cup filled with sterile-disinfected black dirt soil, and five 2-day old cucurbit leaf beetles (AULACOPHORA FEMORALIS) were released. 7 days after, the number of dead insects was checked, and the insecticidal ratio was calculated.

### Test result

The above-mentioned compounds Nos. I-16, I-29, I-30, I-31, I-32, I-33, I-34, I-37, I-38, I-40, I-41, I-42, I-51, I-52, I-53, I-55, I-62, I-65, I-75 manifested a control effect of an insecticidal ratio of 100% at an active ingredient concentration of 500 ppm, as typical examples.

### Biological Test Example 4: test on two-spotted spider mite (irrigation test)

### Test method

On leaves of marrow bean in the two true leaves developing period cultivated in a pot having a diameter of 6 cm, 50 to 100 adult two-spotted spider mite insects were released, and one day after, a water-diluted solution of the active compounds prepared above of a given concentration was irrigated at a certain amount in the pot using pipette. After treatment, the leaves were placed in a greenhouse, and 7 days after, the miticidal ratio was calculated.

### Test results

The above-mentioned compounds Nos. I-4, I-165 and I-166 manifested a control effect of an insecticidal ratio of 98% or more at an active ingredient concentration of 1.5 mg/pot, as typical examples.

### Preparation Example 1 (granule)

Into a mixture of 10 parts of compounds of the present invention (No. I-16), 30 parts of bentonite (montmorillonite), 58 parts of talc and 2 parts of a ligninsulfonate was added 25 parts of water, the mixture was kneaded well, and made into 10 to 40 mesh particles by an extrusion type granulator, and dried at 40 to 50°C to give granules.

### Preparation Example 2 (granule)

95 parts of clay mineral particles having a particle size distribution of 0.2 to 2 mm were placed in a rotation mixer, and 5 parts of compounds of the present invention (No. I-19) was sprayed together with a liquid diluting agent under rotation to cause uniform wetting, then, dried at 40 to 50°C to give granules.

### Preparation Example 3 (emulsion)

30 parts of compounds of the present invention (No. I-30), 55 parts of xylene, 8 parts of polyoxyethylene alkylphenyl ether and 7 parts of calcium alkylbenzenesulfonate were mixed and stirred to give an emulsion.

### Preparation Example 4 (hydrate)

15 parts of compounds of the present invention (No. I-31), 80 parts of a mixture (1:5) of white carbon (water-containing amorphous silicon oxide fine powder) and powdery clay, 2 parts of sodium alkylbenzenesulfonate and 3 parts of sodium alkylnaphthalenesulfonate-formalin condensate were ground and mixed to give a hydrate.

### Preparation Example 5 (hydrated granule)

20 parts of compounds of the present invention (No. I-32), 30 parts of sodium ligninsulfonate and 15 parts of bentonite, and 35 parts of calcined diatomaceous earth powder were mixed sufficiently, and water was added to this, and the mixture was extruded by 0.3 mm screen and dried, to give hydrated granules.

### Summary of the invention

Novel benzamidines of the formula (I) 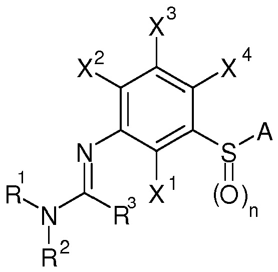 and a use thereof as insecticides.

## Claims

1. Novel Benzamidines of the formula (I), 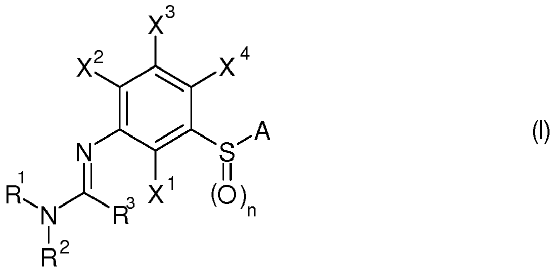 wherein R¹ represents hydrogen, straight or branched C₁₋₁₂ alkyl, C₂₋₅ alkenyl, C₂₋₃ alkynyl, alkoxy, C₃₋₈ cycloalkyl, haloalkyl, alkoxyalkyl, phenyl, α- or β-naphthyl, aralkyl, alkylaminocarbonyl, arylaminocarbonyl, or dialkylaminoalkyl,
R² represents hydrogen or straight or branched C₁₋₁₂ alkyl,
R¹ and R² may form, together with an N atom to which they are attached, pyrrolidine, piperidine or morpholine,
R³ represents hydrogen, straight or branched C₁₋₁₂ alkyl, haloalkyl, cyano or phenyl, α- or β-naphthyl,
R¹ and R³ may form, together with an N atom and C atom to which they are attached, pyrrolidine or piperidine optionally substituted with oxo,
X¹, X², X³ and X⁴ may be the same or different and represent hydrogen, halogen, straight or branched C₁₋₁₂ alkyl, haloalkyl, haloalkoxy, alkoxycarbonyl, nitro or cyano,
A represents trifluoroethyl and
n represents 0 or 1.

2. The compounds according to Claim 1, wherein
R¹ represents hydrogen, C₁₋₆ alkyl, C₂₋₄ alkenyl, C₂₋₃ alkynyl,
C₁₋₄ alkoxy, C₃₋₆ cycloalkyl, C₁₋₄ haloalkyl, C₂₋₄ (total carbon number) alkoxyalkyl, phenyl, α- or β-naphthyl, C₇₋₈ aralkyl, C₁₋₂ alkylaminocarbonyl, C₆₋₁₀ arylaminocarbonyl, or C₂₋₄ (total carbon number) dialkylamino-C₁₋₂ alkyl,
R² represents hydrogen or C₁₋₄ alkyl,
R¹ and R² may form, together with an N atom to which they are attached, pyrrolidine, piperidine or morpholine,
R³ represents hydrogen, C₁₋₆ alkyl, C₁₋₄ haloalkyl, cyano or phenyl,
R¹ and R³ may form, together with an N atom and C atom to which they are attached, pyrrolidine or piperidine optionally substituted with oxo,
X¹, X², X³ and X⁴ may be the same or different and represent hydrogen, halogen, C₁₋₂ alkyl, C₁₋₂ haloalkyl, C₁₋₂ haloalkoxy, C₁₋₂ alkoxycarbonyl, nitro or cyano,
A represents trifluoroethyl, and
n represents 0 or 1.

3. The compounds according to Claim 1 or 2, wherein
R¹ represents hydrogen, C₁₋₃ alkyl, allyl, propargyl, C₁₋₂ alkoxy, cyclopropyl, cyclohexyl, trifluoroethyl, methoxyethyl, trifluoromethylphenyl, phenyl, benzyl, , ethylaminocarbonyl, phenylaminocarbonyl, or dimethylaminoethyl,
R² represents hydrogen or C₁₋₂ alkyl,
R¹ and R² may form, together with an N atom to which they are attached, piperidine or morpholine,
R³ represents hydrogen, C₁ alkyl, fluoro-substituted-C₁-₃ alkyl, cyano or phenyl,
R¹ and R³ may form, together with an N atom and C atom to which they are attached, pyrrolidine or piperidine optionally substituted with oxo,
X¹ represents hydrogen or chloro,
X² represents hydrogen, fluoro, chloro, bromo, iodo, methyl, difluoromethyl, trifluoromethyl, nitro or cyano,
X³ represents hydrogen,
X⁴ represents hydrogen, chloro, bromo, iodo, methyl, difluoromethyl, trifluoromethyl, nitro or cyano,
A represents trifluoroethyl and
n represents 0 or 1.

4. The compounds according to any of Claims 1 to 3 wherein
R¹ represents hydrogen, C₁₋₃ alkyl, allyl, propargyl, methoxy, cyclopropyl, trifluoroethyl, trifluoromethylphenyl, benzyl, or ethylaminocarbonyl,
R² represents hydrogen, or C₁₋₂ alkyl,
R³ represents C₁ alkyl, C₁ alkylene, fluoro-substituted-C₁₋₃ alkyl, cyano or phenyl,
R¹ and R³ may form, together with an N atom and C atom to which they are attached, pyrrolidine
X¹ represents hydrogen,
X² represents hydrogen, or fluoro,
X³ represents hydrogen,
X⁴ represents difluoromethyl, or cyano,
A represents trifluoroethyl, and
n represents 0 or 1.

5. Process for the preparation of the compounds of the formula (I) according to claim 1, **characterized in that** Production method (a): {in the formula (I), at least one of X² and X⁴ represents an electron attractive group, and n represents 0}
compounds of the formula (II) 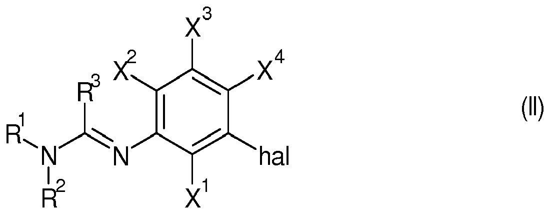 wherein, R¹, R², R³, X¹, X², X³ and X⁴ are as defined in claim 1 and at least one of X² and X⁴ represents an electron attractive group, and hal represents a halogen,
are reacted with compounds of the formula (III)
HS-A (III)
wherein, A is as defined above,
in the presence of inert solvent and if appropriate, in the presence of a base,
Production method (b): {in the formula (I), n represents 1}
compounds of the formula (Ib) 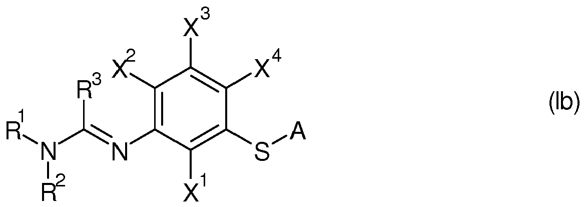 wherein R¹, R², R³, X¹, X², X³, X⁴ and A are as in claim 1, are oxidized in the presence of inert solvents,
Production method (c): {in the formula (I), R³ represents hydrogen, alkyl or aryl}
compounds of the formula (IV) 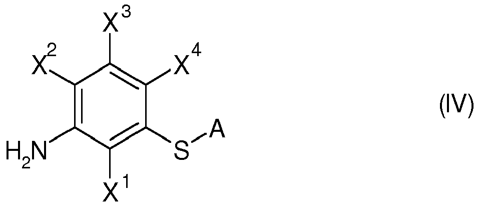 wherein X¹, X², X³, X⁴ and A are as defined in claim 1, are reacted with compounds of the formula (V) 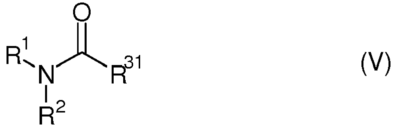 wherein R¹ and R² are as defined in claim 1, R³¹ represents hydrogen, alkyl or aryl,
in the presence of inert solvents,
Production method (d): {in the formula (I), R³ represents perfluoroalkyl}
compounds of the formula (VI) 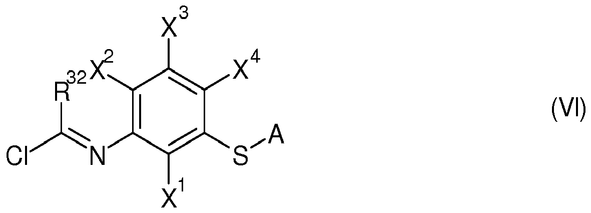 wherein X¹, X², X³, X⁴ and A are as defined in claim 1, and R³² represents perfluoroalkyl,
are reacted with compounds of the formula (VII) 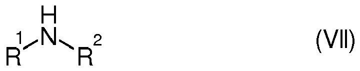 wherein R¹ and R² are as defined in claim 1,
in the presence of inert solvents and if appropriate, in the presence of a base,
Production method (e): {in the formula (I), R³ represents cyano}
compounds of the formula (VIII) 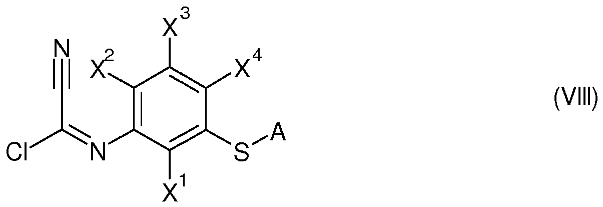 wherein X¹, X², X³, X⁴ and A are as defined in claim 1,
are reacted with compounds of the above-mentioned compounds of the formula (VII)in the presence of inert solvents,
Production method (f): {in the formula (I), R¹ represents alkylaminocarbonyl}
compounds of the formula (If) 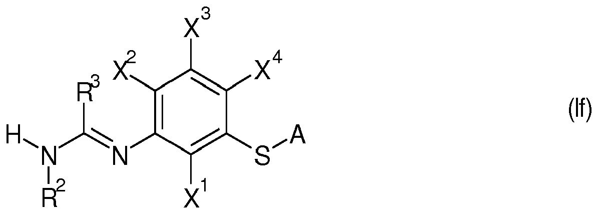 wherein R², R³, X¹, X², X³, X⁴ and A are as defined in claim 1,
are reacted with compounds of the formula (X)
R¹¹-N=C=O (X)
wherein R¹¹ represents alkylaminocarbonyl,
in the presence of inert solvents and if appropriate, in the presence of a base.

6. Insecticidal compositions, **characterized in that** they contain at least one compound of the formula (I)as defined in claim 1 or in claim 2 or in claim 3 or in claim 4.

7. Process for combating harmful insects, **characterized in that** the compounds of the formula (I) as defined in claim 1 or in claim 2 or in claim 3 or in claim 4 are allowed to act on harmful insects and/or their habitat.

8. Use of the compounds of the formula (I) as defined in claim 1 or in claim 2 or in claim 3 or in claim 4 for combating harmful insects.

9. Process for the preparation of insecticidal composition, **characterized in that** the compounds of the formula (I) as defined in claim 1 or in claim 2 or in claim 3 or in claim 4 are mixed with extenders and/or surface active agents.

10. Novel aniline derivatives of the formula (IV-A) 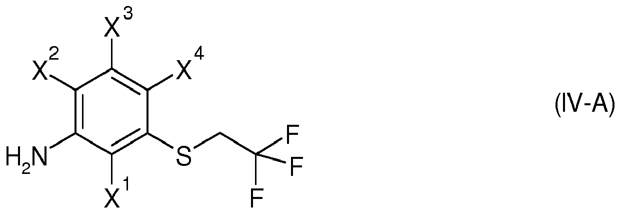 wherein X¹, X² and X³ are as defined in claim 1 and X⁴ represents hydrogen, halogen, haloalkyl, haloalkoxy, alkoxycarbonyl, nitro or cyano.

## Patentansprüche

1. Neue Benzamidine der Formel (I), 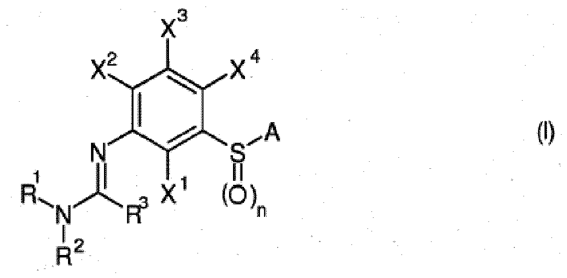 worin R¹ Wasserstoff, geradkettiges oder verzweigtes C₁₋₁₂-Alkyl, C₂₋₅-Alkenyl, C₂₋₃-Alkinyl, Alkoxy, C₃₋₈-Cycloalkyl, Halogenalkyl, Alkoxyalkyl, Phenyl, α- oder β-Naphthyl, Aralkyl, Alkylaminocarbonyl, Arylaminocarbonyl oder Dialkylaminoalkyl bedeutet,
R² Wasserstoff oder geradkettiges oder verzweigtes C₁₋₁₂-Alkyl bedeutet,
R¹ und R² gemeinsam mit einem N-Atom, an das sie gebunden sind, Pyrrolidin, Piperdin oder Morpholin bilden können,
R³ Wasserstoff, geradkettiges oder verzweigtes C₁₋₁₂-Alkyl, Halogenalkyl, Cyano oder Phenyl, α-oder β-Naphthyl bedeutet,
R¹ und R³ gemeinsam mit einem N-Atom und C-Atom, an das sie gebunden sind, gegebenenfalls durch Oxo substituiertes Pyrrolidin oder Piperidin bilden können,
X¹, X², X³ und X⁴ gleich oder verschieden sein können und Wasserstoff, Halogen, geradkettiges oder verzweigtes C₁₋₁₂-Alkyl, Halogenalkyl, Halogenalkoxy, Alkoxycarbonyl, Nitro oder Cyano bedeuten,
A Trifluorethyl bedeutet und
n 0 oder 1 bedeutet.

2. Verbindungen nach Anspruch 1, worin
R¹ Wasserstoff, C₁₋₆-Alkyl, C₂₋₄-Alkenyl, C₂₋₃-Alkinyl, C₁₋₄-Alkoxy, C₃₋₆-Cycloalkyl, C₁₋₄-Halogenalkyl, C₂₋₄(Gesamtanzahl der Kohlenstoffe)-Alkoxyalkyl, Phenyl, α- oder β-Naphthyl, C₇₋₈-Aralkyl, C₁₋₂-Alkylaminocarbonyl, C₆₋₁₀-Arylaminocarbonyl oder C₂₋₄ (Gesamtanzahl der Kohlenstoffe)-Dialkylamino-C₁₋₂-alkyl bedeutet,
R² Wasserstoff oder C₁₋₄-Alkyl bedeutet,
R¹ und R² gemeinsam mit einem N-Atom, an das sie gebunden sind, Pyrrolidin, Piperidin oder Morpholin bilden können,
R³ Wasserstoff, C₁₋₆-Alkyl, C₁₋₄-Halogenalkyl, Cyano oder Phenyl bedeutet,
R¹ und R³ gemeinsam mit einem N-Atom und C-Atom, an das sie gebunden sind, gegebenenfalls durch Oxo substituiertes Pyrrolidin oder Piperidin bilden können,
X¹, X², X³ und X⁴ gleich oder verschieden sein können und Wasserstoff, Halogen, C₁₋₂-Alkyl, C₁₋₂-Halogenalkyl, C₁₋₂-Halogenalkoxy, C₁₋₂-Alkoxycarbonyl, Nitro oder Cyano bedeuten,
A Trifluorethyl bedeutet und
n 0 oder 1 bedeutet.

3. Verbindungen nach Anspruch 1 oder 2, worin
R¹ Wasserstoff, C₁₋₃-Alkyl, Allyl, Propargyl, C₁₋₂-Alkoxy, Cyclopropyl, Cyclohexyl, Trifluorethyl, Methoxyethyl, Trifluormethylphenyl, Phenyl, Benzyl, Ethylaminocarbonyl, Phenylaminocarbonyl oder Dimethylaminoethyl bedeutet,
R² Wasserstoff oder C₁₋₂-Alkyl bedeutet,
R¹ und R² gemeinsam mit einem N-Atom, an das sie gebunden sind, Piperidin oder Morpholin bilden können,
R³ Wasserstoff, C₁-Alkyl, fluorsubstituiertes C₁₋₃-Alkyl, Cyano oder Phenyl bedeutet,
R¹ und R³ gemeinsam mit einem N-Atom und C-Atom, an das sie gebunden sind, gegebenenfalls durch Oxo substituiertes Pyrrolidin oder Piperidin bilden können,
X¹ Wasserstoff oder Chlor bedeutet,
X² Wasserstoff, Fluor, Chlor, Brom, Iod, Methyl, Difluormethyl, Trifluormethyl, Nitro oder Cyano bedeutet,
X³ Wasserstoff bedeutet,
X⁴ Wasserstoff, Chlor, Brom, Iod, Methyl, Difluormethyl, Trifluormethyl, Nitro oder Cyano bedeutet,
A Trifluorethyl bedeutet und
n 0 oder 1 bedeutet.

4. Verbindungen nach einem der Ansprüche 1 bis 3, worin
R¹ Wasserstoff, C₁₋₃-Alkyl, Allyl, Propargyl, Methoxy, Cyclopropyl, Trifluorethyl, Trifluormethylphenyl, Benzyl oder Ethylaminocarbonyl bedeutet,
R² Wasserstoff oder C₁₋₂-Alkyl bedeutet,
R³ C₁-Alkyl, C₁-Alkylen, fluorsubstituiertes C₁₋₃-Alkyl, Cyano oder Phenyl bedeutet,
R¹ und R³ gemeinsam mit einem N-Atom und C-Atom, an das sie gebunden sind, Pyrrolidin bilden können,
X¹ Wasserstoff bedeutet,
X² Wasserstoff oder Fluor bedeutet,
X³ Wasserstoff bedeutet,
X⁴ Difluormethyl oder Cyano bedeutet,
A Trifluorethyl bedeutet und
n 0 oder 1 bedeutet.

5. Verfahren zur Herstellung der Verbindungen der Formel (I) nach Anspruch 1, **dadurch gekennzeichnet, dass** Herstellungsverfahren (a): {in Formel (I) mindestens eines von X² und X⁴ eine elektronenanziehende Gruppe bedeutet und n 0 bedeutet} Verbindungen der Formel (II) 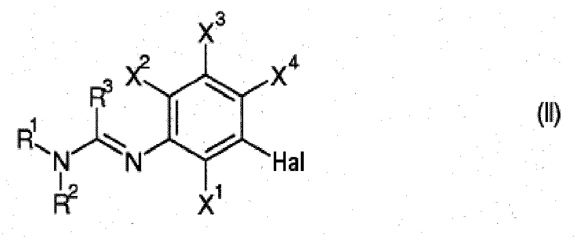 worin R¹, R², R³, X¹, X², X³ und X⁴ wie in Anspruch 1 definiert sind und mindestens eines von X² und X⁴ eine elektronenanziehende Gruppe bedeutet und Hal ein Halogen bedeutet,
mit Verbindungen der Formel (III)
**HS-A** **(III)**
worin A wie oben definiert und
in Gegenwart eines inerten Lösungsmittels und gegebenenfalls in Gegenwart einer Base umgesetzt werden,
Herstellungsverfahren (b): {in Formel (I) n 1 bedeutet}
Verbindungen der Formel (Ib) 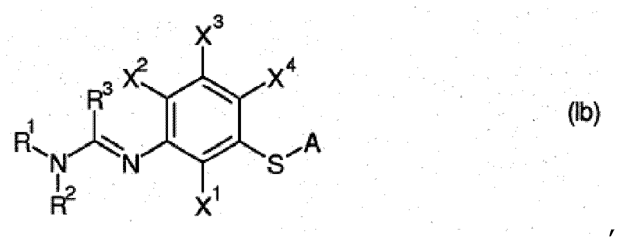 worin R¹, R², R³, X¹, X², X³, X⁴ und A wie in Anspruch 1 definiert sind
in Gegenwart von inerten Lösungsmitteln oxidiert werden,
Herstellungsverfahren (c): {in Formel (I) bedeutet R³ Wasserstoff, Alkyl oder Aryl}
Verbindungen der Formel (IV), 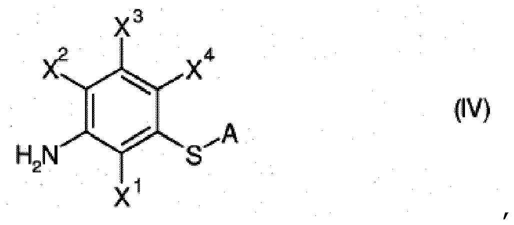 worin X¹, X², X³, X⁴ und A wie in Anspruch 1 definiert sind,
mit Verbindungen der Formel (V) 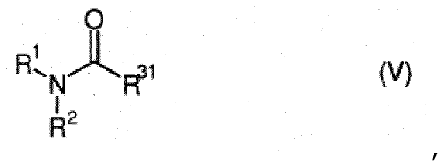 worin R¹ und R² wie in Anspruch 1 definiert sind, R³¹ Wasserstoff, Alkyl oder Aryl bedeutet,
in Gegenwart von inerten Lösungsmitteln umgesetzt werden,
Herstellungsverfahren (d): {in Formel (I) R³ Perfluoralkyl bedeutet}
Verbindungen der Formel (VI), 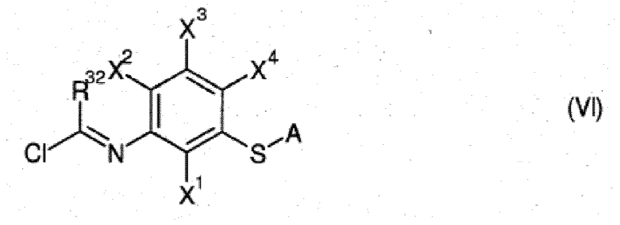 worin X¹, X², X³, X⁴ und A wie in Anspruch 1 definiert sind und R³² Perfluoralkyl bedeutet,
in Gegenwart von inerten Lösungsmitteln und gegebenenfalls in Gegenwart einer Base mit Verbindungen der Formel (VII), 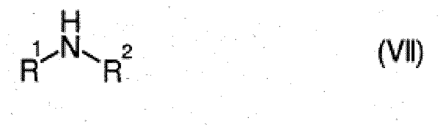 worin R¹ und R² wie in Anspruch 1 definiert sind, umgesetzt werden,
Herstellungsverfahren (e): {in Formel (I) R³ Cyano bedeutet}
Verbindungen der Formel (VIII), 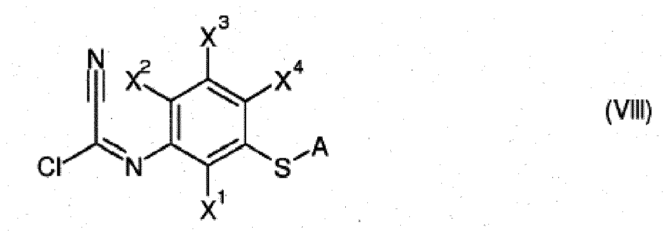 worin X¹, X², X³, X⁴ und A wie in Anspruch 1 definiert sind,
in Gegenwart von inerten Lösungsmitteln mit Verbindungen der oben erwähnten Verbindungen der Formel (VII) umgesetzt werden,
Herstellungsverfahren (f): {in Formel (I) R¹ Alkylaminocarbonyl bedeutet}
Verbindungen der Formel (If), 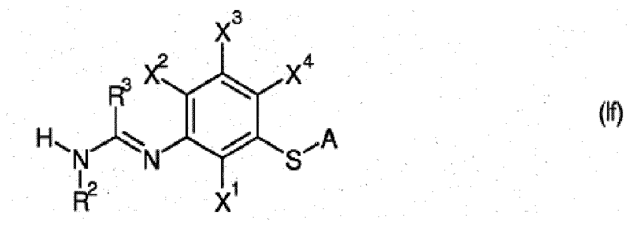 worin R², R³, X¹, X², X³, X⁴ und A wie in Anspruch 1 definiert sind,
in Gegenwart von inerten Lösungsmitteln und gegebenenfalls in Gegenwart einer Base mit Verbindungen der Formel (X), 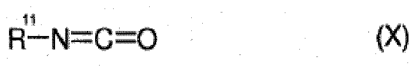 worin R¹¹ Alkylaminocarbonyl bedeutet, umgesetzt werden.

6. Insektizide Zusammensetzungen, **dadurch gekennzeichnet, dass** sie mindestens eine Verbindung der Formel (I) wie in Anspruch 1 oder in Anspruch 2 oder in Anspruch 3 oder in Anspruch 4 definiert enthalten.

7. Verfahren für die Bekämpfung von Schadinsekten, **dadurch gekennzeichnet, dass** man die Verbindungen der Formel (I) wie in Anspruch 1 oder in Anspruch 2 oder in Anspruch 3 oder in Anspruch 4 definiert auf Schadinsekten und/oder ihren Lebensraum einwirken gelassen werden.

8. Verwendung der Verbindungen der Formel (I) wie in Anspruch 1 oder in Anspruch 2 oder in Anspruch 3 oder in Anspruch 4 definiert zum Bekämpfen von Schadinsekten.

9. Verfahren zur Herstellung einer insektiziden Zusammensetzung, **dadurch gekennzeichnet, dass** man die Verbindungen der Formel (I) wie in Anspruch 1 oder in Anspruch 2 oder in Anspruch 3 oder in Anspruch 4 definiert mit Streckmitteln und/oder Tensiden mischt.

10. Neue Anilinderivate der Formel (IV-A) 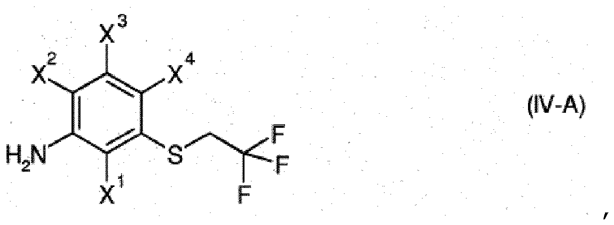 worin X¹, X² und X³ wie in Anspruch 1 definiert sind und X⁴ Wasserstoff, Halogen, Halogenalkyl, Halogenalkoxy, Alkoxycarbonyl, Nitro oder Cyano bedeutet.

## Revendications

1. Nouvelles benzamidines de formule (I) 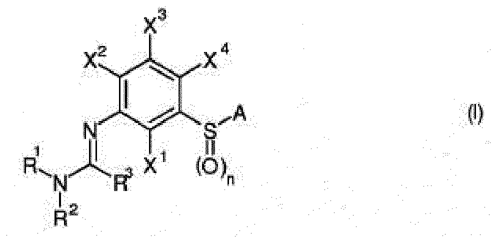 dans laquelle
R1 représente hydrogène, C1-12 alkyle linéaire ou ramifié, C2-5 alcényle, C2-3 alcynyle, alcoxy, C3-8 cycloalkyle, halogénoalkyle, alcoxyalkyle, phényle, α-ou β-naphtyle, aralkyle, alkylaminocarbonyle, arylaminocarbonyle, ou dialkylaminoalkyle,
R2 représente hydrogène ou C1-12 alkyle linéaire ou ramifié,
R1 et R2 peuvent former, conjointement avec un atome de N auquel ils sont fixés, pyrrolidine, pipéridine ou morpholine,
R3 représente hydrogène, C1-12 alkyle linéaire ou ramifié, halogénoalkyle, cyano ou phényle, α- ou β-naphtyle,
R1 et R3 peuvent former, conjointement avec un atome de N et un atome de C auxquels ils sont fixés, pyrrolidine ou pipéridine éventuellement substituée par oxo, X1, X2, X3 et X4 peuvent être identiques ou différents et représentent hydrogène, halogène, C1-12 alkyle linéaire ou ramifié, halogénoalkyle, halogénoalcoxy, alcoxycarbonyle, nitro ou cyano,
A représente trifluoroéthyle, et
n représente 0 ou 1.

2. Composés selon la revendication 1, dans lesquels
R1 représente hydrogène, C1-6 alkyle, C2-4 alcényle, C2-3 alcynyle, C1-4 alcoxy, C3-6 cycloalkyle, C1-4 halogénoalkyle, C2-4 (nombre total de carbones) alcoxyalkyle, phényle, α- ou β-naphtyle, C7-8 aralkyle, C1-2 alkylaminocarbonyle, C6-10 arylaminocarbonyle, ou C2-4 (nombre total de carbones) dialkylamino- C1-2 alkyle,
R2 représente hydrogène ou C1-4 alkyle,
R1 et R2 peuvent former, conjointement avec un atome de N auquel ils sont fixés, pyrrolidine, pipéridine ou morpholine,
R3 représente hydrogène, C1-6 alkyle, C1-4 halogénoalkyle, cyano ou phényle,
R1 et R3 peuvent former, conjointement avec un atome de N et un atome de C auxquels ils sont fixés, pyrrolidine ou pipéridine éventuellement substituée par oxo,
X1, X2, X3 et X4 peuvent être identiques ou différents et représentent hydrogène, halogène, C1-2 alkyle, C1-2 halogénoalkyle, C1-2 halogénoalcoxy, C1-2 alcoxycarbonyle, nitro ou cyano,
A représente trifluoroéthyle, et
n représente 0 ou 1.

3. Composés selon la revendication 1 ou 2, dans lesquels
R1 représente hydrogène, C1-3 alkyle, allyle, propargyle, C1-2 alcoxy, cyclopropyle, cyclohexyle, trifluoroéthyle, méthoxyéthyle, trifluorométhylphényle, phényle, benzyle, éthylaminocarbonyle, phénylaminocarbonyle, ou diméthylaminoéthyle,
R2 représente hydrogène ou C1-2 alkyle,
R1 et R2 peuvent former, conjointement avec un atome de N auquel ils sont fixés, pipéridine ou morpholine,
R3 représente hydrogène, Cl alkyle, C1-3 alkyle substitué par fluoro, cyano ou phényle,
R1 et R3 peuvent former, conjointement avec un atome de N et un atome de C auxquels ils sont fixés, pyrrolidine ou pipéridine éventuellement substituée par oxo,
X1 représente hydrogène ou chloro,
X2 représente hydrogène, fluoro, chloro, bromo, iodo, méthyle, difluorométhyle, trifluorométhyle, nitro ou cyano,
X3 représente hydrogène,
X4 représente hydrogène, chloro, bromo, iodo, méthyle, difluorométhyle, trifluorométhyle, nitro ou cyano,
A représente trifluoroéthyle, et
n représente 0 ou 1.

4. Composés selon l'une quelconque des revendications 1 à 3, dans lesquels
R1 représente hydrogène, C1-3 alkyle, allyle, propargyle, méthoxy, cyclopropyle, trifluoroéthyle, trifluorométhylphényle, benzyle, ou éthylaminocarbonyle,
R2 représente hydrogène, ou C1-2 alkyle,
R3 représente Cl alkyle, Cl alkylène, C1-3 alkyle substitué par fluoro, cyano ou phényle,
R1 et R3 peuvent former, conjointement avec un atome de N et un atome de C auxquels ils sont fixés, pyrrolidine,
X1 représente hydrogène,
X2 représente hydrogène, ou fluoro,
X3 représente hydrogène,
X4 représente difluorométhyle, ou cyano,
A représente trifluoroéthyle, et
n représente 0 ou 1.

5. Procédé de préparation des composés de formule (I) selon la revendication 1, **caractérisé en ce que** Méthode de production (a) : {dans la formule (I), au moins l'un parmi X2 et X4 représente un groupement attirant les électrons, et n représente 0} des composés de formule (II) 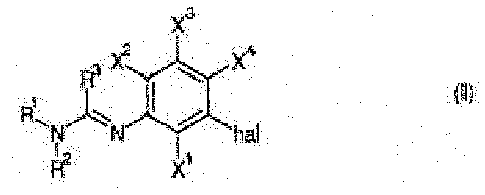 dans laquelle R1, R2, R3, X1, X2, X3 et X4 sont tels que définis selon la revendication 1 et au moins l'un parmi X2 et X4 représente un groupement attirant les électrons, et hal représente a halogène, sont réagis avec des composés de formule (III)
HS-A (III)
dans laquelle A est tel que défini ci-dessus, en présence d'un solvant inerte et le cas échéant, en présence d'une base,
Méthode de production (b) : {dans la formule (I), n représente 1}
des composés de formule (Ib) 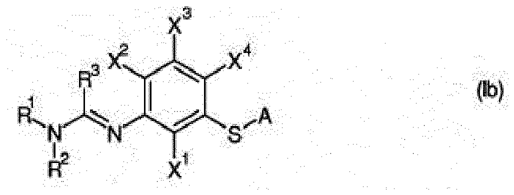 dans laquelle R1, R2, R3, X1, X2, X3, X4 et A sont tels que selon la revendication 1, sont oxydés en présence de solvants inertes,
Méthode de production (c) : {dans la formule (I), R3 représente hydrogène, alkyle ou aryle}
des composés de formule (IV) 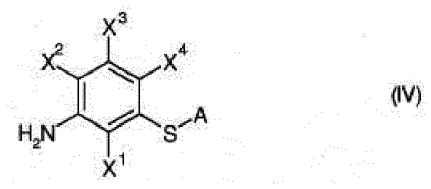 dans laquelle X1, X2, X3, X4 et A sont tels que définis selon la revendication 1, sont réagis avec des composés de formule (V) 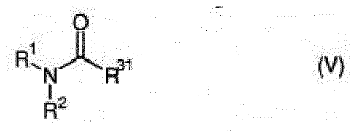 dans laquelle R1 et R2 sont tels que définis selon la revendication 1, R31 représente hydrogène, alkyle ou aryle, en présence de solvants inertes,
Méthode de production (d) : {dans la formule (I), R3 représente perfluoroalkyle}
des composés de formule (VI) 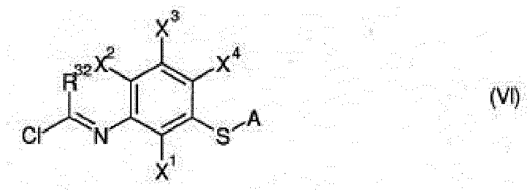 dans laquelle X1, X2, X3, X4 et A sont tels que définis selon la revendication 1, et R32 représente perfluoroalkyle, sont réagis avec des composés de formule (VII) 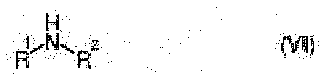 dans laquelle R1 et R2 sont tels que définis selon la revendication 1, en présence de solvants inertes et le cas échéant, en présence d'une base,
Méthode de production (e) : {dans la formule (I), R3 représente cyano}
des composés de formule (VIII) 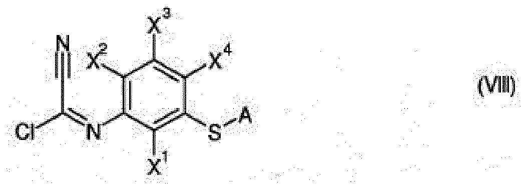 dans laquelle X1, X2, X3, X4 et A sont tels que définis selon la revendication 1, sont réagis avec des composés de composés de formule (VII) susmentionnés en présence de solvants inertes,
Méthode de production (f) : {dans la formule (I), R1 représente alkylaminocarbonyle}
des composés de formule (If) 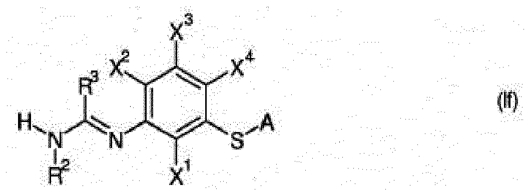 dans laquelle R2, R3, X1, X2, X3, X4 et A sont tels que définis selon la revendication 1, sont réagis avec des composés de formule (X)
R11-N=C=O (X)
dans laquelle R11 représente alkylaminocarbonyle, en présence de solvants inertes et le cas échéant, en présence d'une base.

6. Compositions insecticides, **caractérisées en ce qu'**elles contiennent au moins un composé de formule (I) tel que défini selon la revendication 1 ou selon la revendication 2 ou selon la revendication 3 ou selon la revendication 4.

7. Procédé de lutte contre des insectes néfastes, **caractérisé en ce que** l'on permet aux composés de formule (I) tels que définis selon la revendication 1 ou selon la revendication 2 ou selon la revendication 3 ou selon la revendication 4, d'agir sur les insectes néfastes et/ou leur habitat.

8. Utilisation des composés de formule (I) tels que définis selon la revendication 1 ou selon la revendication 2 ou selon la revendication 3 ou selon la revendication 4, pour lutter contre des insectes néfastes.

9. Procédé de préparation d'une composition insecticide, **caractérisé en ce que** les composés de formule (I) tels que définis selon la revendication 1 ou selon la revendication 2 ou selon la revendication 3 ou selon la revendication 4, sont mélangés avec des charges et/ou des agents tensioactifs.

10. Nouveaux dérivés d'aniline de formule (IV-A) 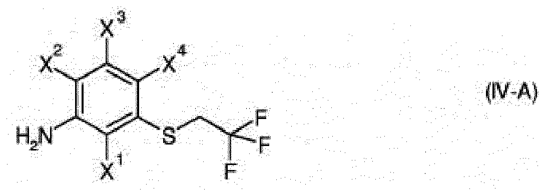 dans laquelle X1, X2 et X3 sont tels que définis selon la revendication 1 et X4 représente hydrogène, halogène, halogénoalkyle, halogénoalcoxy, alcoxycarbonyle, nitro ou cyano.
